(19) Europäisches Patentamt
European Patent Office
Office européen des brevets

(11) EP 1 477 564 A2

(12) # EUROPEAN PATENT APPLICATION

(43) Date of publication:
**17.11.2004 Bulletin 2004/47**

(21) Application number: **04017650.5**

(22) Date of filing: **20.07.2000**

(51) Int Cl.[7]: **C12N 15/52**, C12N 9/00, C07K 16/40, C12Q 1/68, A61K 38/43, A61K 48/00

(84) Designated Contracting States:
**AT BE CH CY DE DK ES FI FR GB GR IE IT LI LU MC NL PT SE**

(30) Priority: **22.07.1999 US 144992**
**02.12.1999 US 168858**

(62) Document number(s) of the earlier application(s) in accordance with Art. 76 EPC:
**00950556.1 / 1 200 596**

(71) Applicant: **Incyte Genomics, Inc.**
**Palo Alto, CA 94304 (US)**

(72) Inventors:
- **Tang, Tom**
**San Jose CA 95118 (US)**
- **Jackson, Jennifer L. (nee Hillmann)**
**Santa Cruz CA 95062 (US)**
- **Bandman, Olga**
**Mountain View CA 94043 (US)**
- **Yue, Henry**
**Sunnyvale CA 94087 (US)**
- **Baughn, Mariah, R.**
**Los Angeles California 90035 (US)**
- **Lal, Preeti**
**Santa Clara CA (US)**
- **Lu, Dyung, Aina, M.**
**San Jose CA 95136 (US)**
- **Shah, Purvi**
**Sunnyvale CA 94087 (US)**
- **Azimzai, Yalda**
**Oakland CA 94618 (US)**

(74) Representative: **Wright, Simon Mark**
**J.A. Kemp & Co.**
**14 South Square**
**Gray's Inn**
**London WC1R 5JJ (GB)**

Remarks:
This application was filed on 26 - 07 - 2004 as a divisional application to the application mentioned under INID code 62.

(54) **Human synthetases**

(57) The invention provides human synthetases (SYNT) and polynucleotides which identify and encode SYNT. The invention also provides expression vectors, host cells, antibodies, agonists, and antagonists. The invention also provide methods for diagnosing, treating, or preventing disorders associated with expression of SYNT.



Printed by Jouve, 75001 PARIS (FR)

SEQUENCE LISTING

<110> INCYTE GENOMICS, INC.
TANG, Y. Tom
HILLMAN, Jennifer L.
BANDMAN, Olga
YUE, Henry
BAUGHN, Mariah R.
LAL, Preeti
LU, Dyung Aina M.
SHAH, Purvi
AZIMZAI, Yalda

<120> HUMAN SYNTHETASES

<130> PF-0721 PCT

<140> To Be Assigned
<141> Herewith

<150> 60/144,992; 60,168,858
<151> 1999-07-22; 1999-12-02

<160> 30
<170> PERL Program

<210> 1
<211> 1176
<212> PRT
<213> Homo sapiens

<220>
<221> misc_feature
<223> Incyte ID No: 1806212CD1

<400> 1
```
Met Ala Glu Arg Lys Gly Thr Ala Lys Val Asp Phe Leu Lys Lys
  1               5                  10                  15
Ile Glu Lys Glu Ile Gln Gln Lys Trp Asp Thr Glu Arg Val Phe
                 20                  25                  30
Glu Val Asn Ala Ser Asn Leu Glu Lys Gln Thr Ser Lys Gly Lys
                 35                  40                  45
Tyr Phe Val Thr Phe Pro Tyr Pro Tyr Met Asn Gly Arg Leu His
                 50                  55                  60
Leu Gly His Thr Phe Ser Leu Ser Lys Cys Glu Phe Ala Val Gly
                 65                  70                  75
Tyr Gln Arg Leu Lys Gly Lys Cys Cys Leu Phe Pro Phe Gly Leu
                 80                  85                  90
His Cys Thr Gly Met Pro Ile Lys Ala Cys Ala Asp Lys Leu Lys
                 95                 100                 105
Arg Glu Ile Glu Leu Tyr Gly Cys Pro Pro Asp Phe Pro Asp Glu
                110                 115                 120
Glu Glu Glu Glu Glu Glu Thr Ser Val Lys Thr Glu Asp Ile Ile
                125                 130                 135
Ile Lys Asp Lys Ala Lys Gly Lys Lys Ser Lys Ala Ala Ala Lys
                140                 145                 150
Ala Gly Ser Ser Lys Tyr Gln Trp Gly Ile Met Lys Ser Leu Gly
                155                 160                 165
Leu Ser Asp Glu Glu Ile Val Lys Phe Ser Glu Ala Glu His Trp
                170                 175                 180
Leu Asp Tyr Phe Pro Pro Leu Ala Ile Gln Asp Leu Lys Arg Met
                185                 190                 195
```

```
Gly Leu Lys Val Asp Trp Arg Arg Ser Phe Ile Thr Thr Asp Val
                200                 205                 210
Asn Pro Tyr Tyr Asp Ser Phe Val Arg Trp Gln Phe Leu Thr Leu
                215                 220                 225
Arg Glu Arg Asn Lys Ile Lys Phe Gly Lys Arg Tyr Thr Ile Tyr
                230                 235                 240
Ser Pro Lys Asp Gly Gln Pro Cys Met Asp His Asp Arg Gln Thr
                245                 250                 255
Gly Glu Gly Val Gly Pro Gln Glu Tyr Thr Leu Leu Lys Leu Lys
                260                 265                 270
Val Leu Glu Pro Tyr Pro Ser Lys Leu Ser Gly Leu Lys Gly Lys
                275                 280                 285
Asn Ile Phe Leu Val Ala Ala Thr Leu Arg Pro Glu Thr Met Phe
                290                 295                 300
Gly Gln Thr Asn Cys Trp Val Arg Pro Asp Met Lys Tyr Ile Gly
                305                 310                 315
Phe Glu Thr Val Asn Gly Asp Ile Phe Ile Cys Thr Gln Lys Ala
                320                 325                 330
Ala Arg Asn Met Ser Tyr Gln Gly Phe Thr Lys Asp Asn Gly Val
                335                 340                 345
Val Pro Val Val Lys Glu Leu Met Gly Glu Glu Ile Leu Gly Ala
                350                 355                 360
Ser Leu Ser Ala Pro Leu Thr Ser Tyr Lys Val Ile Tyr Val Leu
                365                 370                 375
Pro Met Leu Thr Ile Lys Glu Asp Lys Gly Thr Gly Val Val Thr
                380                 385                 390
Ser Val Pro Ser Asp Ser Pro Asp Asp Ile Ala Ala Leu Arg Asp
                395                 400                 405
Leu Lys Lys Lys Gln Ala Leu Arg Ala Lys Tyr Gly Ile Arg Asp
                410                 415                 420
Asp Met Val Leu Pro Phe Glu Pro Val Pro Val Ile Glu Ile Pro
                425                 430                 435
Gly Phe Gly Asn Leu Ser Ala Val Thr Ile Cys Asp Glu Leu Lys
                440                 445                 450
Ile Gln Ser Gln Asn Asp Arg Glu Lys Leu Ala Glu Ala Lys Glu
                455                 460                 465
Lys Ile Tyr Leu Lys Gly Phe Tyr Glu Gly Ile Met Leu Val Asp
                470                 475                 480
Gly Phe Lys Gly Gln Lys Val Gln Asp Val Lys Lys Thr Ile Gln
                485                 490                 495
Lys Lys Met Ile Asp Ala Gly Asp Ala Leu Ile Tyr Met Glu Pro
                500                 505                 510
Glu Lys Gln Val Met Ser Arg Ser Ser Asp Glu Cys Val Val Ala
                515                 520                 525
Leu Cys Asp Gln Trp Tyr Leu Asp Tyr Gly Glu Glu Asn Trp Lys
                530                 535                 540
Lys Gln Thr Ser Gln Cys Leu Lys Asn Leu Glu Thr Phe Cys Glu
                545                 550                 555
Glu Thr Arg Arg Asn Phe Glu Ala Thr Leu Gly Trp Leu Gln Glu
                560                 565                 570
His Ala Cys Ser Arg Thr Tyr Gly Leu Gly Thr His Leu Pro Trp
                575                 580                 585
Asp Glu Gln Trp Leu Ile Glu Ser Leu Ser Asp Ser Thr Ile Tyr
                590                 595                 600
Met Ala Phe Tyr Thr Val Ala His Leu Leu Gln Gly Gly Asn Leu
                605                 610                 615
His Gly Gln Ala Glu Ser Pro Leu Gly Ile Arg Pro Gln Gln Met
                620                 625                 630
Thr Lys Glu Val Trp Asp Tyr Val Phe Phe Lys Glu Ala Pro Phe
                635                 640                 645
Pro Lys Thr Gln Ile Ala Lys Glu Lys Leu Asp Gln Leu Lys Gln
                650                 655                 660
Glu Phe Glu Phe Trp Tyr Pro Val Asp Leu Arg Val Ser Gly Lys
                665                 670                 675
Asp Leu Val Pro Asn His Leu Ser Tyr Tyr Leu Tyr Asn His Val
```

```
                        680                 685                 690
Ala Met Trp Pro Glu Gln Ser Asp Lys Trp Pro Thr Ala Val Arg
                        695                 700                 705
Ala Asn Gly His Leu Leu Leu Asn Ser Glu Lys Met Ser Lys Ser
                        710                 715                 720
Thr Gly Asn Phe Leu Thr Leu Thr Gln Ala Ile Asp Lys Phe Ser
                        725                 730                 735
Ala Asp Gly Met Arg Leu Ala Leu Ala Asp Ala Gly Asp Thr Val
                        740                 745                 750
Glu Asp Ala Asn Phe Val Glu Ala Met Ala Asp Ala Gly Ile Leu
                        755                 760                 765
Arg Leu Tyr Thr Trp Val Glu Trp Val Lys Glu Met Val Ala Asn
                        770                 775                 780
Trp Asp Ser Leu Arg Ser Gly Pro Ala Ser Thr Phe Asn Asp Arg
                        785                 790                 795
Val Phe Ala Ser Glu Leu Asn Ala Gly Ile Ile Lys Thr Asp Gln
                        800                 805                 810
Asn Tyr Glu Lys Met Met Phe Lys Glu Ala Leu Lys Thr Gly Phe
                        815                 820                 825
Phe Glu Phe Gln Ala Ala Lys Asp Lys Tyr Arg Glu Leu Ala Val
                        830                 835                 840
Glu Gly Met His Arg Glu Leu Val Phe Arg Phe Ile Glu Val Gln
                        845                 850                 855
Thr Leu Leu Leu Ala Pro Phe Cys Pro His Leu Cys Glu His Ile
                        860                 865                 870
Trp Thr Leu Leu Gly Lys Pro Asp Ser Ile Met Asn Ala Ser Trp
                        875                 880                 885
Pro Val Ala Gly Pro Val Asn Glu Val Leu Ile His Ser Ser Gln
                        890                 895                 900
Tyr Leu Met Glu Val Thr His Asp Leu Arg Leu Arg Leu Lys Asn
                        905                 910                 915
Tyr Met Met Pro Ala Lys Gly Lys Lys Thr Asp Lys Gln Pro Leu
                        920                 925                 930
Gln Lys Pro Ser His Cys Thr Ile Tyr Val Ala Lys Asn Tyr Pro
                        935                 940                 945
Pro Trp Gln His Thr Thr Leu Ser Val Leu Arg Lys His Phe Glu
                        950                 955                 960
Ala Asn Asn Gly Lys Leu Pro Asp Asn Lys Val Ile Ala Ser Glu
                        965                 970                 975
Leu Gly Ser Met Pro Glu Leu Lys Lys Tyr Met Lys Lys Val Met
                        980                 985                 990
Pro Phe Val Ala Met Ile Lys Glu Asn Leu Glu Lys Met Gly Pro
                        995                 1000                1005
Arg Ile Leu Asp Leu Gln Leu Glu Phe Asp Glu Lys Ala Val Leu
                        1010                1015                1020
Met Glu Asn Ile Val Tyr Leu Thr Asn Ser Leu Glu Leu Glu His
                        1025                1030                1035
Ile Glu Val Lys Phe Ala Ser Glu Ala Glu Asp Lys Ile Arg Glu
                        1040                1045                1050
Asp Cys Cys Pro Gly Lys Pro Leu Asn Val Phe Arg Ile Glu Pro
                        1055                1060                1065
Gly Val Ser Val Ser Leu Val Asn Pro Gln Pro Ser Asn Gly His
                        1070                1075                1080
Phe Ser Thr Lys Ile Glu Ile Arg Gln Gly Asp Asn Cys Asp Ser
                        1085                1090                1095
Ile Ile Arg Arg Leu Met Lys Met Asn Arg Gly Ile Lys Asp Leu
                        1100                1105                1110
Ser Lys Val Lys Leu Met Arg Phe Asp Asp Pro Leu Leu Gly Pro
                        1115                1120                1125
Arg Arg Val Pro Val Leu Gly Lys Glu Tyr Thr Glu Lys Thr Pro
                        1130                1135                1140
Ile Ser Glu His Ala Val Phe Asn Val Asp Leu Met Ser Lys Lys
                        1145                1150                1155
Ile His Leu Thr Glu Asn Gly Ile Arg Val Asp Ile Gly Asp Thr
                        1160                1165                1170
```

Ile Ile Tyr Leu Val His
1175

<210> 2
<211> 739
<212> PRT
<213> Homo sapiens

<220>
<221> misc_feature
<223> Incyte ID No: 2083883CD1

<400> 2
Met Asp Ala Leu Lys Pro Pro Cys Leu Trp Arg Asn His Glu Arg
1 5 10 15
Gly Lys Lys Asp Arg Asp Ser Cys Gly Arg Lys Asn Ser Glu Pro
20 25 30
Gly Ser Pro His Ser Leu Glu Ala Leu Arg Asp Ala Ala Pro Ser
35 40 45
Gln Gly Leu Asn Phe Leu Leu Leu Phe Thr Lys Met Leu Phe Ile
50 55 60
Phe Asn Phe Leu Phe Ser Pro Leu Pro Thr Pro Ala Leu Ile Cys
65 70 75
Ile Leu Thr Phe Gly Ala Ala Ile Phe Leu Trp Leu Ile Thr Arg
80 85 90
Pro Gln Pro Val Leu Pro Leu Leu Asp Leu Asn Asn Gln Ser Val
95 100 105
Gly Ile Glu Gly Gly Ala Arg Lys Gly Val Ser Gln Lys Asn Asn
110 115 120
Asp Leu Thr Ser Cys Cys Phe Ser Asp Ala Lys Thr Met Tyr Glu
125 130 135
Val Phe Gln Arg Gly Leu Ala Val Ser Asp Asn Gly Pro Cys Leu
140 145 150
Gly Tyr Arg Lys Pro Asn Gln Pro Tyr Arg Trp Leu Ser Tyr Lys
155 160 165
Gln Val Ser Asp Arg Ala Glu Tyr Leu Gly Ser Cys Leu Leu His
170 175 180
Lys Gly Tyr Lys Ser Ser Pro Asp Gln Phe Val Gly Ile Phe Ala
185 190 195
Gln Asn Arg Pro Glu Trp Ile Ile Ser Glu Leu Ala Cys Tyr Thr
200 205 210
Tyr Ser Met Val Ala Val Pro Leu Tyr Asp Thr Leu Gly Pro Glu
215 220 225
Ala Ile Val His Ile Val Asn Lys Ala Asp Ile Ala Val Val Ile
230 235 240
Cys Asp Thr Pro Gln Lys Ala Leu Val Leu Ile Gly Asn Val Glu
245 250 255
Lys Gly Phe Thr Pro Ser Leu Lys Val Ile Ile Leu Met Asp Pro
260 265 270
Phe Asp Asp Asp Leu Lys Gln Arg Gly Glu Lys Ser Gly Ile Glu
275 280 285
Ile Leu Ser Leu Tyr Asp Ala Glu Asn Leu Gly Lys Glu His Phe
290 295 300
Arg Lys Pro Val Pro Pro Ser Pro Glu Asp Leu Ser Val Ile Cys
305 310 315
Phe Thr Ser Gly Thr Thr Gly Asp Pro Lys Gly Ala Met Ile Thr
320 325 330
His Gln Asn Ile Val Ser Asn Ala Ala Ala Phe Leu Lys Cys Val
335 340 345
Glu His Ala Tyr Glu Pro Thr Pro Asp Asp Val Ala Ile Ser Tyr
350 355 360
Leu Pro Leu Ala His Met Phe Glu Arg Ile Val Gln Ala Val Val
365 370 375
Tyr Ser Cys Gly Ala Arg Val Gly Phe Phe Gln Gly Asp Ile Arg
380 385 390
Leu Leu Ala Asp Asp Met Lys Thr Leu Lys Pro Thr Leu Phe Pro

```
                    395                 400                 405
Ala Val Pro Arg Leu Leu Asn Arg Ile Tyr Asp Lys Val Gln Asn
                  - 410                 415                 420
Glu Ala Lys Thr Pro Leu Lys Lys Phe Leu Leu Lys Leu Ala Val
                425                 430                 435
Ser Ser Lys Phe Lys Glu Leu Gln Lys Gly Ile Ile Arg His Asp
                440                 445                 450
Ser Phe Trp Asp Lys Leu Ile Phe Ala Lys Ile Gln Asp Ser Leu
                455                 460                 465
Gly Gly Arg Val Arg Val Ile Val Thr Gly Ala Ala Pro Met Ser
                470                 475                 480
Thr Ser Val Met Thr Phe Phe Arg Ala Ala Met Gly Cys Gln Val
                485                 490                 495
Tyr Glu Ala Tyr Gly Gln Thr Glu Cys Thr Gly Gly Cys Thr Phe
                500                 505                 510
Thr Leu Pro Gly Asp Trp Thr Ser Gly His Val Gly Val Pro Leu
                515                 520                 525
Ala Cys Asn Tyr Val Lys Leu Glu Asp Val Ala Asp Met Asn Tyr
                530                 535                 540
Phe Thr Val Asn Asn Glu Gly Glu Val Cys Ile Lys Gly Thr Asn
                545                 550                 555
Val Phe Lys Gly Tyr Leu Lys Asp Pro Glu Lys Thr Gln Glu Ala
                560                 565                 570
Leu Asp Ser Asp Gly Trp Leu His Thr Gly Asp Ile Gly Arg Trp
                575                 580                 585
Leu Pro Asn Gly Thr Leu Lys Ile Ile Asp Arg Lys Lys Asn Ile
                590                 595                 600
Phe Lys Leu Ala Gln Gly Glu Tyr Ile Ala Pro Glu Lys Ile Glu
                605                 610                 615
Asn Ile Tyr Asn Arg Ser Gln Pro Val Leu Gln Ile Phe Val His
                620                 625                 630
Gly Glu Ser Leu Arg Ser Ser Leu Val Gly Val Val Val Pro Asp
                635                 640                 645
Thr Asp Val Leu Pro Ser Phe Ala Ala Lys Leu Gly Val Lys Gly
                650                 655                 660
Ser Phe Glu Glu Leu Cys Gln Asn Gln Val Val Arg Glu Ala Ile
                665                 670                 675
Leu Glu Asp Leu Gln Lys Ile Gly Lys Glu Ser Gly Leu Lys Thr
                680                 685                 690
Phe Glu Gln Val Lys Ala Ile Phe Leu His Pro Glu Pro Phe Ser
                695                 700                 705
Ile Glu Asn Gly Leu Leu Thr Pro Thr Leu Lys Ala Lys Arg Gly
                710                 715                 720
Glu Leu Ser Lys Tyr Phe Arg Thr Gln Ile Asp Ser Leu Tyr Glu
                725                 730                 735
His Ile Gln Asp

<210> 3
<211> 589
<212> PRT
<213> Homo sapiens

<220>
<221> misc_feature
<223> Incyte ID No: 2454288CD1

<400> 3
Met Pro Thr Val Ser Val Lys Arg Asp Leu Leu Phe Gln Ala Leu
  1               5                  10                  15
Gly Arg Thr Tyr Thr Asp Glu Glu Phe Asp Glu Leu Cys Phe Glu
                 20                  25                  30
Phe Gly Leu Glu Leu Asp Glu Ile Thr Ser Glu Lys Glu Ile Ile
                 35                  40                  45
Ser Lys Glu Gln Gly Asn Val Lys Ala Ala Gly Ala Ser Asp Val
                 50                  55                  60
```

```
Val Leu Tyr Lys Ile Asp Val Pro Ala Asn Arg Tyr Asp Leu Leu
                65                  70                  75
Cys Leu Glu Gly Leu Val Arg Gly Leu Gln Val Phe Lys Glu Arg
                80                  85                  90
Ile Lys Ala Pro Val Tyr Lys Arg Val Met Pro Asp Gly Lys Ile
                95                  100                 105
Gln Lys Leu Ile Ile Thr Glu Glu Thr Ala Lys Ile Arg Pro Phe
                110                 115                 120
Ala Val Ala Ala Val Leu Arg Asn Ile Lys Phe Thr Lys Asp Arg
                125                 130                 135
Tyr Asp Ser Phe Ile Glu Leu Gln Glu Lys Leu His Gln Asn Ile
                140                 145                 150
Cys Arg Lys Arg Ala Leu Val Ala Ile Gly Thr His Asp Leu Asp
                155                 160                 165
Thr Leu Ser Gly Pro Phe Thr Tyr Thr Ala Lys Arg Pro Ser Asp
                170                 175                 180
Ile Lys Phe Lys Pro Leu Asn Lys Thr Lys Glu Tyr Thr Ala Cys
                185                 190                 195
Glu Leu Met Asn Ile Tyr Lys Thr Asp Asn His Leu Lys His Tyr
                200                 205                 210
Leu His Ile Ile Glu Asn Lys Pro Leu Tyr Pro Val Ile Tyr Asp
                215                 220                 225
Ser Asn Gly Val Val Leu Ser Met Pro Pro Ile Ile Asn Gly Asp
                230                 235                 240
His Ser Arg Ile Thr Val Asn Thr Arg Asn Ile Phe Ile Glu Cys
                245                 250                 255
Thr Gly Thr Asp Phe Thr Lys Ala Lys Ile Val Leu Asp Ile Ile
                260                 265                 270
Val Thr Met Phe Ser Glu Tyr Cys Glu Asn Gln Phe Thr Val Glu
                275                 280                 285
Ala Ala Glu Val Val Phe Pro Asn Gly Lys Ser His Thr Phe Pro
                290                 295                 300
Glu Leu Ala Tyr Arg Lys Glu Met Val Arg Ala Asp Leu Ile Asn
                305                 310                 315
Lys Lys Val Gly Ile Arg Glu Thr Pro Glu Asn Leu Ala Lys Leu
                320                 325                 330
Leu Thr Arg Met Tyr Leu Lys Ser Glu Val Ile Gly Asp Gly Asn
                335                 340                 345
Gln Ile Glu Ile Glu Ile Pro Pro Thr Arg Ala Asp Ile Ile His
                350                 355                 360
Ala Cys Asp Ile Val Glu Asp Ala Ala Ile Ala Tyr Gly Tyr Asn
                365                 370                 375
Asn Ile Gln Met Thr Leu Pro Lys Thr Tyr Thr Ile Ala Asn Gln
                380                 385                 390
Phe Pro Leu Asn Lys Leu Thr Glu Leu Leu Arg His Asp Met Ala
                395                 400                 405
Ala Ala Gly Phe Thr Glu Ala Leu Thr Phe Ala Leu Cys Ser Gln
                410                 415                 420
Glu Asp Ile Ala Asp Lys Leu Gly Val Asp Ile Ser Ala Thr Lys
                425                 430                 435
Ala Val His Ile Ser Asn Pro Lys Thr Ala Glu Phe Gln Val Ala
                440                 445                 450
Arg Thr Thr Leu Leu Pro Gly Leu Leu Lys Thr Ile Ala Ala Asn
                455                 460                 465
Arg Lys Met Pro Leu Pro Leu Lys Leu Phe Glu Ile Ser Asp Ile
                470                 475                 480
Val Ile Lys Asp Ser Asn Thr Asp Val Gly Ala Lys Asn Tyr Arg
                485                 490                 495
His Leu Cys Ala Val Tyr Tyr Asn Lys Asn Pro Gly Phe Glu Ile
                500                 505                 510
Ile His Gly Leu Leu Asp Arg Ile Met Gln Leu Leu Asp Val Pro
                515                 520                 525
Pro Gly Glu Asp Lys Gly Gly Tyr Val Ile Lys Ala Ser Glu Gly
                530                 535                 540
Pro Ala Phe Phe Pro Gly Arg Cys Ala Glu Ile Phe Ala Arg Gly
```

```
                545                 550                 555
Gln Ser Val Gly Lys Leu Gly Val Leu His Pro Asp Val Ile Thr
                560                 565                 570
Lys Phe Glu Leu Thr Met Pro Cys Ser Ser Leu Glu Ile Asn Ile
                575                 580                 585
Gly Pro Phe Leu

<210> 4
<211> 157
<212> PRT
<213> Homo sapiens

<220>
<221> misc_feature
<223> Incyte ID No: 1513539CD1

<400> 4
Met Lys Leu Lys Cys Ile Phe Gly Phe Ala Thr Lys Glu Thr Ser
  1               5                  10                  15
Cys Tyr Asn Val Thr Asn Ile Gly Phe Lys Ser Pro Ser Asp Phe
                 20                  25                  30
Trp Gln Ser Val His Ser Thr Leu Pro Arg Glu Leu Ala Pro Cys
                 35                  40                  45
Leu Val Phe Asn Thr Ser Pro Asn Leu Ala Leu Phe Ser Ala Ala
                 50                  55                  60
Phe Ala Phe Ile Val Val Lys Asp Ser Ala Gly Asp Ser Asp Val
                 65                  70                  75
Val Val Gln Glu Leu Lys Ser Met Val Ala Thr Lys Ile Ala Lys
                 80                  85                  90
Tyr Ala Val Pro Asp Glu Ile Leu Val Val Lys Arg Leu Pro Lys
                 95                 100                 105
Thr Arg Ser Gly Lys Val Met Arg Arg Leu Leu Arg Lys Ile Ile
                110                 115                 120
Thr Ser Glu Ala Gln Glu Leu Gly Asp Thr Thr Thr Leu Glu Asp
                125                 130                 135
Pro Ser Ile Ile Ala Glu Ile Leu Ser Val Tyr Gln Lys Cys Lys
                140                 145                 150
Asp Lys Gln Ala Ala Ala Lys
                155
<210> 5
<211> 643
<212> PRT
<213> Homo sapiens

<220>
<221> misc_feature
<223> Incyte ID No: 2148623CD1

<400> 5
Met Cys Gly Ile Cys Cys Ser Val Asn Phe Ser Ala Glu His Phe
  1               5                  10                  15
Ser Gln Asp Leu Lys Glu Asp Leu Leu Tyr Asn Leu Lys Gln Arg
                 20                  25                  30
Gly Pro Asn Ser Ser Lys Gln Leu Leu Lys Ser Asp Val Asn Tyr
                 35                  40                  45
Gln Cys Leu Phe Ser Ala His Val Leu His Leu Arg Gly Val Leu
                 50                  55                  60
Thr Thr Gln Pro Val Glu Asp Glu Arg Gly Asn Val Phe Leu Trp
                 65                  70                  75
Asn Gly Glu Ile Phe Ser Gly Ile Lys Val Glu Ala Glu Glu Asn
                 80                  85                  90
Asp Thr Gln Ile Leu Phe Asn Tyr Leu Ser Ser Cys Lys Asn Glu
                 95                 100                 105
Ser Glu Ile Leu Ser Leu Phe Ser Glu Val Gln Gly Pro Trp Ser
                110                 115                 120
```

```
Phe Ile Tyr Tyr Gln Ala Ser Ser His Tyr Leu Trp Phe Gly Arg
                125                 130                 135
Asp Phe Phe Gly Arg Arg Ser Leu Leu Trp His Phe Ser Asn Leu
                140                 145                 150
Gly Lys Ser Phe Cys Leu Ser Ser Val Gly Thr Gln Thr Ser Gly
                155                 160                 165
Leu Ala Asn Gln Trp Gln Glu Val Pro Ala Ser Gly Leu Phe Arg
                170                 175                 180
Ile Asp Leu Lys Ser Thr Val Ile Ser Arg Cys Ile Ile Leu Gln
                185                 190                 195
Leu Tyr Pro Trp Lys Tyr Ile Ser Arg Glu Asn Ile Ile Glu Glu
                200                 205                 210
Asn Val Asn Ser Leu Ser Gln Ile Ser Ala Asp Leu Pro Ala Phe
                215                 220                 225
Val Ser Val Val Ala Asn Glu Ala Lys Leu Tyr Leu Glu Lys Pro
                230                 235                 240
Val Val Pro Leu Asn Met Met Leu Pro Gln Ala Ala Leu Glu Thr
                245                 250                 255
His Cys Ser Asn Ile Ser Asn Val Pro Pro Thr Arg Glu Ile Leu
                260                 265                 270
Gln Val Phe Leu Thr Asp Val His Met Lys Glu Val Ile Gln Gln
                275                 280                 285
Phe Ile Asp Val Leu Ser Val Ala Val Lys Lys Arg Val Leu Cys
                290                 295                 300
Leu Pro Arg Asp Glu Asn Leu Thr Ala Asn Glu Val Leu Lys Thr
                305                 310                 315
Cys Asp Arg Lys Ala Asn Val Ala Ile Leu Phe Ser Gly Gly Ile
                320                 325                 330
Asp Ser Met Val Ile Ala Thr Leu Ala Asp Arg His Ile Pro Leu
                335                 340                 345
Asp Glu Pro Ile Asp Leu Leu Asn Val Ala Phe Ile Ala Glu Glu
                350                 355                 360
Lys Thr Met Pro Thr Thr Phe Asn Arg Glu Gly Asn Lys Gln Lys
                365                 370                 375
Asn Lys Cys Glu Ile Pro Ser Glu Glu Phe Ser Lys Asp Val Ala
                380                 385                 390
Ala Ala Ala Ala Asp Ser Pro Asn Lys His Val Ser Val Pro Asp
                395                 400                 405
Arg Ile Thr Gly Arg Ala Gly Leu Lys Glu Leu Gln Ala Val Ser
                410                 415                 420
Pro Ser Arg Ile Trp Asn Phe Val Glu Ile Asn Val Ser Met Glu
                425                 430                 435
Glu Leu Gln Lys Leu Arg Arg Thr Arg Ile Cys His Leu Ile Arg
                440                 445                 450
Pro Leu Asp Thr Val Leu Asp Asp Ser Ile Gly Cys Ala Val Trp
                455                 460                 465
Phe Ala Ser Arg Gly Ile Gly Trp Leu Val Ala Gln Glu Gly Val
                470                 475                 480
Lys Ser Tyr Gln Ser Asn Ala Lys Val Val Leu Thr Gly Ile Gly
                485                 490                 495
Ala Asp Glu Gln Leu Ala Gly Tyr Ser Arg His Arg Val Arg Phe
                500                 505                 510
Gln Ser His Gly Leu Glu Gly Leu Asn Lys Glu Ile Met Met Glu
                515                 520                 525
Leu Gly Arg Ile Ser Ser Arg Asn Leu Gly Arg Asp Asp Arg Val
                530                 535                 540
Ile Gly Asp His Gly Lys Glu Ala Arg Phe Pro Phe Leu Asp Glu
                545                 550                 555
Asn Val Val Ser Phe Leu Asn Ser Leu Pro Ile Trp Glu Lys Ala
                560                 565                 570
Asn Leu Thr Leu Pro Arg Gly Ile Gly Glu Lys Leu Leu Leu Arg
                575                 580                 585
Leu Ala Ala Val Glu Leu Gly Leu Thr Ala Ser Ala Leu Leu Pro
                590                 595                 600
Lys Arg Ala Met Gln Phe Gly Ser Arg Ile Ala Lys Met Glu Lys
```

```
                    605                 610                 615
Ile Asn Glu Lys Ala Ser Asp Lys Cys Gly Arg Leu Gln Ile Met
                    620                 625                 630
Ser Leu Glu Asn Leu Ser Ile Glu Lys Glu Thr Lys Leu
                    635                 640
```

<210> 6
<211> 660
<212> PRT
<213> Homo sapiens

<220>
<221> misc_feature
<223> Incyte ID No: 2579405CD1

<400> 6

```
Met Asp Ile Leu Val Ser Glu Cys Ser Ala Arg Leu Leu Gln Gln
 1               5                  10                  15
Glu Glu Glu Ile Lys Ser Leu Thr Ala Glu Ile Asp Arg Leu Lys
                20                  25                  30
Asn Cys Gly Cys Leu Gly Ala Ser Pro Asn Leu Glu Gln Leu Gln
                35                  40                  45
Glu Glu Asn Leu Lys Leu Lys Tyr Arg Leu Asn Ile Leu Arg Lys
                50                  55                  60
Ser Leu Gln Ala Glu Arg Asn Lys Pro Thr Lys Asn Met Ile Asn
                65                  70                  75
Ile Ile Ser Arg Leu Gln Glu Val Phe Gly His Ala Ile Lys Ala
                80                  85                  90
Ala Tyr Pro Asp Leu Glu Asn Pro Pro Leu Leu Val Thr Pro Ser
                95                 100                 105
Gln Gln Ala Lys Phe Gly Asp Tyr Gln Cys Asn Ser Ala Met Gly
               110                 115                 120
Ile Ser Gln Met Leu Lys Thr Lys Glu Gln Lys Val Asn Pro Arg
               125                 130                 135
Glu Ile Ala Glu Asn Ile Thr Lys His Leu Pro Asp Asn Glu Cys
               140                 145                 150
Ile Glu Lys Val Glu Ile Ala Gly Pro Gly Phe Ile Asn Val His
               155                 160                 165
Leu Arg Lys Asp Phe Val Ser Glu Gln Leu Thr Ser Leu Leu Val
               170                 175                 180
Asn Gly Val Gln Leu Pro Ala Leu Gly Glu Asn Lys Lys Val Ile
               185                 190                 195
Val Asp Phe Ser Ser Pro Asn Ile Ala Lys Glu Met His Val Gly
               200                 205                 210
His Leu Arg Ser Thr Ile Ile Gly Glu Ser Ile Ser Arg Leu Phe
               215                 220                 225
Glu Phe Ala Gly Tyr Asp Val Leu Arg Leu Asn His Val Gly Asp
               230                 235                 240
Trp Gly Thr Gln Phe Gly Met Leu Ile Ala His Leu Gln Asp Lys
               245                 250                 255
Phe Pro Asp Tyr Leu Thr Val Ser Pro Pro Ile Gly Asp Leu Gln
               260                 265                 270
Val Phe Tyr Lys Glu Ser Lys Lys Arg Phe Asp Thr Glu Glu Glu
               275                 280                 285
Phe Lys Lys Arg Ala Tyr Gln Cys Val Val Leu Leu Gln Gly Lys
               290                 295                 300
Asn Pro Asp Ile Thr Lys Ala Trp Lys Leu Ile Cys Asp Val Ser
               305                 310                 315
Arg Gln Glu Leu Asn Lys Ile Tyr Asp Ala Leu Asp Val Ser Leu
               320                 325                 330
Ile Glu Arg Gly Glu Ser Phe Tyr Gln Asp Arg Met Asn Asp Ile
               335                 340                 345
Val Lys Glu Phe Glu Asp Arg Gly Phe Val Gln Val Asp Asp Gly
               350                 355                 360
Arg Lys Ile Val Phe Val Pro Gly Cys Ser Ile Pro Leu Thr Ile
               365                 370                 375
```

```
Val Lys Ser Asp Gly Gly Tyr Thr Tyr Asp Thr Ser Asp Leu Ala
                380                 385                 390
Ala Ile Lys Gln Arg Leu Phe Glu Glu Lys Ala Asp Met Ile Ile
                395                 400                 405
Tyr Val Val Asp Asn Gly Gln Ser Val His Phe Gln Thr Ile Phe
                410                 415                 420
Ala Ala Ala Gln Met Ile Gly Trp Tyr Asp Pro Lys Val Thr Arg
                425                 430                 435
Val Phe His Ala Gly Phe Gly Val Val Leu Gly Glu Asp Lys Lys
                440                 445                 450
Lys Phe Lys Thr Arg Ser Gly Glu Thr Val Arg Leu Met Asp Leu
                455                 460                 465
Leu Gly Glu Gly Leu Lys Arg Ser Met Asp Lys Leu Lys Glu Lys
                470                 475                 480
Glu Arg Asp Lys Val Leu Thr Ala Glu Glu Leu Asn Ala Ala Gln
                485                 490                 495
Thr Ser Val Ala Tyr Gly Cys Ile Lys Tyr Ala Asp Leu Ser His
                500                 505                 510
Asn Arg Leu Asn Asp Tyr Ile Phe Ser Phe Asp Lys Met Leu Asp
                515                 520                 525
Asp Arg Gly Asn Thr Ala Ala Tyr Leu Leu Tyr Ala Phe Thr Arg
                530                 535                 540
Ile Arg Ser Ile Ala Arg Leu Ala Asn Ile Asp Glu Glu Met Leu
                545                 550                 555
Gln Lys Ala Ala Arg Glu Thr Lys Ile Leu Leu Asp His Glu Lys
                560                 565                 570
Glu Trp Lys Leu Gly Arg Cys Ile Leu Arg Phe Pro Glu Ile Leu
                575                 580                 585
Gln Lys Ile Leu Asp Asp Leu Phe Leu His Thr Leu Cys Asp Tyr
                590                 595                 600
Ile Tyr Glu Leu Ala Thr Ala Phe Thr Glu Phe Tyr Asp Ser Cys
                605                 610                 615
Tyr Cys Val Glu Lys Asp Arg Gln Thr Gly Lys Ile Leu Lys Val
                620                 625                 630
Asn Met Trp Arg Met Leu Leu Cys Glu Ala Val Ala Ala Val Met
                635                 640                 645
Ala Lys Gly Phe Asp Ile Leu Gly Ile Lys Pro Val Gln Arg Met
                650                 655                 660
```

<210> 7
<211> 725
<212> PRT
<213> Homo sapiens

<220>
<221> misc_feature
<223> Incyte ID No: 2662427CD1

<400> 7

```
Met Ala Ala Ala Ser Ala Val Ser Val Leu Leu Val Ala Ala Glu
 1               5                  10                  15
Arg Asn Arg Trp His Arg Leu Pro Ser Leu Leu Leu Pro Pro Arg
                20                  25                  30
Thr Trp Val Trp Arg Gln Arg Thr Met Lys Tyr Thr Thr Ala Thr
                35                  40                  45
Gly Arg Asn Ile Thr Lys Val Leu Ile Ala Asn Arg Gly Glu Ile
                50                  55                  60
Ala Cys Arg Val Met Arg Thr Ala Lys Lys Leu Gly Val Gln Thr
                65                  70                  75
Val Ala Val Tyr Ser Glu Ala Asp Arg Asn Ser Met His Val Asp
                80                  85                  90
Met Ala Asp Glu Ala Tyr Ser Ile Gly Pro Ala Pro Ser Gln Gln
                95                  100                 105
Ser Tyr Leu Ser Met Glu Lys Ile Ile Gln Val Ala Lys Thr Ser
                110                 115                 120
```

```
Ala Ala Gln Ala Ile His Pro Gly Cys Arg Phe Leu Ser Glu Asn
                125                 130                 135
Met Glu Phe Ala Glu Leu Cys Lys Gln Glu Gly Ile Ile Phe Ile
                140                 145                 150
Gly Pro Pro Pro Ser Ala Ile Arg Asp Met Gly Ile Lys Ser Thr
                155                 160                 165
Ser Lys Ser Ile Met Ala Ala Ala Gly Val Pro Val Val Glu Gly
                170                 175                 180
Tyr His Gly Glu Asp Gln Ser Asp Gln Cys Leu Lys Glu His Ala
                185                 190                 195
Arg Arg Ile Gly Tyr Pro Val Met Ile Lys Ala Val Arg Gly Gly
                200                 205                 210
Gly Gly Lys Gly Met Arg Ile Val Arg Ser Glu Gln Glu Phe Gln
                215                 220                 225
Glu Gln Leu Glu Ser Ala Arg Arg Glu Ala Lys Lys Ser Phe Asn
                230                 235                 240
Asp Asp Ala Met Leu Ile Glu Lys Phe Val Asp Thr Pro Arg His
                245                 250                 255
Val Glu Val Gln Val Phe Gly Asp His His Gly Asn Ala Val Tyr
                260                 265                 270
Leu Phe Glu Arg Asp Cys Ser Val Gln Arg Arg His Gln Lys Ile
                275                 280                 285
Ile Glu Glu Ala Pro Ala Pro Gly Ile Lys Ser Glu Val Arg Lys
                290                 295                 300
Lys Leu Gly Glu Ala Ala Val Arg Ala Ala Lys Ala Val Asn Tyr
                305                 310                 315
Val Gly Ala Gly Thr Val Glu Phe Ile Met Asp Ser Lys His Asn
                320                 325                 330
Phe Cys Phe Met Glu Met Asn Thr Arg Leu Gln Val Glu His Pro
                335                 340                 345
Val Thr Glu Met Ile Thr Gly Thr Asp Leu Val Glu Trp Gln Leu
                350                 355                 360
Arg Ile Ala Ala Gly Glu Lys Ile Pro Leu Ser Gln Glu Glu Ile
                365                 370                 375
Thr Leu Gln Gly His Ala Phe Glu Ala Arg Ile Tyr Ala Glu Asp
                380                 385                 390
Pro Ser Asn Asn Phe Met Pro Val Ala Gly Pro Leu Val His Leu
                395                 400                 405
Ser Thr Pro Arg Ala Asp Pro Ser Thr Arg Ile Glu Thr Gly Val
                410                 415                 420
Arg Gln Gly Asp Glu Val Ser Val His Tyr Asp Pro Met Ile Ala
                425                 430                 435
Lys Leu Val Val Trp Ala Ala Asp Arg Gln Ala Ala Leu Thr Lys
                440                 445                 450
Leu Arg Tyr Ser Leu Arg Gln Tyr Asn Ile Val Gly Leu Pro Thr
                455                 460                 465
Asn Ile Asp Phe Leu Leu Asn Leu Ser Gly His Pro Glu Phe Glu
                470                 475                 480
Ala Gly Asn Val His Thr Asp Phe Ile Pro Gln His His Lys Gln
                485                 490                 495
Leu Leu Leu Ser Arg Lys Ala Ala Ala Lys Glu Ser Leu Cys Gln
                500                 505                 510
Ala Ala Leu Gly Leu Ile Leu Lys Glu Lys Ala Met Thr Asp Thr
                515                 520                 525
Phe Thr Leu Gln Ala His Asp Gln Phe Ser Pro Phe Ser Ser Ser
                530                 535                 540
Ser Gly Arg Arg Leu Asn Ile Ser Tyr Thr Arg Asn Met Thr Leu
                545                 550                 555
Lys Asp Gly Lys Asn Asn Val Ala Ile Ala Val Thr Tyr Asn His
                560                 565                 570
Asp Gly Ser Tyr Ser Met Gln Ile Glu Asp Lys Thr Phe Gln Val
                575                 580                 585
Leu Gly Asn Leu Tyr Ser Glu Gly Asp Cys Thr Tyr Leu Lys Cys
                590                 595                 600
Ser Val Asn Gly Val Ala Ser Lys Ala Lys Leu Ile Ile Leu Glu
```

```
                605                 610                 615
Asn Thr Ile Tyr Leu Phe Ser Lys Glu Gly Ser Ile Glu Ile Asp
                620                 625                 630
Ile Pro Val Pro Lys Tyr Leu Ser Ser Val Ser Ser Gln Glu Thr
                635                 640                 645
Gln Gly Gly Pro Leu Ala Pro Met Thr Gly Thr Ile Glu Lys Val
                650                 655                 660
Phe Val Lys Ala Gly Asp Lys Val Lys Ala Gly Asp Ser Leu Met
                665                 670                 675
Val Met Ile Ala Met Lys Met Glu His Thr Ile Lys Ser Pro Lys
                680                 685                 690
Asp Gly Thr Val Lys Lys Val Phe Tyr Arg Glu Gly Ala Gln Ala
                695                 700                 705
Asn Arg His Thr Pro Leu Val Glu Phe Glu Glu Glu Glu Ser Asp
                710                 715                 720
Lys Arg Glu Ser Glu
                725
```

<210> 8
<211> 644
<212> PRT
<213> Homo sapiens

<220>
<221> misc_feature
<223> Incyte ID No: 2844928CD1

<400> 8

```
Met Ala Val Tyr Val Gly Met Leu Arg Leu Gly Arg Leu Cys Ala
  1             5                   10                  15
Gly Ser Ser Gly Val Leu Gly Ala Arg Ala Ala Leu Ser Arg Ser
                20                  25                  30
Trp Gln Glu Ala Arg Leu Gln Gly Val Arg Phe Leu Ser Ser Arg
                35                  40                  45
Glu Val Asp Arg Met Val Ser Thr Pro Ile Gly Gly Leu Ser Tyr
                50                  55                  60
Val Gln Gly Cys Thr Lys Lys His Leu Asn Ser Lys Thr Val Gly
                65                  70                  75
Gln Cys Leu Glu Thr Thr Ala Gln Arg Val Pro Glu Arg Glu Ala
                80                  85                  90
Leu Val Val Leu His Glu Asp Val Arg Leu Thr Phe Ala Gln Leu
                95                  100                 105
Lys Glu Glu Val Asp Lys Ala Ala Ser Gly Leu Leu Ser Ile Gly
                110                 115                 120
Leu Cys Lys Gly Asp Arg Leu Gly Met Trp Gly Pro Asn Ser Tyr
                125                 130                 135
Ala Trp Val Leu Met Gln Leu Ala Thr Ala Gln Ala Gly Ile Ile
                140                 145                 150
Leu Val Ser Val Asn Pro Ala Tyr Gln Ala Met Glu Leu Glu Tyr
                155                 160                 165
Val Leu Lys Lys Val Gly Cys Lys Ala Leu Val Phe Pro Lys Gln
                170                 175                 180
Phe Lys Thr Gln Gln Tyr Tyr Asn Val Leu Lys Gln Ile Cys Pro
                185                 190                 195
Glu Val Glu Asn Ala Gln Pro Gly Ala Leu Lys Ser Gln Arg Leu
                200                 205                 210
Pro Asp Leu Thr Thr Val Ile Ser Val Asp Ala Pro Leu Pro Gly
                215                 220                 225
Thr Leu Leu Leu Asp Glu Val Val Ala Ala Gly Ser Thr Arg Gln
                230                 235                 240
His Leu Asp Gln Leu Gln Tyr Asn Gln Gln Phe Leu Ser Cys His
                245                 250                 255
Asp Pro Ile Asn Ile Gln Phe Thr Ser Gly Thr Thr Gly Ser Pro
                260                 265                 270
Lys Gly Ala Thr Leu Ser His Tyr Asn Ile Val Asn Asn Ser Asn
                275                 280                 285
```

```
Ile Leu Gly Glu Arg Leu Lys Leu His Glu Lys Thr Pro Glu Gln
                290                 295                 300
Leu Arg Met Ile Leu Pro Asn Pro Leu Tyr His Cys Leu Gly Ser
                305                 310                 315
Val Ala Gly Thr Met Met Cys Leu Met Tyr Gly Ala Thr Leu Ile
                320                 325                 330
Leu Ala Ser Pro Ile Phe Asn Gly Lys Lys Ala Leu Glu Ala Ile
                335                 340                 345
Ser Arg Glu Arg Gly Thr Phe Leu Tyr Gly Thr Pro Thr Met Phe
                350                 355                 360
Val Asp Ile Leu Asn Gln Pro Asp Phe Ser Ser Tyr Asp Ile Ser
                365                 370                 375
Thr Met Cys Gly Gly Val Ile Ala Gly Ser Pro Ala Pro Pro Glu
                380                 385                 390
Leu Ile Arg Ala Ile Ile Asn Lys Ile Asn Met Lys Asp Leu Val
                395                 400                 405
Val Ala Tyr Gly Thr Thr Glu Asn Ser Pro Val Thr Phe Ala His
                410                 415                 420
Phe Pro Glu Asp Thr Val Glu Gln Lys Ala Glu Ser Val Gly Arg
                425                 430                 435
Ile Met Pro His Thr Glu Ala Arg Ile Met Asn Met Glu Ala Gly
                440                 445                 450
Thr Leu Ala Lys Leu Asn Thr Pro Gly Glu Leu Cys Ile Arg Gly
                455                 460                 465
Tyr Cys Val Met Leu Gly Tyr Trp Gly Glu Pro Gln Lys Thr Glu
                470                 475                 480
Glu Ala Val Asp Gln Asp Lys Trp Tyr Trp Thr Gly Asp Val Ala
                485                 490                 495
Thr Met Asn Glu Gln Gly Phe Cys Lys Ile Val Gly Arg Ser Lys
                500                 505                 510
Asp Met Ile Ile Arg Gly Gly Glu Asn Ile Tyr Pro Ala Glu Leu
                515                 520                 525
Glu Asp Phe Phe His Thr His Pro Lys Val Gln Glu Val Gln Val
                530                 535                 540
Arg His Leu Ala Gln Val Ser Pro Gln Lys Gln Glu Thr His Met
                545                 550                 555
Asn Thr Val Met Ser Asp Ile Phe Leu Trp Pro Trp Asn Val Val
                560                 565                 570
Gly Val Lys Asp Asp Arg Met Gly Glu Glu Ile Cys Ala Cys Ile
                575                 580                 585
Arg Leu Lys Asp Gly Glu Glu Thr Thr Val Glu Glu Ile Lys Ala
                590                 595                 600
Phe Cys Lys Gly Lys Ile Ser His Phe Lys Ile Pro Lys Tyr Ile
                605                 610                 615
Val Phe Val Thr Asn Tyr Pro Leu Thr Ile Ser Gly Lys Ile Gln
                620                 625                 630
Lys Phe Lys Leu Arg Glu Gln Met Glu Arg His Leu Asn Leu
                635                 640
```

<210> 9
<211> 504
<212> PRT
<213> Homo sapiens

<220>
<221> misc_feature
<223> Incyte ID No: 3231586CD1

<400> 9

```
Met Phe Pro Arg Glu Lys Thr Trp Asn Ile Ser Phe Ala Gly Cys
  1               5                  10                  15
Gly Phe Leu Gly Val Tyr Tyr Val Gly Val Ala Ser Cys Leu Arg
                 20                  25                  30
Glu His Ala Pro Phe Leu Val Ala Asn Ala Thr His Ile Tyr Gly
                 35                  40                  45
Ala Ser Ala Gly Ala Leu Thr Ala Thr Ala Leu Val Thr Gly Val
```

```
                    50                  55                  60
Cys Leu Gly Glu Ala Gly Ala Lys Phe Ile Glu Val Ser Lys Glu
                65                  70                  75
Ala Arg Lys Arg Phe Leu Gly Pro Leu His Pro Ser Phe Asn Leu
                80                  85                  90
Val Lys Ile Ile Arg Ser Phe Leu Leu Lys Val Leu Pro Ala Asp
                95                  100                 105
Ser His Glu His Ala Ser Gly Arg Leu Gly Ile Ser Leu Thr Arg
                110                 115                 120
Val Ser Asp Gly Glu Asn Val Ile Ile Ser His Phe Asn Ser Lys
                125                 130                 135
Asp Glu Leu Ile Gln Ala Asn Val Cys Ser Gly Phe Ile Pro Val
                140                 145                 150
Tyr Cys Gly Leu Ile Pro Pro Ser Leu Gln Gly Val Arg Tyr Val
                155                 160                 165
Asp Gly Gly Ile Ser Asp Asn Leu Pro Leu Tyr Glu Leu Lys Asn
                170                 175                 180
Thr Ile Thr Val Ser Pro Phe Ser Gly Glu Ser Asp Ile Cys Pro
                185                 190                 195
Gln Asp Ser Ser Thr Asn Ile His Glu Leu Arg Val Thr Asn Thr
                200                 205                 210
Ser Ile Gln Phe Asn Leu Arg Asn Leu Tyr Arg Leu Ser Lys Ala
                215                 220                 225
Leu Phe Pro Pro Glu Pro Leu Val Leu Arg Glu Met Cys Lys Gln
                230                 235                 240
Gly Tyr Arg Asp Gly Leu Arg Phe Leu Gln Arg Asn Gly Leu Leu
                245                 250                 255
Asn Arg Pro Asn Pro Leu Leu Ala Leu Pro Pro Ala Arg Pro His
                260                 265                 270
Gly Pro Glu Asp Lys Asp Gln Ala Val Glu Ser Ala Gln Ala Glu
                275                 280                 285
Asp Tyr Ser Gln Leu Pro Gly Glu Asp His Ile Leu Glu His Leu
                290                 295                 300
Pro Ala Arg Leu Asn Glu Ala Leu Leu Glu Ala Cys Val Glu Pro
                305                 310                 315
Thr Asp Leu Leu Thr Thr Leu Ser Asn Met Leu Pro Val Arg Leu
                320                 325                 330
Ala Thr Ala Met Met Val Pro Tyr Thr Leu Pro Leu Glu Ser Ala
                335                 340                 345
Leu Ser Phe Thr Ile Arg Leu Leu Glu Trp Leu Pro Asp Val Pro
                350                 355                 360
Glu Asp Ile Arg Trp Met Lys Glu Gln Thr Gly Ser Ile Cys Gln
                365                 370                 375
Tyr Leu Val Met Arg Ala Lys Arg Lys Leu Gly Arg His Leu Pro
                380                 385                 390
Ser Arg Leu Pro Glu Gln Val Glu Leu Arg Arg Val Gln Ser Leu
                395                 400                 405
Pro Ser Val Pro Leu Ser Cys Ala Ala Tyr Arg Glu Ala Leu Pro
                410                 415                 420
Gly Trp Met Arg Asn Asn Leu Ser Leu Gly Asp Ala Leu Ala Lys
                425                 430                 435
Trp Glu Glu Cys Gln Arg Gln Leu Leu Leu Gly Leu Phe Cys Thr
                440                 445                 450
Asn Val Ala Phe Pro Pro Glu Ala Leu Arg Met Arg Ala Pro Ala
                455                 460                 465
Asp Pro Ala Pro Ala Pro Ala Asp Pro Ala Ser Pro Gln His Gln
                470                 475                 480
Leu Ala Gly Pro Ala Pro Leu Leu Ser Thr Pro Ala Pro Glu Ala
                485                 490                 495
Arg Pro Val Ile Gly Ala Leu Gly Leu
                500
```

<210> 10
<211> 489
<212> PRT
<213> Homo sapiens

<220>
<221> misc_feature
<223> Incyte ID No: 3580770CD1

<400> 10
Met Lys Tyr Ile Leu Val Thr Gly Gly Val Ile Ser Gly Ile Gly
1 5 10 15
Lys Gly Ile Ile Ala Ser Ser Ile Gly Thr Ile Leu Lys Ser Cys
20 25 30
Gly Leu Arg Val Thr Ala Ile Lys Ile Asp Pro Tyr Ile Asn Ile
35 40 45
Asp Ala Gly Thr Phe Ser Pro Tyr Glu His Gly Glu Val Phe Val
50 55 60
Leu Asn Asp Gly Gly Glu Val Asp Leu Asp Leu Gly Asp Tyr Glu
65 70 75
Arg Phe Leu Asp Ile Asn Leu Tyr Lys Asp Thr Ile Val Thr Thr
80 85 90
Gly Lys Ile Tyr Gln His Val Ile Asn Lys Glu Arg Arg Gly Asp
95 100 105
Tyr Leu Gly Lys Thr Val Gln Val Val Pro His Ile Thr Asp Ala
110 115 120
Val Gln Glu Trp Val Met Asn Gln Ala Lys Val Pro Val Asp Gly
125 130 135
Asn Lys Glu Glu Pro Gln Ile Cys Val Ile Glu Leu Gly Gly Thr
140 145 150
Ile Gly Asp Ile Glu Gly Met Pro Phe Val Glu Ala Phe Arg Gln
155 160 165
Phe Gln Phe Lys Ala Lys Arg Glu Asn Phe Cys Asn Ile His Val
170 175 180
Ser Leu Val Pro Gln Leu Ser Ala Thr Gly Glu Gln Lys Thr Lys
185 190 195
Pro Thr Gln Asn Ser Val Arg Ala Leu Arg Gly Leu Gly Leu Ser
200 205 210
Pro Asp Leu Ile Val Cys Arg Ser Ser Thr Pro Ile Glu Met Ala
215 220 225
Val Lys Glu Lys Ile Ser Met Phe Cys His Val Asn Pro Glu Gln
230 235 240
Val Ile Cys Ile His Asp Val Ser Ser Thr Tyr Arg Val Pro Val
245 250 255
Leu Leu Glu Glu Gln Ser Ile Val Lys Tyr Phe Lys Glu Arg Leu
260 265 270
His Leu Pro Ile Gly Asp Ser Ala Ser Asn Leu Leu Phe Lys Trp
275 280 285
Arg Asn Met Ala Asp Arg Tyr Glu Arg Leu Gln Lys Ile Cys Ser
290 295 300
Ile Ala Leu Val Gly Lys Tyr Thr Lys Leu Arg Asp Cys Tyr Ala
305 310 315
Ser Val Phe Lys Ala Leu Glu His Ser Ala Leu Ala Ile Asn His
320 325 330
Lys Leu Asn Leu Met Val Ile Asp Met Pro Glu His Asn Pro Gly
335 340 345
Asn Leu Gly Gly Thr Met Arg Leu Gly Ile Arg Arg Thr Val Phe
350 355 360
Lys Thr Glu Asn Ser Ile Leu Arg Lys Leu Tyr Gly Asp Val Pro
365 370 375
Phe Ile Glu Glu Arg His Arg His Arg Phe Glu Val Asn Pro Asn
380 385 390
Leu Ile Lys Gln Phe Glu Gln Asn Asp Leu Ser Phe Val Gly Gln
395 400 405
Asp Val Asp Gly Asp Arg Met Glu Ile Ile Glu Leu Ala Asn His
410 415 420
Pro Tyr Phe Val Gly Val Gln Phe His Pro Glu Phe Ser Ser Arg
425 430 435
Pro Met Lys Pro Ser Pro Pro Tyr Leu Gly Leu Leu Leu Ala Ala
440 445 450

Thr Gly Asn Leu Asn Ala Tyr Leu Gln Gln Gly Cys Lys Leu Ser
455 460 465
Ser Ser Asp Arg-Tyr Ser Asp Ala Ser Asp Asp Ser Phe Ser Glu
470 475 480
Pro Arg Ile Ala Glu Leu Glu Ile Ser
485

<210> 11
<211> 258
<212> PRT
<213> Homo sapiens

<220>
<221> misc_feature
<223> Incyte ID No: 3778612CD1

<400> 11
Met Glu Arg Gln Lys Arg Lys Ala Asp Ile Glu Lys Gly Leu Gln
1 5 10 15
Phe Ile Gln Ser Thr Leu Pro Leu Lys Gln Glu Glu Tyr Glu Ala
20 25 30
Phe Leu Leu Lys Leu Val Gln Asn Leu Phe Ala Glu Gly Asn Asp
35 40 45
Leu Phe Arg Glu Lys Asp Tyr Lys Gln Ala Leu Val Gln Tyr Met
50 55 60
Glu Gly Leu Asn Val Ala Asp Tyr Ala Ala Ser Asp Gln Val Ala
65 70 75
Leu Pro Arg Glu Leu Leu Cys Lys Leu His Val Asn Arg Ala Ala
80 85 90
Cys Tyr Phe Thr Met Gly Leu Tyr Glu Lys Ala Leu Glu Asp Ser
95 100 105
Glu Lys Ala Leu Gly Pro Asp Ser Glu Ser Ile Arg Ala Leu Phe
110 115 120
Arg Lys Ala Arg Ala Leu Asn Glu Leu Gly Arg His Lys Glu Ala
125 130 135
Tyr Glu Cys Ser Ser Arg Cys Ser Leu Ala Leu Pro His Asp Glu
140 145 150
Ser Val Thr Gln Leu Gly Gln Gly Pro Leu Gly Ser Gly Ala Ser
155 160 165
Trp Pro Gly Gln Ser Trp Ser Pro His Arg Val Arg Lys Arg Glu
170 175 180
Trp Glu Ala Glu Cys Asp Gly Glu Glu Gly Gln Glu Asp Pro Phe
185 190 195
Asn Asp Glu Gly Asn Tyr Phe Ser Cys Glu Pro Ser Arg Ala Pro
200 205 210
Gly Trp Glu Ala Gln Arg Thr Glu Ser Gly Thr Cys Val Pro Pro
215 220 225
Gly Arg Gln Gly Gln Asp Gly Met Ala Ser Met Gly Ala Gly Trp
230 235 240
Val Gly Arg Asp Ala Ala Phe Leu Ser Trp Ala Val Ile Asn Leu
245 250 255
Met Val Leu

<210> 12
<211> 555
<212> PRT
<213> Homo sapiens

<220>
<221> misc_feature
<223> Incyte ID No: 4574912CD1

<220>
<221> unsure
<222> 63-65
<223> unknown or other

<400> 12

```
Met Glu Gly Ala Val Leu Glu Ala Gly Gly Ala Arg Cys Phe Cys
1               5                   10                  15
Arg Phe Gly Cys Glu Leu Ser Lys Tyr Glu Asn Pro Gly Tyr Ser
            20                  25                  30
Ser Pro Arg Ser Asp Tyr Phe Lys Asn Tyr Met Ile Ile Ile Thr
        35                  40                  45
Gln Asn Arg Met Ser Phe Leu Ala Asn Met Phe His Thr Met Asp
    50                  55                  60
Cys Val Xaa Xaa Xaa Arg Tyr Ser Cys Gly Pro Thr Val Tyr Asp
65                  70                  75
His Ala His Leu Gly His Ala Cys Ser Tyr Val Arg Phe Asp Ile
80                  85                  90
Ile Arg Arg Ile Leu Thr Lys Val Phe Gly Cys Ser Ile Val Met
95                  100                 105
Val Met Gly Ile Thr Asp Val Asp Asp Lys Ile Ile Lys Arg Ala
110                 115                 120
Asn Glu Met Asn Ile Ser Pro Ala Ser Leu Ala Ser Leu Tyr Glu
125                 130                 135
Glu Asp Phe Lys Gln Asp Met Ala Ala Leu Lys Val Leu Pro Pro
140                 145                 150
Thr Val Tyr Leu Arg Val Thr Glu Asn Ile Pro Gln Ile Ile Ser
155                 160                 165
Phe Ile Glu Gly Ile Ile Ala Arg Gly Asn Ala Tyr Ser Thr Ala
170                 175                 180
Lys Gly Asn Val Tyr Phe Asp Leu Lys Ser Arg Gly Asp Lys Tyr
185                 190                 195
Gly Lys Leu Val Gly Val Val Pro Gly Pro Val Gly Glu Pro Ala
200                 205                 210
Asp Ser Asp Lys Arg His Ala Ser Asp Phe Ala Leu Trp Lys Ala
215                 220                 225
Ala Lys Pro Gln Glu Val Phe Trp Ala Ser Pro Trp Gly Pro Gly
230                 235                 240
Arg Pro Gly Trp His Ile Glu Cys Ser Ala Ile Ala Ser Met Val
245                 250                 255
Phe Gly Ser Gln Leu Asp Ile His Ser Gly Gly Ile Asp Leu Ala
260                 265                 270
Phe Pro His His Glu Asn Glu Ile Ala Gln Cys Glu Val Phe His
275                 280                 285
Gln Cys Glu Gln Trp Gly Asn Tyr Phe Leu His Ser Gly His Leu
290                 295                 300
His Ala Lys Gly Lys Glu Glu Lys Met Ser Lys Ser Leu Lys Asn
305                 310                 315
Tyr Ile Thr Ile Lys Asp Phe Leu Lys Thr Phe Ser Pro Asp Val
320                 325                 330
Phe Arg Phe Phe Cys Leu Arg Ser Ser Tyr Arg Ser Ala Ile Asp
335                 340                 345
Tyr Ser Asp Ser Ala Met Leu Gln Ala Gln Gln Leu Leu Leu Gly
350                 355                 360
Leu Gly Ser Phe Leu Glu Asp Ala Arg Ala Tyr Met Lys Gly Gln
365                 370                 375
Leu Ala Cys Gly Ser Val Arg Glu Ala Met Leu Trp Glu Arg Leu
380                 385                 390
Ser Ser Thr Lys Arg Ala Val Lys Ala Ala Leu Ala Asp Asp Phe
395                 400                 405
Asp Thr Pro Arg Val Val Asp Ala Ile Leu Gly Leu Ala His His
410                 415                 420
Gly Asn Gly Gln Leu Arg Ala Ser Leu Lys Glu Pro Glu Gly Pro
425                 430                 435
Arg Ser Pro Ala Val Phe Gly Ala Ile Ile Ser Tyr Phe Glu Gln
440                 445                 450
Phe Phe Glu Thr Val Gly Ile Ser Leu Ala Asn Gln Gln Tyr Val
455                 460                 465
Ser Gly Asp Gly Ser Glu Ala Thr Leu His Gly Val Val Asp Glu
470                 475                 480
```

Leu Val Arg Phe Arg Gln Lys Val Arg Gln Phe Ala Leu Ala Met
485 490 495
Pro Glu Ala Thr Gly Asp Ala Arg Arg Gln Gln Leu Leu Glu Arg
500 505 510
Gln Pro Leu Leu Glu Ala Cys Asp Thr Leu Arg Arg Gly Leu Thr
515 520 525
Ala His Gly Ile Asn Ile Lys Asp Arg Ser Ser Thr Thr Ser Thr
530 535 540
Trp Glu Leu Leu Asp Gln Arg Thr Lys Asp Gln Lys Ser Ala Gly
545 550 555

<210> 13
<211> 463
<212> PRT
<213> Homo sapiens

<220>
<221> misc_feature
<223> Incyte ID No: 5630806CD1

<400> 13
Met Ala Ala Ser Met Phe Tyr Gly Arg Leu Val Ala Val Ala Thr
1 5 10 15
Leu Arg Asn His Arg Pro Arg Thr Ala Gln Arg Ala Ala Ala Gln
20 25 30
Val Leu Gly Ser Ser Gly Leu Phe Asn Asn His Gly Leu Gln Val
35 40 45
Gln Gln Gln Gln Gln Arg Asn Leu Ser Leu His Glu Tyr Met Ser
50 55 60
Met Glu Leu Leu Gln Glu Ala Gly Val Ser Val Pro Lys Gly Tyr
65 70 75
Val Ala Lys Ser Pro Asp Glu Ala Tyr Ala Ile Ala Lys Lys Leu
80 85 90
Gly Ser Lys Asp Val Val Ile Lys Ala Gln Val Leu Ala Gly Gly
95 100 105
Arg Gly Lys Gly Thr Phe Glu Ser Gly Leu Lys Gly Gly Val Lys
110 115 120
Ile Val Phe Ser Pro Glu Glu Ala Lys Ala Val Ser Ser Gln Met
125 130 135
Ile Gly Lys Lys Leu Phe Thr Lys Gln Thr Gly Glu Lys Gly Arg
140 145 150
Ile Cys Asn Gln Val Leu Val Cys Glu Arg Lys Tyr Pro Arg Arg
155 160 165
Glu Tyr Tyr Phe Ala Ile Thr Met Glu Arg Ser Phe Gln Gly Pro
170 175 180
Val Leu Ile Gly Ser Ser His Gly Gly Val Asn Ile Glu Asp Val
185 190 195
Ala Ala Glu Thr Pro Glu Ala Ile Ile Lys Glu Pro Ile Asp Ile
200 205 210
Glu Glu Gly Ile Lys Lys Glu Gln Ala Leu Gln Leu Ala Gln Lys
215 220 225
Met Gly Phe Pro Pro Asn Ile Val Glu Ser Ala Ala Glu Asn Met
230 235 240
Val Lys Leu Tyr Ser Leu Phe Leu Lys Tyr Asp Ala Thr Met Ile
245 250 255
Glu Ile Asn Pro Met Val Glu Asp Ser Asp Gly Ala Val Leu Cys
260 265 270
Met Asp Ala Lys Ile Asn Phe Asp Ser Asn Ser Ala Tyr Arg Gln
275 280 285
Lys Lys Ile Phe Asp Leu Gln Asp Trp Thr Gln Glu Asp Glu Arg
290 295 300
Asp Lys Asp Ala Ala Lys Ala Asn Leu Asn Tyr Ile Gly Leu Asp
305 310 315
Gly Asn Ile Gly Cys Leu Val Asn Gly Ala Gly Leu Ala Met Ala
320 325 330

Thr Met Asp Ile Ile Lys Leu His Gly Gly Thr Pro Ala Asn Phe
335 340 345
Leu Asp Val Gly Gly Gly Ala Thr Val His Gln Val Thr Glu Ala
350 355 360
Phe Lys Leu Ile Thr Ser Asp Lys Lys Val Leu Ala Ile Leu Val
365 370 375
Asn Ile Phe Gly Gly Ile Met Arg Cys Asp Val Ile Ala Gln Gly
380 385 390
Ile Val Met Ala Val Lys Asp Leu Glu Ile Lys Ile Pro Val Val
395 400 405
Val Arg Leu Gln Gly Thr Arg Val Asp Asp Ala Lys Ala Leu Ile
410 415 420
Ala Asp Ser Gly Leu Lys Ile Leu Ala Cys Asp Asp Leu Asp Glu
425 430 435
Ala Ala Arg Met Val Val Lys Leu Ser Glu Ile Val Thr Leu Ala
440 445 450
Lys Gln Ala His Val Asp Val Lys Phe Gln Leu Pro Ile
455 460

<210> 14
<211> 399
<212> PRT
<213> Homo sapiens

<220>
<221> misc_feature
<223> Incyte ID No: 5854855CD1

<400> 14
Met Tyr Phe Gly Ala Val Ser Trp Leu Cys Gly Pro Arg Ala Pro
1 5 10 15
Asp Glu Val Ser Arg Arg Pro Asp Pro Arg Lys Gly Gln Leu Gly
20 25 30
Val Ala Phe Val Leu Leu Pro Pro His Ser Glu Gly Ala Arg Val
35 40 45
Phe Gly Ala Leu Gly Pro Ile Gly Pro Ser Ser Pro Gly Leu Thr
50 55 60
Leu Gly Gly Leu Ala Val Ser Glu His Arg Leu Ser Asn Lys Leu
65 70 75
Leu Ala Trp Ser Gly Val Leu Glu Trp Gln Glu Lys Arg Arg Pro
80 85 90
Tyr Ser Asp Ser Thr Ala Lys Leu Lys Arg Thr Leu Pro Cys Gln
95 100 105
Ala Tyr Val Asn Gln Gly Glu Asn Leu Glu Thr Asp Gln Trp Pro
110 115 120
Gln Lys Leu Ile Met Gln Leu Ile Pro Gln Gln Leu Leu Thr Thr
125 130 135
Leu Gly Pro Leu Phe Arg Asn Ser Gln Leu Ala Gln Phe His Phe
140 145 150
Thr Asn Arg Asp Cys Asp Ser Leu Lys Gly Leu Cys Arg Ile Met
155 160 165
Gly Asn Gly Phe Ala Gly Cys Met Leu Phe Pro His Ile Ser Pro
170 175 180
Cys Glu Val Arg Val Leu Met Leu Leu Tyr Ser Ser Lys Lys Lys
185 190 195
Ile Phe Met Gly Leu Ile Pro Tyr Asp Gln Ser Gly Phe Val Ser
200 205 210
Ala Ile Arg Gln Val Ile Thr Thr Arg Lys Gln Ala Val Gly Pro
215 220 225
Gly Gly Val Asn Ser Gly Pro Val Gln Ile Val Asn Asn Lys Phe
230 235 240
Leu Ala Trp Ser Gly Val Met Glu Trp Gln Glu Pro Arg Pro Glu
245 250 255
Pro Asn Ser Arg Ser Lys Arg Trp Leu Pro Ser His Val Tyr Val
260 265 270
Asn Gln Gly Glu Ile Leu Arg Thr Glu Gln Trp Pro Arg Lys Leu

```
                    275                     280                 285
Tyr Met Gln Leu Ile Pro Gln Gln Leu Leu Thr Thr Leu Val Pro
                290                     295                 300
Leu Phe Arg Asn Ser Arg Leu Val Gln Phe His Phe Thr Lys Asp
                305                     310                 315
Leu Glu Thr Leu Lys Ser Leu Cys Arg Ile Met Asp Asn Gly Phe
                320                     325                 330
Ala Gly Cys Val His Phe Ser Tyr Lys Ala Ser Cys Glu Ile Arg
                335                     340                 345
Val Leu Met Leu Leu Tyr Ser Ser Glu Lys Lys Ile Phe Ile Gly
                350                     355                 360
Leu Ile Pro His Asp Gln Gly Asn Phe Val Asn Gly Ile Arg Arg
                365                     370                 375
Val Ile Ala Asn Gln Gln Gln Val Leu Gln Arg Asn Leu Glu Gln
                380                     385                 390
Glu Gln Gln Gln Arg Gly Met Gly Gly
                395
<210> 15
<211> 339
<212> PRT
<213> Homo sapiens

<220>
<221> misc_feature
<223> Incyte ID No: 5993973CD1

<400> 15
Met Val Ser Gly Cys Gln Thr Arg Ser Ile Leu Glu Tyr Leu Arg
  1               5                  10                  15
Val Gly Gly Arg Gly Gly Gly Lys Gly Lys Gly Arg Ala Glu Gly
                 20                  25                  30
Ser Glu Lys Glu Glu Ser Arg Arg Lys Arg Arg Glu Arg Lys Gln
                 35                  40                  45
Arg Arg Glu Gly Gly Asp Gly Glu Glu Gln Asp Val Gly Asp Ala
                 50                  55                  60
Gly Arg Leu Leu Leu Arg Val Leu His Val Ser Glu Asn Pro Val
                 65                  70                  75
Pro Leu Thr Val Arg Val Ser Pro Glu Val Arg Asp Val Arg Pro
                 80                  85                  90
Tyr Ile Val Gly Ala Val Val Arg Gly Met Asp Leu Gln Pro Gly
                 95                 100                 105
Asn Ala Leu Lys Arg Phe Leu Thr Ser Gln Thr Lys Leu His Glu
                110                 115                 120
Asp Leu Cys Glu Lys Arg Thr Ala Ala Thr Leu Ala Thr His Glu
                125                 130                 135
Leu Arg Ala Val Lys Gly Pro Leu Leu Tyr Cys Ala Arg Pro Pro
                140                 145                 150
Gln Asp Leu Lys Ile Val Pro Leu Gly Arg Lys Glu Ala Lys Ala
                155                 160                 165
Lys Glu Leu Val Arg Gln Leu Gln Leu Glu Ala Glu Glu Gln Arg
                170                 175                 180
Lys Gln Lys Lys Arg Gln Ser Val Ser Gly Leu His Arg Tyr Leu
                185                 190                 195
His Leu Leu Asp Gly Asn Glu Asn Tyr Pro Cys Leu Val Asp Ala
                200                 205                 210
Asp Gly Asp Val Ile Ser Phe Pro Pro Ile Thr Asn Ser Glu Lys
                215                 220                 225
Thr Lys Val Lys Lys Thr Thr Ser Asp Leu Phe Leu Glu Val Thr
                230                 235                 240
Ser Ala Thr Ser Leu Gln Ile Cys Lys Asp Val Met Asp Ala Leu
                245                 250                 255
Ile Leu Lys Met Ala Glu Met Lys Lys Tyr Thr Leu Glu Asn Lys
                260                 265                 270
Glu Glu Gly Ser Leu Ser Asp Thr Glu Ala Asp Ala Val Ser Gly
                275                 280                 285
```

```
Gln Leu Pro Asp Pro Thr Thr Asn Pro Ser Ala Gly Lys Asp Gly
                290                 295                 300
Pro Ser Leu Leu Val Val Glu Gln Val Arg Val Val Asp Leu Glu
                305                 310                 315
Gly Ser Leu Lys Val Val Tyr Pro Ser Lys Ala Asp Leu Ala Thr
                320                 325                 330
Ala Pro Pro His Val Thr Val Val Arg
                335
<210> 16
<211> 3902
<212> DNA
<213> Homo sapiens

<220>
<221> misc_feature
<223> Incyte ID No: 1806212CB1

<400> 16
ggatgattgc ctcagcaggt gtgaagcgtg tgctttagtt tcgtgggagg cctggcatcc 60
ccgagaggga ggggaaaggt aaccactcct ttgtggaggt cgccagggtc attgtcgtgg 120
atttgcacag tcggctgggc ggtgcaatgg cggaaagaaa aggaacagcc aaagtggact 180
ttttgaagaa gattgagaaa gaaatccaac agaaatggga tactgagaga gtgtttgagg 240
tcaatgcatc taatttagag aaacagacca gcaagggcaa gtattttgta accttcccat 300
acccatatat gaatggacgc cttcatttgg gacacacgtt ttctttatcc aaatgtgagt 360
ttgctgtagg gtaccagcga ttgaaaggaa aatgttgtct gtttcccttt ggcctgcact 420
gtactggaat gcctattaag gcatgtgctg ataagttgaa aagagaaata gagctgtatg 480
gttgcccccc tgattttcca gatgaagaag aggaagagga agaaaccagt gttaaaacag 540
aagatataat aattaaggat aaagctaaag gaaaaaagag taaagctgct gctaaagctg 600
gatcttctaa ataccagtgg ggcattatga aatcccttgg cctgtctgat gaagagatag 660
taaaattttc tgaagcagaa cattggcttg attatttccc gccactggct attcaggatt 720
taaaaagaat gggtttgaag gtagactggc gtcgttcctt catcaccact gatgttaatc 780
cttactatga ttcatttgtc agatggcaat ttttaacatt aagagaaaga aacaaaatta 840
aatttgggaa gcggtataca atttactctc cgaaagatgg acagccttgc atggatcatg 900
atagacaaac tggagagggt gttggacctc aggaatatac tttactcaaa ttgaaggtgc 960
ttgagccata cccatctaaa ttaagtggcc tgaaaggtaa aaatattttc ttggtggctg 1020
ctactctcag acctgagacc atgtttgggc agacaaattg ttgggttcgt cctgatatga 1080
agtacattgg atttgagacg gtgaatggtg atatattcat ctgtacccaa aaagcagcca 1140
ggaatatgtc ataccagggc tttaccaaag acaatggcgt ggtgcctgtt gttaaggaat 1200
taatggggga ggaaattctt ggtgcatcac tttctgcacc tttaacatca tacaaggtga 1260
tctatgttct cccaatgcta actattaagg aggataaagg cactggtgtg gttacaagtg 1320
ttccttccga ctcccctgat gatattgctg ccctcagaga cttgaagaaa aagcaagcct 1380
tacgagcaaa atatggaatt agagatgaca tggtcttgcc atttgagccg gtgccagtca 1440
ttgaaatccc aggttttgga aatctttctg ctgtaaccat ttgtgatgag ttgaaaattc 1500
agagccagaa tgaccgggaa aaacttgcag aagcaaagga gaagatatat ctaaaaggat 1560
tttatgaggg tatcatgttg gtggatggat ttaaaggaca gaaggttcaa gatgtaaaga 1620
agactattca gaaaaagatg attgacgctg gagatgcact tatttacatg gaaccagaga 1680
aacaagtgat gtccaggtcg tcagatgaat gtgttgtggc tctgtgtgac cagtggtact 1740
tggattatgg agaagagaat tggaagaaac agacatctca gtgcttgaag aacctggaaa 1800
cattctgtga ggagaccagg aggaatttttg aagccacctt aggttggcta caagaacatg 1860
cttgctcaag aacttatggt ctaggcactc acctgccttg ggatgagcag tggctgattg 1920
aatcactttc tgactccact atttacatgg cattttacac agttgcacac ctattgcagg 1980
ggggtaactt gcatggacag gcagagtctc cgctgggcat tagaccgcaa cagatgacca 2040
aggaagtttg ggattatgtt ttcttcaagg aggctccatt tcctaagact cagattgcaa 2100
aggaaaaatt agatcagtta aagcaggagt ttgaattctg gtatcctgtt gatcttcgcg 2160
tctctggcaa ggatcttgtt ccaaatcatc tttcatatta cctttataat catgtggcta 2220
tgtggccgga acaaagtgac aaatggccta cagctgtgag agcaaatgga catctcctcc 2280
tgaactctga gaagatgtca aaatccacag gcaacttcct cactttgacc caagctattg 2340
acaaattttc agcagatgga atgcgtttgg ctctggctga tgctggtgac actgtagaag 2400
atgccaactt tgtggaagcc atggcagatg caggtattct ccgtctgtac acctgggtag 2460
agtgggtgaa agaaatggtt gccaactggg acagcctaag aagtggtcct gccagcactt 2520
tcaatgatag agtttttgcc agtgaattga atgcaggaat tataaaaaca gatcaaaact 2580
atgaaaagat gatgtttaaa gaagctttga aaacagggtt ttttgagttt caggccgcaa 2640
aagataagta ccgtgaattg gctgtggaag ggatgcacag agaacttgtg ttccggttta 2700
ttgaagttca gacacttctc ctcgctccat tctgtccaca tttgtgtgag cacatctgga 2760
cactcctggg aaagcctgac tcaattatga atgcttcatg gcctgtggca ggtcctgtta 2820
```

atgaagtttt aatacactcc tcacagtatc ttatggaagt aacacatgac cttagactac 2880
gactcaagaa ctatatgatg ccagctaaag ggaagaagac tgacaaacaa cccctgcaga 2940
agccctcaca ttgcaccatc tatgtggcaa agaactatcc accttggcaa cataccaccc 3000
tgtctgttct acgtaaacac tttgaggcca ataacggaaa actgcctgac aacaaagtca 3060
ttgctagtga actaggcagt atgccagaac tgaagaaata catgaagaaa gtcatgccat 3120
ttgttgccat gattaaggaa aatctggaga agatggggcc tcgtattctg gatttgcaat 3180
tagaatttga tgaaaaggct gtgcttatgg agaatatagt ctatctgact aattcgcttg 3240
agctagaaca catagaagtc aagtttgcct ccgaagcaga agataaaatc agggaagact 3300
gctgtcctgg gaaaccactt aatgttttta gaatagaacc tggtgtgtcc gtttctctgg 3360
tgaatcccca gccatccaat ggccacttct caaccaaaat tgaaatcagg caaggagata 3420
actgtgattc cataatcagg cgtttaatga aaatgaatcg aggaattaaa gacctttcca 3480
aagtgaaact gatgagattt gatgatccac tgttggggcc tcgacgagtt cctgtcctgg 3540
gaaaggagta caccgagaag acccccattt ctgagcatgc tgttttcaat gtggacctca 3600
tgagcaagaa aattcatctg actgagaatg ggataagggt ggatattggc gatacaataa 3660
tctatctggt tcattaaact catgcacatt ggagatttat cctggtttct taggaatact 3720
actactctga ttgtgtctac tgattggcta tcagaacctt aggctggacc taaatagatt 3780
gatttcattt ctaaccatcc aattctgcat gtattcataa ttctatcaag tcatctttga 3840
ttcctggacc taataaattt tttttccctg ttctttgggt gtccaagaaa aaaaaaaaaa 3900
aa 3902

<210> 17
<211> 3317
<212> DNA
<213> Homo sapiens

<220>
<221> misc_feature
<223> Incyte ID No: 2083883CB1

<400> 17
catggcctga ctcgggacag ctcagagcag ggcagaactg gggacactct gggccggcct 60
tctgcctgca tggacgctct gaagccaccc tgtctctgga ggaaccacga gcgagggaag 120
aaggacaggg actcgtgtgg caggaagaac tcagagccgg gaagcccccca ttcactagaa 180
gcactgagag atgcggcccc ctcgcagggt ctgaatttcc tgctgctgtt cacaaagatg 240
ctttttatct ttaacttttt gttttcccca cttccgaccc cggcgttgat ctgcatcctg 300
acatttggag ctgccatctt cttgtggctg atcaccagac ctcaacccgt cttacctctt 360
cttgacctga acaatcagtc tgtgggaatt gagggaggag cacggaaggg ggtttcccag 420
aagaacaatg acctaacaag ttgctgcttc tcagatgcca agactatgta tgaggttttc 480
caaagaggac tcgctgtgtc tgacaatggg ccctgcttgg gatatagaaa accaaaccag 540
ccctacagat ggctatctta caaacaggtg tctgatagag cagagtacct gggttcctgt 600
ctcttgcata aaggttataa atcatcacca gaccagtttg tcggcatctt tgctcagaat 660
aggccagagt ggatcatctc cgaattggct tgttacacgt actctatggt agctgtacct 720
ctgtatgaca ccttgggacc agaagccatc gtacatattg tcaacaaggc tgatatcgcc 780
gtggtgatct gtgacacacc ccaaaaggca ttggtgctga tagggaatgt agagaaaggc 840
ttcacccccga gcctgaaggt gatcatcctt atggaccccct ttgatgatga cctgaagcaa 900
agaggggaga agagtggaat tgagatctta tccctatatg atgctgagaa cctaggcaaa 960
gagcacttca gaaaacctgt gcctcctagc ccagaagacc tgagcgtcat ctgcttcacc 1020
agtgggacca caggtgaccc caaaggagcc atgataaccc atcaaaatat tgtttcaaat 1080
gctgctgcct ttctcaaatg tgtggagcat gcttatgagc ccactcctga tgatgtggcc 1140
atatcctacc tccctctggc tcatatgttt gagaggattg tacaggctgt tgtgtacagc 1200
tgtggagcca gagttggatt cttccaaggg gatattcggt tgctggctga cgacatgaag 1260
actttgaagc ccacattgtt tcccgcggtg cctcgactcc ttaacaggat ctacgataag 1320
gtacaaaatg aggccaagac acccttgaag aagttcttgt tgaagctggc tgtttccagt 1380
aaattcaaag agcttcaaaa gggtatcatc aggcatgata gtttctggga caagctcatc 1440
tttgcaaaga tccaggacag cctgggcgga agggttcgtg taattgtcac tggagctgcc 1500
cccatgtcca cttcagtcat gacattcttc cgggcagcaa tgggatgtca ggtgtatgaa 1560
gcttatggtc aaacagaatg cacaggtggc tgtacattta cattacctgg ggactggaca 1620
tcaggtcacg ttggggtgcc cctggcttgc aattacgtga agctggaaga tgtggctgac 1680
atgaactact ttacagtgaa taatgaagga gaggtctgca tcaagggtac aaacgtgttc 1740
aaaggatacc tgaaggaccc tgagaagaca caggaagccc tggacagtga tggctggctt 1800
cacacaggag acattggtcg ctggctcccg aatggaactc tgaagatcat cgaccgtaaa 1860
aagaacattt tcaagctggc ccaaggagaa tacattgcac cagagaagat agaaaatatc 1920
tacaacagga gtcaaccagt gttacaaatt tttgtacacg gggagagctt acggtcatcc 1980
ttagtaggag tggtggttcc tgacacagat gtacttccct catttgcagc caagcttggg 2040
gtgaagggct cctttgagga actgtgccaa aaccaagttg taagggaagc cattttagaa 2100

gacttgcaga aaattgggaa agaaagtggc cttaaaactt ttgaacaggt caaagccatt 2160
tttcttcatc cagagccatt ttccattgaa aatgggctct tgacaccaac attgaaagca 2220
aagcgaggag agctttccaa atactttcgg acccaaattg acagcctgta tgagcacatc 2280
caggattagg ataaggtact taagtacctg ccggcccact gtgcactgct tgtgagaaaa 2340
tggattaaaa actattctta catttgtttt gcctttcctc ctattttttt ttaacctgtt 2400
aaactctaaa gccatagctt ttgttttata ttgagacata taatgtgtaa acttagttcc 2460
caaataaatc aatcctgtct ttcccatctt cgatgttgct aatattaagg cttcagggct 2520
acttttatca acatgcctgt cttcaagatc ccagtttatg ttctgtgtcc ttcctcatga 2580
tttccaacct taatactatt agtaaccaca agttcaaggg tcaaagggac cctctgtgcc 2640
ttcttctttg ttttgtgata aacataactt gccaacagtc tctatgctta tttacatctt 2700
ctactgttca aactaagaga tttttaaatt ctgaaaaact gcttacaatt catgttttct 2760
agccactcca caaaccacta aaatttagt tttagcctat cactcatgtc aatcatatct 2820
atgagacaaa tgtctccgat gctcttctgc gtaaattaaa ttgtgtactg aagggaaaag 2880
tttgatcata ccaaacattt cctaaactct ctagttagat atctgacttg ggagtattaa 2940
aaattgggtc tatgacatac tgtccaaaag gaatgctgtt cttaaagcat tatttacagt 3000
aggaactggg gagtaaatct gttccctaca gtttgctgct gagctggaag ctgtggggga 3060
aggagttgac aggtgggccc agtgaacttt tccagtaaat gaagcaagca ctgaataaaa 3120
acctcctgaa ctgggaacaa agatctacag gcaagcaaga tgcccacaca acaggcttat 3180
tttctgtgaa ggaaccaact gatctccccc acccttggat tagagttcct gctctacctt 3240
acccacagat aacacatgtt gtttctactt gtaaatgtaa agtctttaaa ataaactatt 3300
acagataaaa aaaaaaa 3317

<210> 18
<211> 1928
<212> DNA
<213> Homo sapiens

<220>
<221> misc_feature
<223> Incyte ID No: 2454288CB1

<400> 18
cccacgcgtc cgcgacacac catgccgact gtcagcgtga agcgtgatct gctcttccaa 60
gccctgggcc gcacctacac tgacgaagaa tttgatgaac tatgttttga atttggtctg 120
gagcttgatg aaattacatc tgagaaggaa ataataagta aagaacaagg taatgtaaag 180
gcagcaggag cctctgatgt tgttctttac aaaattgacg tccctgccaa tagatatgat 240
ctcctgtgtc tggaaggatt ggttcgagga cttcaggtct tcaaagaaag gataaaggct 300
ccagtgtata aacgggtaat gcctgatgga aaaatccaga aattgattat cacagaagag 360
acagctaaga tacgtccttt tgcggtagca gcagttctcc gtaatataaa gtttactaaa 420
gatcgatatg acagcttcat tgaacttcag gagaaattac atcagaatat ttgcaggaaa 480
agagcactgg ttgccattgg tacccatgat ttggacactt tgtcgggccc atttacttat 540
actgcaaagc gtccttcaga tatcaaattc aagcctctaa ataagaccaa ggagtataca 600
gcctgtgaac tgatgaacat atacaagact gacaatcacc tgaaacatta tttacatatc 660
attgaaaaca aacccctgta tccagttatc tatgatagca atggtgtcgt cctttcaatg 720
cctcccatca tcaatgggga tcattccaga ataacagtaa atactagaaa tattttttatt 780
gaatgcacgg gaactgactt tactaaggca aaaatagttc ttgatattat tgtcaccatg 840
ttcagtgaat attgtgagaa tcaatttacg gtcgaagctg ctgaagtggt tttttcctaat 900
ggaaaatcac atacctttcc agaattagct taccgaaagg agatggtgag agctgaccta 960
attaacaaaa aagttggaat cagagaaact ccagaaaatc ttgccaaact tctgaccagg 1020
atgtatttaa aatcagaagt cataggtgat gggaatcaga ttgagattga aatccctcca 1080
accagagctg acattatcca tgcatgtgat attgtagaag atgcagctat tgcttatgga 1140
tataacaaca ttcagatgac tctcccgaaa acttacacca tagctaatca atttcctctt 1200
aataagctca ctgaacttct ccgacatgac atggcagccg ctggcttcac tgaagcactt 1260
acctttgccc tgtgctccca agaagatatt gctgataaac taggtgtgga tatctctgca 1320
acaaaggcag tccacataag taatcctaaa acagctgaat ttcaggtggc acgcactacc 1380
cttcttcctg gcctcctgaa gaccatagca gcaaatcgta agatgcccct tccactgaaa 1440
ctgtttgaaa tctctgacat tgtaataaaa gattctaata cagatgtagg tgcaaaaaac 1500
tacagacatc tctgtgctgt ttattacaac aagaatcctg ggtttgagat cattcatggg 1560
ctgctggaca gaattatgca gttgctcgat gtgcctcctg gtgaagacaa gggggggatat 1620
gtgatcaaag catcagaagg gcctgctttc ttccccgggc gatgtgcaga gatctttgcc 1680
aggggtcaaa gcgtcgggaa gcttggggtc cttcatcctg acgttatcac caaatttgag 1740
ctgaccatgc cctgctcctc cctagaaatc aatattggac ccttttttgtg aagattggtc 1800
tctgtggtgt gattctcttc ccaggtgtcc ctttctcctc ccctagtgtc cttaagtcct 1860
cctccacagg gaacatctat ttgggctttg atgtttaata aagtagaaag cactgtcaaa 1920
aaaaaaaa 1928

<210> 19
<211> 2122
<212> DNA
<213> Homo sapiens

<220>
<221> misc_feature
<223> Incyte ID No: 1513539CB1

<400> 19
caggtcacac gcagccagtc agcattagag ctgagatgcc aagctctcag ccacacatgt 60
gcactcacct ggaacatctg tggagctgtt agaatcgctg ccacttttca tgaaactgaa 120
gtgcatcttt gggtttgcaa ctaaagaaac cagctgctac aatgtcacta acataggatt 180
caaaagccct tcggatttct ggcagtctgt gcatagcacc ctgcctcggg agttggctcc 240
ttgtctagta tttaatacat ccccaaactt ggctttattt tcagctgcct ttgccttcat 300
tgtggtgaaa gatagtgcgg gtgactcaga tgtggtggtg caggagctca agtccatggt 360
ggccaccaag atcgccaaat atgctgtgcc tgatgagatc ctggtggtga aacgtcttcc 420
aaaaaccagg tctgggaagg tcatgcggcg gctcctgagg aagatcatca ctagtgaggc 480
ccaggagctg ggagacacta ccaccttgga ggaccccagc atcatcgcag agatcctgag 540
tgtctaccag aagtgcaagg acaagcaggc tgctgctaag tgagctggca ccttgtgggg 600
ctcttgggat gggcgggcac ccaagccctg gcttgtcctt cccagaaggt acccctgagg 660
ttggcgtctt cctacgtccc agaagcagcc cccaccccac acatgaccca caccgccctc 720
acgtgaagct gggctgagag ccctttctcc catccattgg aggtcccagg agtgtcaccc 780
atggagaggc tatgcgacat ggctagggct ggttctgcca tctgagtttg gtttcctgga 840
atgaaaaggc attgccatct ccattcctct gccctcttga gccagcacag gaaggtgagg 900
ccctgggata gcgcgcctgc tcagataaca cagagctagt tagctagtag caaccgtgtt 960
ttctccagat ctgtctagat acaaaggtca gaaatcttat ttttatactt ttatattgtg 1020
gaagaacagc atgcaacact cacatgtagt gtgtggattt acttgaacat gttcttttta 1080
acatgtagtt atgaaaatct cctttttttgc ctctactggt gaggaaacat gaggatcaga 1140
ggccacattt ttaattattg ttagtgtatt tggaagtctg aattggagat gtttgtacct 1200
ctgtctaaac agttcccttg agaacttcca agcctccggc atcttttcct ggtgagtgtt 1260
tctcctgtgc ttggttgtgt ataatggagc taactcctaa gcggtggggt gaatgtggcc 1320
gccttagttc tgaagctact ccagttatgt tctgtttctt caagctgtga tccagaaaga 1380
tttttgtgcc cccagatgcc tcttgatagg agaggcaaca tactccaaat agttgggttc 1440
ttcagggaag ctattagaaa ctcaggtgac ttgttagagc actaacttgg tcagagccaa 1500
atcctggcaa acgctgcctg accttcactc tgtggttggg gcagtgagaa ccactgaggt 1560
ccaatgatga gacttggagg tctggatcca gtctctcttt gttttaatgt gacttaggtg 1620
ctgtcaacat tagcaagata atggaaatca cgacgccagt gggtgcttac ctcccctgcta 1680
ggcatgcagg ggctggcggt tggcagggga aggaggccca gtgagccggg tcccttaggg 1740
gagggagagt ttgtcctctt tgccccacag tctacccttc agggccttgt ggcagtgcca 1800
gtgttcgggg ggtgtctggg ccactgagta cccactcggt cgtggttgtg ctggcctctt 1860
gggtgagtga acctgtgaag cccaggaggt ggtgttggct gcagggtaca caaatactga 1920
gtggtggtct tttgttacag gcttagcaac aaagctgtgc cctgggcatg gggggctgta 1980
gtgtagctac agttgtgcgt ttgtgaaatg gcttagcttt ccatgttgct gagaggaacc 2040
tggacatggt cccgggcatc tgaatgatct gtaggggagg gagttcaaat aaagctttat 2100
tttgttcatt ttcaaaaaaa aa 2122

<210> 20
<211> 2357
<212> DNA
<213> Homo sapiens

<220>
<221> misc_feature
<223> Incyte ID No: 2148623CB1

<400> 20
gctctttcag ggctgagcca tcctgcgtgt cttgcgctcg gtggaaatgc ccagccgagg 60
gacgcgacca gaggacagct ctgtgctgat ccccaccgac aattcgaccc cacacaagga 120
ggatctaagc agcaagatta aagaacaaaa aattgtggtg gatgaacttt ctaaccttaa 180
gaagaatagg accgaacaga aatgctgtct gagagcaaga atatattgga tgaactgaaa 240
aaagaatacc aagaaataga aaacttagac aagaccaaaa tcaagaaata gtcaacctga 300
tttcacataa caatgtgtgg catttgttgt tctgtaaact tttctgctga gcatttcagt 360
caagatttaa aagaggactt actatataat cttaaacagc ggggacccaa tagtagtaaa 420
caattgttaa agtctgatgt taactaccag tgtttatttt ctgctcacgt cctacacttg 480

aggggtgttt tgactaccca gcctgtggaa gatgaaagag gcaatgtgtt tctatggaat 540
ggagaaattt ttagtggaat aaaggttgaa gctgaagaga atgacactca aattttgttt 600
aattatcttt cctcctgtaa gaatgaatct gagattttgt cactcttctc agaagtacaa 660
ggtccctggt catttatata ttatcaagca tctagtcatt atttatggtt tggtagggat 720
ttttttggtc gccgtagctt gctttggcat tttagtaatt tgggcaagag tttctgcctc 780
tcttcagttg gcacccaaac atctggattg gcaaatcagt ggcaagaagt tccagcatct 840
ggacttttca gaattgatct taagtctact gtcatttcca gatgcattat tttacaactg 900
tatccttgga aatatatttc tagggagaat attattgaag aaaatgttaa tagcctgagt 960
caaatttcag cagacttacc agcatttgta tcagtggtag caaatgaagc caaactgtat 1020
cttgaaaaac ctgttgttcc tttaaatatg atgttgccac aagctgcatt ggagactcat 1080
tgcagtaata tttccaatgt gccacctaca agagagatac ttcaagtctt tcttactgat 1140
gtacacatga aggaagtaat tcagcagttc attgatgtcc tgagtgtagc agtcaagaaa 1200
cgtgtcttgt gtttacctag ggatgaaaac ctgacagcaa atgaagtttt gaaaacgtgt 1260
gataggaaag caaatgttgc aatcctgttt tctgggggca ttgattccat ggttattgca 1320
acccttgctg accgtcatat tcctttagat gaaccaattg atcttcttaa tgtagctttc 1380
atagctgaag aaaagaccat gccaactacc tttaacagag aagggaataa acagaaaaat 1440
aaatgtgaaa taccttcaga agaattctct aaagatgttg ctgctgctgc tgctgacagt 1500
cctaataaac atgtcagtgt accagatcga atcacaggaa gggcgggact aaaggaacta 1560
caagctgtta gcccttcccg aatttggaat tttgttgaaa ttaatgtttc tatggaagaa 1620
ctgcagaaat taagaagaac tcgaatatgt cacttaattc ggccattgga tacagttttg 1680
gatgatagca ttggctgtgc agtctggttt gcttctagag gaattggttg gttagtggcc 1740
caggaaggag tgaaatccta tcagagcaat gcaaaggtag ttctcactgg aattggtgca 1800
gatgagcaac ttgcaggtta ttctcgtcat cgtgtccgct ttcagtcgca tgggctggaa 1860
ggattgaata aggaaataat gatggaactg ggtcgaattt cttctagaaa tcttggtcgt 1920
gatgacagag ttattggtga tcatggaaaa gaagcaagat ttcctttcct ggatgaaaat 1980
gttgtctcct ttctaaattc tctgccgatt tgggaaaaag caaacttgac tttaccccga 2040
ggaattggtg aaaaattact tttacgcctt gcagctgtgg aacttggtct tacagcctct 2100
gctcttctgc ccaaacgggc catgcagttt ggatcaagaa ttgcaaaaat ggaaaaaatt 2160
aatgaaaagg catctgataa atgtggacgg ctccaaatca tgtccttaga aaatctttct 2220
attgaaaagg agactaaatt gtaatgtgat tcacaatgta acaatataaa aataagtttt 2280
tatataatta tataaaagta agatactctg ctgctttact attgtataat atagtagttt 2340
taaagttcaa aaaaaaa 2357

<210> 21
<211> 2136
<212> DNA
<213> Homo sapiens

<220>
<221> misc_feature
<223> Incyte ID No: 2579405CB1

<400> 21
ccgtccactt ggcgagtgag acgctgatgg gaggatggac atactggtgt ctgagtgctc 60
cgcgcggctg ctgcagcagg aagaagagat taaatctctg actgctgaaa ttgaccggtt 120
gaaaaactgt ggctgtttag gagcttctcc aaatttggag cagttacaag aagaaaattt 180
aaaattaaag tatcgactga atattcttcg aaagagtctt caggcagaaa ggaacaaacc 240
aactaaaaat atgattaaca ttattagccg cctacaagag gtctttggtc atgcaattaa 300
ggctgcatat ccagatttgg aaaatcctcc tctgctagtg acaccaagtc agcaggccaa 360
gtttggggac tatcagtgta atagtgctat gggtatttct cagatgctca aaaccaagga 420
acagaaagtt aatccaagag aaattgctga aaacattacc aaacacctcc cagacaatga 480
atgtattgaa aaagttgaaa ttgctggtcc tggttttatt aatgtccact taagaaagga 540
ttttgtatca gaacaattga ccagtcttct agtgaatgga gttcaactac ctgctctggg 600
agagaataaa aaggttatag ttgacttttc ctcccctaat atagctaaag agatgcatgt 660
aggccacctg aggtcaacta tcataggaga gagtataagc cgcctctttg aatttgcagg 720
gtatgacgtg ctcaggttaa atcatgtagg agactggggg acccagtttg gcatgctcat 780
cgctcacctg caagacaaat ttccagatta tctaacagtt tcacctccta ttggggatct 840
tcaggtcttt tataaggaat ctaagaagag gtttgatact gaggaggaat ttaagaagcg 900
agcatatcag tgtgtagttc tgctccaggg taaaaaccca gatattacaa aagcttggaa 960
gcttatctgt gatgtctccc gccaagagtt aaataaaatc tatgatgcat tggacgtctc 1020
tttaatagag agaggggaat ccttctatca agataggatg aatgatattg taaaggaatt 1080
tgaagataga ggatttgtgc aggtggatga tggcagaaag attgtatttg tcccagggtg 1140
ttccatacca ttaaccatag taaaatcaga tggaggttat acctatgata catctgacct 1200
ggctgctatt aaacaaagac tatttgagga aaaagcagat atgattatct atgttgtgga 1260
caatggacaa tctgtgcact tccagacaat atttgctgct gctcaaatga ttggttggta 1320

```
tgaccctaaa gtaactcgag tcttccatgc tggatttggt gtggtgctag gggaagacaa 1380
gaaaaagttt aaaacacgtt cgggtgaaac agtgcgcctc atggatcttc tgggagaagg 1440
actaaaacga tccatggaca agttgaagga aaaagaaaga gacaaggtct taactgcaga 1500
ggaattgaat gctgctcaga catccgttgc atatggctgc atcaaatatg ctgacctttc 1560
ccataaccgg ttgaatgact acatcttctc ctttgacaaa atgctagatg acagaggaaa 1620
tacagctgct tacttgttgt atgccttcac tagaatcagg tctattgcac gtctggccaa 1680
tattgatgaa gaaatgctcc aaaaagctgc tcgagaaacc aagattcttt tggatcatga 1740
gaaggaatgg aaactaggcc ggtgcatttt acggttccct gagattctgc aaaagatttt 1800
agatgactta tttctccaca ctctctgtga ttatatatat gagctggcaa ctgctttcac 1860
agagttctat gatagctgct actgtgtgga gaaagataga cagactggaa aaatattgaa 1920
ggtgaacatg tggcgtatgc tgctatgtga agcagtagct gctgtcatgg ccaaggggtt 1980
tgatatcctg ggaataaaac ctgtccaaag gatgtaatcc ttcataggtt tgaacactgt 2040
gtgtttttac caaagtggcc attggcactg tttgcttttt tacaatcatg tggacacaag 2100
cataagtaaa gaaaatttgt caaccaaaaa aaaaaa                           2136
```

<210> 22
<211> 2480
<212> DNA
<213> Homo sapiens

<220>
<221> misc_feature
<223> Incyte ID No: 2662427CB1

<400> 22

```
ggaacgggtt tggcgtgtgg gacgcagctg cctctgtact ggggagtcac ggagtggccg 60
ggctccaggg acatggcggc ggcctctgcg gtgtcggtgc tgctggtggc ggcggagagg 120
aaccggtggc atcgtctccc gagcctgctc ctgccgccga ggacatgggt gtggaggcaa 180
agaaccatga agtacacaac agccacagga agaaacatta ccaaggtcct cattgcaaac 240
agaggagaaa ttgcctgcag ggtgatgcgc acagccaaaa aactgggtgt acagactgtg 300
gcggtttata gtgaggctga cagaaattcc atgcatgtag atatggcaga tgaagcatat 360
tccatcggcc ccgctccctc ccagcagagc tacctatcta tggagaaaat cattcaagtg 420
gccaagacct ctgctgcaca ggctatccat ccaggatgcc gttttctttc agaaaacatg 480
gaatttgctg aactttgtaa gcaagaagga attattttta taggccctcc tccatctgca 540
attagagaca tgggtataaa gagcacatcc aaatccataa tggctgctgc tggagtacct 600
gttgtggagg gttatcatgg tgaggaccaa tcagaccagt gcctgaagga acacgccagg 660
agaattggct atcctgtcat gattaaagcc gtccggggtg gaggaggaaa aggaatgagg 720
attgttagat cagaacaaga atttcaagaa cagttagagt cagcacggag agaagctaag 780
aagtctttca atgatgatgc tatgctgatc gagaagtttg tagacacacc gaggcatgta 840
gaagtccagg tgtttggtga tcaccatggc aatgctgtgt acttgtttga aagagactgt 900
agtgtgcaga ggcgacatca gaagatcatt gaggaggccc cagcgcctgg tattaaatct 960
gaagtaagaa aaaagctggg agaagctgca gtcagagctg ctaaagctgt aaattatgtt 1020
ggagcaggga ctgtggagtt tattatggac tcaaaacata atttctgttt catggagatg 1080
aatacaaggc tgcaagtgga acatcctgtt actgagatga tcacaggaac tgacttggtg 1140
gagtggcagc ttagaattgc agcaggagag aagattcctt tgagccagga agaaataact 1200
ctgcagggcc atgccttcga agctagaata tatgcagaag atcctagcaa taacttcatg 1260
cctgtggcag gcccattagt gcacctctct actcctcgag cagacccttc caccaggatt 1320
gaaactggag tacggcaagg agacgaagtt tccgtgcatt atgaccccat gattgcgaag 1380
ctggtcgtgt gggcagcaga tcgccaggcg gcattgacaa aactgaggta cagccttcgt 1440
cagtacaata ttgttggact gcccaccaac attgacttct tactcaacct gtctggccac 1500
ccagagtttg aagctgggaa cgtgcacact gatttcatcc ctcaacacca caaacagttg 1560
ttgctcagtc ggaaggctgc agccaaagag tctttatgcc aggcagccct gggtctcatc 1620
ctcaaggaga aagccatgac cgacactttc actcttcagg cacatgatca attctctcca 1680
ttttcgtcta gcagtggaag aagactgaat atctcgtata ccagaaacat gactcttaaa 1740
gatggtaaaa acaatgtagc catagctgta acgtataacc atgatgggtc ttatagcatg 1800
cagattgaag ataaaacttt ccaagtcctt ggtaatcttt acagcgaggg agactgcact 1860
tacctgaaat gttctgttaa tggagttgct agtaaagcga agctgattat cctggaaaac 1920
actatttacc tattttccaa ggaaggaagt attgagattg acattccagt ccccaaatac 1980
ttatcttctg tgagctcaca agaaactcag ggcggcccct tagctcctat gactggaacc 2040
attgaaaagg tgtttgtcaa agctggagac aaagtgaaag cgggagattc cctcatggtt 2100
atgatcgcca tgaagatgga gcataccata aagtctccaa aggatggcac agtaaagaaa 2160
gtgttctaca gagaaggtgc tcaggccaac agacacactc ctttagtcga gtttgaggag 2220
gaagaatcag acaaaaggga atcggaataa actccagcaa ggaaatggcc agttaagtag 2280
tgtcttctct ctccaccaaa aagaggaagt gcctccagct tttctggggg tctcataaag 2340
agcagtttta ctaaatgatt gtatgcttat gctgaacacc tttcatattg gagaatcatg 2400
```

catttgggtc actaattatc tcaaaatatt tcatactaat aaagttgaat tattttttat 2460
tggaagccaa aaaaaaaaaa 2480

<210> 23
<211> 2254
<212> DNA
<213> Homo sapiens

<220>
<221> misc_feature
<223> Incyte ID No: 2844928CB1

<400> 23
ggccgggacg cagggcaaag cgagccatgg ctgtctacgt cgggatgctg cgcctgggga 60
ggctgtgcgc cgggagctcg gggtgctgg gggcccgggc cgccctctct cggagttggc 120
aggaagccag gttgcagggt gtccgcttcc tcagttccag agaggtggat cgcatggtct 180
ccacgcccat cggaggcctc agctacgttc aggggtgcac caaaaagcat cttaacagca 240
agactgtggg ccagtgcctg gagaccacag cacagagggt cccagaacga gaggccttgg 300
tcgtcctcca tgaagacgtc aggttgacct ttgcccaact caaggaggag gtggacaaag 360
ctgcttctgg cctcctgagc attggcctct gcaaaggtga ccggctgggc atgtggggac 420
ctaactccta tgcatgggtg ctcatgcagt tggccaccgc ccaggcgggc atcattctgg 480
tgtctgtgaa cccagcctac caggctatgg aactggagta tgtcctcaag aaggtgggct 540
gcaaggccct tgtgttcccc aagcaattca agacccagca atactacaac gtcctgaagc 600
agatctgtcc agaagtggag aatgcccagc caggggcctt gaagagtcag aggctcccag 660
atctgaccac agtcatctcg gtggatgccc ctttgccggg gaccctgctc ctggatgaag 720
tggtggcggc tggcagcaca cggcagcatc tggaccagct ccaatacaac cagcagttcc 780
tgtcctgcca tgaccccatc aacatccagt tcacctcggg gacaacaggc agccccaagg 840
gggccaccct ctcccactac aacattgtca acaactccaa cattttagga gagcgcctga 900
aactgcatga gaagacacca gagcagttgc ggatgatcct gcccaacccc ctgtaccatt 960
gcctgggttc cgtggcaggc acaatgatgt gtctgatgta cggtgccacc ctcatcctgg 1020
cctctcccat cttcaatggc aagaaggcac tggaggccat cagcagagag agaggcacct 1080
tcctgtatgg tacccccacg atgttcgtgg acattctgaa ccagccagac ttctccagtt 1140
atgacatctc gaccatgtgt ggaggtgtca ttgctgggtc ccctgcacct ccagagttga 1200
tccgagccat catcaacaag ataaatatga aggacctggt ggttgcttat ggaaccacag 1260
agaacagtcc cgtgacattc gcgcacttcc ctgaggacac tgtggagcag aaggcagaaa 1320
gcgtgggcag aattatgcct cacacggagg cccggatcat gaacatggag gcagggacgc 1380
tggcaaagct gaacacgccc ggggagctgt gcatccgagg gtactgcgtc atgctgggct 1440
actggggtga gcctcagaag acagaggaag cagtggatca ggacaagtgg tattggacag 1500
gagatgtcgc cacaatgaat gagcagggct tctgcaagat cgtgggccgc tctaaggata 1560
tgatcatccg gggtggtgag aacatctacc ccgcagagct cgaggacttc tttcacacac 1620
acccgaaggt gcaggaagtg caggtgaggc acttggctca ggtgagcccc cagaaacaag 1680
aaacacacat gaacacggtg atgtctgata tttttctttg gccctggaac gtggtgggag 1740
tgaaggacga tcggatgggg gaagagattt gtgcctgcat tcggctgaag gacggggagg 1800
agaccacggt ggaggagata aaagctttct gcaaagggaa gatctctcac ttcaagattc 1860
cgaagtacat cgtgtttgtc acaaactacc ccctcaccat ttcaggaaag atccagaaat 1920
tcaaacttcg agagcagatg gaacgacatc taaatctgtg aataaagcag caggcctgtc 1980
ctggccggtt ggcttgactc tctcctgtca gaatgcaacc tggctttatg cacctagatg 2040
tccccagcac ccagttctga gccaggcaca tcaaatgtca aggaattgac tgaacgaact 2100
aagagctcct ggatgggtcc gggaactcgc ctgggcacaa ggtgccaaaa ggcaggcagc 2160
ctgcccaggc cctccctcct gtccatcccc cacattcccc tgtctgtcct tgtgatttgg 2220
cataaagagc ttctgttttc aaaaaaaaaa aaaa 2254

<210> 24
<211> 1954
<212> DNA
<213> Homo sapiens

<220>
<221> misc_feature
<223> Incyte ID No: 3231586CB1

<400> 24
atccggtacc acggccagtg caagctaaaa ttaaccctca ctaaagggaa taagcttggc 60
ccccgccgcg atgtttcccc gcgagaagac gtggaacatc tcgttcgcgg gctgcggctt 120
cctcggcgtc tactacgtcg gcgtggcctc ctgcctccgc gagcacgcgc ccttcctggt 180

ggccaacgcc acgcacatct acggcgcctc ggccggggcg ctcacggcca cggcgctggt 240
caccggggtc tgcctgggtg aggctggtgc caagttcatt gaggtatcta aagaggcccg 300
gaagcggttc ctgggccccc tgcacccctc cttcaacctg gtaaagatca tccgcagttt 360
cctgctgaag gtcctgcctg ctgatagcca tgagcatgcc agtgggcgcc tgggcatctc 420
cctgacccgc gtgtcagacg gcgagaatgt cattatatcc cacttcaact ccaaggacga 480
gctcatccag gccaatgtct gcagcggttt catccccgtg tactgtgggc tcatccctcc 540
ctccctccag gggtgcgct acgtggatgg tggcatttca gacaacctgc cactctatga 600
gcttaagaac accatcacag tgtccccctt ctcgggcgag agtgacatct gtccgcagga 660
cagctccacc aacatccacg agctgcgggt caccaacacc agcatccagt tcaacctgcg 720
caacctctac cgcctctcca aggccctctt cccgccggag cccctggtgc tgcgagagat 780
gtgcaagcag ggataccggg atggcctgcg ctttctgcag cggaacggcc tcctgaaccg 840
gcccaacccc ttgctggcgt tgcccccgc ccgcccccac ggcccagagg acaaggacca 900
ggcagtggag agcgcccaag cggaggatta ctcgcagctg ccgggagaag atcacatcct 960
ggagcacctg cccgcccggc tcaatgaggc cctgctggag gcctgcgtgg agcccacgga 1020
cctgctgacc accctctcca acatgctgcc tgtgcgtctg gccacggcca tgatggtgcc 1080
ctacacgctg ccgctggaga gcgctctgtc cttcaccatc cgcttgctgg agtggctgcc 1140
cgacgttccc gaggacatcc ggtggatgaa ggagcagacg ggcagcatct gccagtacct 1200
ggtgatgcgc gccaagagga agctgggcag gcacctgccc tccaggctgc cggagcaggt 1260
ggagctgcgc cgcgtccagt cgctgccgtc cgtgccgctg tcctgcgccg cctacagaga 1320
ggcactgccc ggctggatgc gcaacaacct ctcgctgggg gacgcgctgg ccaagtggga 1380
ggagtgccag cgccagctgc tgctcggcct cttctgcacc aacgtggcct tcccgcccga 1440
agctctgcgc atgcgcgcac ccgccgaccc ggctcccgcc cccgcggacc cagcatcccc 1500
gcagcaccag ctggccgggc ctgccccttt gctgagcacc cctgctcccg aggcccggcc 1560
cgtgatcggg gccctggggc tgtgagaccc cgaccctctc gaggaaccct gcctgagacg 1620
cctccattac cactgcgcag tgagatgagg ggactcacag ttgccaagag ggtctttgc 1680
cgtgggcccc ctcgccagcc actcaccagc tgcatgcact gagaggggag gtttccacac 1740
ccctcccctg ggccgctgag gccccgcgca cctgtgcctt aatcttccct cccctgtgct 1800
gcccgagcac ctcccccgcc cctttactcc tgggaacttt gcagctgccc ttccctcccc 1860
gtttttcatg gcctgctgaa atatgtgtgt gaagaattat ttattttcgc caaagcacat 1920
gtaataaatg ctgcagccca aaaaaaaaaa aaaa 1954

<210> 25
<211> 1937
<212> DNA
<213> Homo sapiens

<220>
<221> misc_feature
<223> Incyte ID No: 3580770CB1

<400> 25
gcagttcctg gggcgtagta ggggatccac aagcgtttgt gaccagtgaa gttctttaca 60
agggtgagat ctgcacggga ggacccgagc gagggtctcg gcttgccagg aagccggggt 120
tccccgggaa gcgtggagtt cacccgcgca ctcgaagtgc ctttgcaaaa ttatatctgg 180
gtgttggcac ccagccacta ttctgccaat gaagtacatc ctggtcacgg gtggggtcat 240
ctcaggcatt ggtaaaggga tcattgccag cagcattgga acgattctaa aatcatgtgg 300
actccgagtt actgccataa aaatcgaccc ctatattaac atcgatgctg gcactttttc 360
accttatgaa cacggtgaag tcttcgtctt aaatgatggt ggagaagttg atttagacct 420
tggagattat gaaagatttt tggatattaa tctttataaa gacacaatag tcaccacggg 480
gaagatatat cagcatgtga tcaataaaga gaggcgtggt gattacctgg ggaaaacagt 540
gcaagttgtc cctcacatta ctgatgctgt ccaggagtgg gttatgaatc aagccaaggt 600
gccggtggat ggtaataagg aagagcccca aatatgcgtt attgagctgg gaggcaccat 660
tggagacatc gaaggaatgc cgtttgtgga ggcgtttaga caattccagt ttaaggcgaa 720
aagagagaat ttctgtaata tccacgttag ccttgtccca cagctcagtg ctaccggaga 780
acaaaaaacc aaacccaccc aaaacagcgt ccgcgcactg aggggtttag gcctgtctcc 840
agatctgatt gtctgccgaa gttcaacgcc cattgagatg gccgtgaagg agaagatttc 900
tatgtttttgt cacgtgaacc ctgaacaggt catatgtatc catgatgttt cttccacata 960
ccgagttcct gtgcttttag aggaacaaag cattgtgaaa tattttaagg agagattgca 1020
cctgcccatc ggtgattctg caagtaattt gctttttaag tggagaaata tggctgacag 1080
gtatgaaagg ttacagaaaa tatgctccat agccctggtt ggcaaataca ccaagctcag 1140
agactgctac gcctctgtgt tcaaagccct ggaacactca gccctggcca tcaaccacaa 1200
gttgaatctg atggtgattg atatgcccga gcacaaccct ggcaatttgg gaggaacaat 1260
gagactggga ataagaagaa ctgttttcaa aactgaaaat tcaatattaa ggaaacttta 1320
tggtgatgtt ccttttatag aagaaagaca cagacatcgg ttcgaggtaa accctaacct 1380
gatcaaacaa tttgagcaga atgacttaag ttttgtaggt caggatgttg atggagacag 1440

gatggaaatc attgaactgg caaatcatcc ttattttgtt ggtgtccagt tccatcctga 1500
gttttcttct aggccgatga agccttcccc tccgtatctg ggctgttac ttgcagcaac 1560
tgggaacctg aatgcctact tgcaacaggg ttgcaaactg tcttccagtg atagatacag 1620
tgatgccagt gatgacagct tttcagagcc aaggatagct gagttggaaa taagctgaaa 1680
tgaatacatg actgggaata atggggactg cctgtgaggc ctctgaaata attgaaggca 1740
agatgaagga actatctgaa gaaatcacta cactcttaga gaatccctct gttctccagc 1800
aaacatggga tgtaaagcct cacagggaat ctgataatac atacttctgt caaccagaac 1860
cagaggggta gtttcttttt ccctccagag gcaacctttg atacttaaaa tatctgtagc 1920
tgattaaatt tcgccca 1937

<210> 26
<211> 970
<212> DNA
<213> Homo sapiens

<220>
<221> misc_feature
<223> Incyte ID No: 3778612CB1

<400> 26
taataataat aacgtaatca tacctctagt catagcatac catttatcgg gctcggcgca 60
ggcccgcggg gagcgcagcc cggcggagag actgatggag aggcagaaac ggaaggcgga 120
catcgagaaa gggctgcagt tcattcagtc gacactaccc ctaaagcaag aagaatatga 180
ggcctttctg ctcaagctgg tgcagaatct gtttgctgag ggcaatgatc tgttccggga 240
gaaggactat aagcaggctc tggtgcagta catggaaggg ctgaacgtgg ccgactacgc 300
tgcctctgac caggtggccc tgccccggga gctgctgtgc aagctgcatg tcaatagggc 360
cgcctgctac ttcaccatgg gcctgtatga gaaggcgctg gaggacagcg agaaggcgct 420
gggccctgac agtgagagta tccgggcgtt gttccgcaaa gcacgcgctc tcaatgaact 480
gggacgccac aaggaggcct acgagtgcag cagccggtgt tccctcgccc tgccccacga 540
tgaaagcgtg actcagcttg gtcagggacc cttgggatct ggggcttcct ggcctggcca 600
gagctggagc cccacaggg taaggaagag agagtgggag gcagagtgtg atggggagga 660
gggacaggaa gacccttttta atgatgaggg taactatttc agttgtgagc cttctagggc 720
cccaggctgg gaggctcaga ggactgaatc tgggacctgt gttccccccg gcaggcaggg 780
acaagatggc atggcaagca tgggggcggg gtgggtgggg agggatgctg catttctcag 840
ctgggcagta atcaatttaa tggtccttta aaatgtctgt gtattaaaaa tttaagaata 900
ccacacttta atattaaata ttcataaggt ctagtatctt gataataatg tagatgtttt 960
aataacaatt 970

<210> 27
<211> 1810
<212> DNA
<213> Homo sapiens

<220>
<221> misc_feature
<223> Incyte ID No: 4574912CB1

<220>
<221> unsure
<222> 193, 196-198
<223> a, t, c, g, or other

<400> 27
gctgcatgga gggcgccgtt ctagaagctg gggtgcgcg gtgcttttgc aggtttgggt 60
gtgaattgag caagtatgaa aaccccggct attcatcccc cagaagtgat tactttaaaa 120
attatatgat cattataact cagaatcgaa tgtcctttct tgcaaatatg ttccacacga 180
tggactgtgt tgntgnnncc aggtatagct gtggaccaac tgtatatgat catgcgcacc 240
ttggccatgc ttgctcatat gttagatttg atatcattcg aaggatccta accaaggttt 300
ttggatgcag catagtcatg gtgatgggta ttacagatgt agatgataaa atcatcaaaa 360
gagccaatga gatgaatatt tccccccgctt ccctcgccag tctttatgag gaagacttca 420
agcaggacat ggcagccctg aaggttctcc cacccacggt gtacctgagg gtaaccgaaa 480
atattcctca gataatttct ttcattgaag gaatcattgc tcgtgggaac gcttattcaa 540
cggcaaaagg caatgtctac ttcgatctga agtctagagg agacaagtat ggcaaattgg 600
tcggcgtggt ccctggtcca gtcggagagc cagcggactc tgacaagcgt catgccagtg 660
acttcgccct gtggaaggcg gccaaacccc aggaggtgtt ctgggcctct ccctggggac 720

ccgggaggcc gggctggcac atcgagtgct ctgccatcgc tagtatggta tttggaagtc 780
aactggatat ccattcaggt gggatagatt tagcttttcc acatcatgag aacgaaattg 840
cacagtgcga agtctttcat cagtgcgagc agtggggaaa ttattttctg cattctgggc 900
atttgcacgc caaaggcaaa gaagaaaaaa tgtccaaatc attaaagaac tacattacta 960
ttaaggactt tctgaagacc ttttcccccg atgtcttccg gttcttctgc ctgcggagca 1020
gctaccgctc agccatcgac tacagtgaca gcgccatgct ccaagctcag cagctgctcc 1080
tggggctggg ctctttcctg gaggacgcac gtgcctacat gaaggggcag ctggcctgcg 1140
gctccgtcag ggaagcgatg ctgtgggaga ggctctccag caccaagagg gccgtgaagg 1200
cggccttggc agatgacttt gacacaccca gggtggttga tgccatcctg ggccttgcac 1260
accacgggaa tggacagctc agggcgtccc tgaaggaacc tgaagggccg agaagtcctg 1320
ctgtgtttgg tgccatcatc tcttactttg aacagttttt tgaaactgtt ggaatttctc 1380
tggcaaatca acagtacgtt tcaggagacg gcagcgaggc taccttgcat ggtgtggtgg 1440
acgagctggt gcggttccgg cagaaggtcc ggcagtttgc gctggccatg cccgaggcca 1500
cgggggacgc ccggcggcag cagctcctag aaaggcagcc cctgctggaa gcatgcgaca 1560
ccctgcgccg gggcctgact gcccacggca tcaacatcaa ggacagaagc agtacaacat 1620
ccacgtggga actgctggat caaaggacaa aagaccaaaa atcagcgggc tgaggatgga 1680
gcacagccat gaacctgctc acgacaagac gcacccatgc ttctcagggt caaggcttta 1740
tgttaaagct tcctgtcggg gctgctaggt cagcattaaa gtaaggcaac caacagtgaa 1800
aaaaaaaaaa 1810

<210> 28
<211> 2162
<212> DNA
<213> Homo sapiens

<220>
<221> misc_feature
<223> Incyte ID No: 5630806CB1

<400> 28
gaccgtgcgc gcggcacgag gggacggggt ccgactcaga aatggcggcc tccatgttct 60
acggcaggct agtggccgtg gccacccttc ggaaccaccg gcctcggacg gcccagcggg 120
ctgctgctca ggttctggga agttctggat tgtttaataa ccatggactc caagtacagc 180
agcaacagca aaggaatctc tcactacatg aatacatgag tatggaatta ttgcaagaag 240
ctggtgtctc cgttcccaaa ggatatgtgg caaagtcacc agatgaagct tatgcaattg 300
ccaaaaaatt aggttcaaaa gatgtcgtga taaaggcaca ggttttagct ggtggtagag 360
gaaaaggaac atttgaaagt ggcctcaaag gaggagtgaa gatagttttc tctccagaag 420
aagcaaaagc tgtttcttca caaatgattg ggaaaaaatt gtttaccaag caaacgggag 480
aaaagggcag aatatgcaat caagtattgg tctgtgagcg aaaatatccc aggagagaat 540
actactttgc aataacaatg gaaaggtcat ttcaaggtcc tgtattaata ggaagttcac 600
atggtggtgt caacattgaa gatgttgctg ctgagactcc tgaagcaata attaaagaac 660
ctattgatat tgaagaaggc atcaaaaagg aacaagctct ccagcttgca cagaagatgg 720
gatttccacc taatattgtg gaatcagcag cagaaaacat ggtcaagctt tacagccttt 780
ttctgaaata cgatgcaacc atgatagaaa taaatccaat ggtggaagat tcagatggag 840
ctgtattgtg tatggatgca aagatcaatt ttgactctaa ttcagcctat cgccaaaaga 900
aaatctttga tctacaggac tggacccagg aagatgaaag ggacaaagat gctgctaagg 960
caaatctcaa ctacattggc ctcgatggaa atataggctg cctagtaaat ggtgctggtt 1020
tggctatggc cacaatggat ataataaaac ttcatggagg gactccagcc aacttccttg 1080
atgttggtgg tggtgctaca gtccatcaag taacagaagc atttaagctt atcacttcag 1140
ataaaaaggt actggctatt ctggtcaaca tttttggagg aatcatgcgc tgtgatgtta 1200
ttgcacaggg tatagtcatg gcagtaaaag acttggaaat taaaatacct gttgtggtac 1260
ggttacaagg tacacgagtc gatgatgcta aggcactgat agcggacagt ggacttaaaa 1320
tacttgcttg tgatgacttg gatgaagctg ctagaatggt tgtaaagctc tctgaaatag 1380
tgaccttagc gaagcaagca catgtggatg tgaaatttca gttgccaata tgatctgaaa 1440
acccagtgga tggctgaagg tgttaaatgt gctataatca ttaagaatac tgtgttctgt 1500
gttattgttc tttttctttt tagtgtgtgg agattgtaat tgccatctag gcacacaaac 1560
atttaaaagg atttggactg catttaattg taccattcag aatggactgt ttgtacgaag 1620
catgtataat gcagttatct tctttctttc gtcgcagcca gtctttttttg cttctcctac 1680
aaaacgtaac ttgcaatttg ccagtttatt attgttggat acaaagttct tcattgataa 1740
gagtcctata aataagataa atacgaagat aaagctttat tctttagtgt taaaatacag 1800
tatatctaat aactagcctc attagtagag cagtatatta aaacaatgtt ttatgtaaaa 1860
agtgtttatc ttcagcacca aatacatgat aaatgtatca atcactattt ataaacagag 1920
ctttcaaaca ctcctcagaa tattcttcta agtattttga tgaagtaact ttgtaattat 1980
ttgaacattg ttttaatcat taggaaacac tgattaactg caagtcttca tgattctgtc 2040
atattaagaa acacctgtag gtttgcttca aataaaggca tatataccaa ggacttacag 2100

acaaaattaa gaatgtcaat ttaagttaat aaaaatctcc caatatgaaa aaaaaaaaaa 2160
aa 2162

<210> 29
<211> 1477
<212> DNA
<213> Homo sapiens

<220>
<221> misc_feature
<223> Incyte ID No: 5854855CB1

<400> 29
gcaatccgta ccctcagtgg gttccctttc agtgggttcc tttgtcccca ggcccattat 60
tccgtcctcc cctcttccct gatgtatttt ggcgcggtct cctggctctg cgggcccagg 120
gctccggatg aggtctcccg ccgtcccgac ccccgcaagg gccagcttgg tgtcgccttc 180
gttcttctgc caccccattc ggaaggtgct cgggtcttcg ggcactgggg tcccatcggt 240
ccctcctcac ctgggctcac cctcgggggt ctggccgtga gcgagcaccg gctcagcaac 300
aagctgctgg cttggagcgg cgtcctcgag tggcaggaga agcgcagacc ctactctgac 360
tccactgcaa agctgaagcg gaccctgccc tgccaagcct acgtgaacca aggcgagaac 420
ctggagaccg accagtggcc gcagaagctg atcatgcagc tgatcccgca gcagctgctg 480
accaccctgg gccccctgtt ccggaactcc cagttggcac agttccactt caccaacaga 540
gactgcgact cgctcaaggg gctctgccgc atcatgggca acggcttcgc gggctgcatg 600
ctgttccccc acatctcccc ctgtgaggtg cgcgtgctca tgctcctgta ctcgtccaag 660
aagaagatct tcatgggcct catcccctac gaccagagcg gcttcgtcag tgccatccgg 720
caggtcatca ccacccgcaa gcaggcagtg ggacctggtg gtgtcaactc aggcccagtc 780
cagatcgtca acaacaagtt tctggcatgg agtggtgtca tggagtggca ggagcccagg 840
cctgagccca acagtcggtc caagaggtgg ctgccatccc acgtctacgt gaaccagggg 900
gagatcctga ggaccgagca gtggccaagg aagctgtaca tgcagctcat cccgcagcag 960
ctgctgacca ccctagtgcc gctgttccgg aactcgcgcc tggtccagtt ccacttcacc 1020
aaggacctgg agacactgaa gagcctgtgc cggatcatgg acaatggctt cgccggctgc 1080
gtgcactttt cctacaaagc atcgtgtgag atccgcgtgc ttatgctcct gtactcttca 1140
gagaagaaaa tcttcattgg cctcatcccc catgaccagg gcaactttgt caacggcatc 1200
cggcgtgtca ttgccaacca gcagcaggtc ctgcagcgga acctggagca ggagcaacag 1260
caacgaggga tggggggGta gtggttaccc cgggctgggc ccctccagga gtcacagatg 1320
aggcccccgc agagactggt gacacgcttc tgagcagggg cccctgggga cttcaactgc 1380
ccagcaacat ggaggatggt gtcctgaggc ctccaaggac ggtccccacc cctctacgtt 1440
tccccaataa agccttttaa aaacctgaaa aaaaaaa 1477

<210> 30
<211> 1660
<212> DNA
<213> Homo sapiens

<220>
<221> misc_feature
<223> Incyte ID No: 5993973CB1

<400> 30
gggccttccg cagctgcaga gcctcaacct cagcggcaac cggctgcgcg agccgccagc 60
cgacctggcg cgctgcgccc cgcgcctgca gagcctcaac ctcaccggca attgcctaga 120
ctcctttccc gccgagctct ttcgccccgg cgcgctgccc ctgctcagtg aactggcggc 180
tgctgacaac tgcctccgag aactcagccc cgacatcgcc cacctggcct cgctcaagac 240
gttggacctc tcgaacaacc agctgagcga gatccctgca gagcttgcgg actgccccaa 300
gctcaaggag atcaatttcc gtgggaacaa gctgagggac aagcgcctgg agaagatggt 360
cagcggctgc cagaccagat ccatcctgga gtacctgcgc gtcggaggcc gtggtggcgg 420
gaagggcaag ggccgtgccg agggctcgga gaaggaagag agccggagga agaggaggga 480
gaggaagcag aggcgggaag gtggtgatgg ggaggagcag gacgtgggag atgccggccg 540
gctgctgctc agggtcctgc acgtctctga aaaccccgta cctctgacag tcagagtgag 600
ccccgaggtc cgggatgtgc ggccctacat tgtgggggcc gtggtgcgag gcatggacct 660
gcagccaggg aatgcactca agcgcttcct cacctcgcag accaagctcc acgaagatct 720
ctgtgagaag aggacggctg ccacccttgc cacccacgag ctccgtgccg tcaaagggcc 780
cctgctgtac tgcgcccggc ccccacagga cctcaagatt gtccccttgg ggcggaaaga 840
agccaaggcc aaggagctgg tgcggcagct gcagctggag gccgaggagc agaggaagca 900
gaagaagcgg cagagtgtgt cgggcctgca cagatacctt cacttgctgg atggaaatga 960

```
aaattacccg tgtcttgtgg atgcagacgg tgatgtgatt tccttcccac caataaccaa 1020
cagtgagaag acaaaggtta agaaaacgac ttctgatttg tttttggaag taacaagtgc 1080
caccagtctg cagatttgca aggatgtcat ggatgccctc attctgaaaa tggcagaaat 1140
gaaaaagtac actttagaaa ataaagagga aggatcactc tcagatactg aagccgatgc 1200
agtctctgga caacttccag atcccacaac gaatcccagt gctggaaagg acgggccctc 1260
ccttctggtg gtggagcagg tccgggtggt ggatctggaa gggagcctga aggtggtgta 1320
cccgtccaag gccgacctgg ccactgcccc tccccacgtg actgtcgtgc gctgacgcca 1380
gggccgcctg tccgcgtttg tttggccggt tttgcggagg tttctatgcg gcaatgctga 1440
attatccgtt agattttcac cccagttttt ttgttggttt ttttttttg agatggagtc 1500
tcgctctgtc gccaggctgg agtgcagtgg cgtgatctcg agtcactgca gcctgtgtct 1560
cctgggttca agcgattctc ctgcctcagc ctcccaagta gctgggacta caggtgtgtg 1620
ccactaagct cagctaattt ttgtattttt agtagagacg                       1660
```

## Description

## TECHNICAL FIELD

This invention relates to nucleic acid and amino acid sequences of synthetases and to the use of these sequences in the diagnosis, treatment, and prevention of immune, neuronal, and reproductive disorders, and cell proliferative disorders including cancer.

## BACKGROUND OF THE INVENTION

A large number of cellular biosynthetic intermediary metabolism processes involve intermolecular transfer of carbon atom-containing substrates (carbon substrates). Examples of such reactions include the tricarboxylic acid cycle, synthesis of fatty acids and long-chain phospholipids, synthesis of alcohols and aldehydes, synthesis of intermediary metabolites, and reactions involved in the amino acid degradation pathways. Many of these reactions are catalyzed by synthetases (also called ligases), which catalyze the formation of a bond between two substrate molecules. Some of these reactions require input of energy, usually in the form of conversion of ATP to either ADP or AMP and pyrophosphate. Synthetases are named for the products of the reaction they catalyze and are involved in such processes as metabolism and the synthesis of macromolecules.

Ligases forming carbon-oxygen bonds

Proteins make up more than half of the total dry mass of a cell. The synthesis of proteins is central to cell maintenance, growth, and development. Synthesis occurs on ribosomes and depends on the cooperative interaction of several classes of RNA molecules. The process begins with transcription of the genetic code contained within the DNA to form messenger RNA (mRNA). The mRNA moves in steps through a ribosome and the nucleotide sequence of the mRNA is translated into a corresponding sequence of amino acids to construct a distinct protein chain.

The aminoacyl-transfer RNA (tRNA) synthetases are important RNA-associated enzymes with roles in translation. Protein biosynthesis depends on each amino acid forming a linkage with the appropriate tRNA. The aminoacyl-tRNA synthetases are responsible for the activation and correct attachment of an amino acid with its cognate tRNA. The 20 aminoacyl-tRNA synthetase enzymes can be divided into two structural classes, and each class is characterized by a distinctive topology of the catalytic domain. Class I enzymes contain a catalytic domain based on the nucleotide-binding Rossman 'fold'. Class II enzymes contain a central catalytic domain, which consists of a seven-stranded antiparallel β-sheet motif, as well as N- and C- terminal regulatory domains. Class II enzymes are separated into two groups based on the heterodimeric or homodimeric structure of the enzyme; the latter group is further subdivided by the structure of the N- and C-terminal regulatory domains (Hartlein, M. and Cusack, S. (1995) J. Mol. Evol. 40:519-530). Autoantibodies against aminoacyl-tRNAs are generated by patients with dermatomyositis and polymyositis, and correlate strongly with complicating interstitial lung disease (ILD). These antibodies appear to be generated in response to viral infection, and coxsackie virus has been used to induce experimental viral myositis in animals (Friedman, A.W. et al. (1996) Semin. Arthritis Rheum. 26:459-467). A synthetase homolog has been shown to be expressed in chronic myeloid leukemia (CML). A phenylalanine-tRNA synthetase homolog has been found to be tumor-selective and expressed in a cell cycle stage- and differentiation-dependent fashion in an acute-phase human CML cell line (Sen, S. et al. (1997) Proc. Natl. Acad. Sci.USA 94:6164-6169).

Ligases forming carbon-sulfur bonds (Acid-thiol ligases)

In many cases, a carbon substrate is derived from a small molecule containing at least two carbon atoms. The carbon substrate is often covalently bound to a larger molecule which acts as a carbon substrate carrier molecule within the cell. In the biosynthetic mechanisms described above, the carrier molecule is coenzyme A. Coenzyme A (CoA) is structurally related to derivatives of the nucleotide ADP and consists of 4'-phosphopantetheine linked via a phosphodiester bond to the alpha phosphate group of adenosine 3',5'-bisphosphate. The terminal thiol group of 4'-phosphopantetheine acts as the site for carbon substrate bond formation. The predominant carbon substrates which utilize CoA as a carrier molecule during biosynthesis and intermediary metabolism in the cell are acetyl, succinyl, and propionyl moieties, collectively referred to as acyl groups. Other carbon substrates include enoyl lipid, which acts as a fatty acid oxidation intermediate, and carnitine, which acts as an acetyl-CoA flux regulator/ mitochondrial acyl group transfer protein. Acyl-CoA and acetyl-CoA are synthesized in the cell by acyl-CoA synthetase and acetyl-CoA synthetase, respectively.

Activation of fatty acids is mediated by at least three forms of acyl-CoA synthetase activity: i) acetyl-CoA synthetase, which activates acetate and several other low molecular weight carboxylic acids and is found in muscle mitochondria and the cytosol of other tissues; ii) medium-chain acyl-CoA synthetase, which activates fatty acids containing between four and eleven carbon atoms (predominantly from dietary sources), and is present only in liver mitochondria; and iii) acyl CoA synthetase, which is specific for long chain fatty acids with between six and twenty carbon atoms, and is found in microsomes and the mitochondria. Proteins associated with acyl-CoA synthetase activity have been identified from many sources including bacteria, yeast, plants, mouse, and man. The activity of acyl-CoA synthetase may be modulated by phosphorylation of the enzyme by cAMP-dependent protein kinase. The COL4A5 (collagen, type IV, alpha-5) chromosomal region found deleted in 2 patients with Alport syndrome, elliptocytosis, and mental retardation (Piccini. M. et al, (1998) Genomics 47: 350-358) is contiguous with the region containing long-chain acyl-CoA synthetase 4 (FACL4). Therefore, it has been suggested (Piccini supra) that the absence of FACL4 may be involved in the development of mental retardation and other phenotypes associated with Alport syndrome in these patients.

Ligases forming carbon-nitrogen bonds

A key representative of the amide synthases is the enzyme glutamine synthetase (glutamate-ammonia ligase) that catalyzes the amination of glutamic acid to glutamine by ammonia using the energy of ATP hydrolysis. Glutamine is the primary source for the amino group in various amide transfer reactions involved in de novo pyrimidine nucleotide synthesis and in purine and pyrimidine ribonucleotide interconversions, as well as the conversion of aspartate to asparagine. Overexpression of glutamine synthetase has been observed in primary liver cancer (Christa. L. et al. (1994) Gastroent. 106:1312-1320).

Cyclo-ligases and other carbon-nitrogen ligases comprise various enzymes and enzyme complexes that participate in the de novo pathways to purine and pyrimidine biosynthesis. Because these pathways are critical to the synthesis of nucleotides for replication of both RNA and DNA, many of these enzymes have been the targets of clinical agents for the treatment of cell proliferative disorders such as cancer and infectious diseases.

Purine biosynthesis occurs de novo from the amino acids glycine and glutamine, and other small molecules. Three of the key reactions in this process are catalyzed by a trifunctional enzyme composed of glycinamide-ribonucleotide synthetase (GARS), aminoimidazole ribonucleotide synthetase (AIRS), and glycinamide ribonucleotide transformylase (GART). Together these three enzymes combine ribosylamine phosphate with glycine to yield phosphoribosyl aminoimidazole, a precursor to both adenylate and guanylate nucleotides. This trifunctional protein has been implicated in the pathology of Downs syndrome (Aimi, J. et al. ( 1990) Nucleic Acid Res. 18:6665-6672). Adenylosuccinate synthetase catalyzes a later step in purine biosynthesis that converts inosinic acid to adenylosuccinate, a key step on the path to ATP synthesis. This enzyme is also similar to another carbon-nitrogen ligase, argininosuccinate synthetase, that catalyzes a similar reaction in the urea cycle (Powell, S.M. et al. (1992) FEBS Lett. 303:4-10).

Like the de novo biosynthesis of purines, de novo synthesis of the pyrimidine nucleotides uridylate and cytidylate also arises from a common precursor, in this instance the nucleotide orotidylate derived from orotate and phosphoribosyl pyrophosphate (PPRP). Again a trifunctional enzyme comprising three carbon-nitrogen ligases plays a key role in the process. In this case the enzymes aspartate transcarbamylase (ATCase), carbamyl phosphate synthetase II, and dihydroorotase (DHOase) are encoded by a single gene called CAD. Together these three enzymes combine the initial reactants in pyrimidine biosynthesis, glutamine, $CO_2$, and ATP to form dihydroorotate, the precursor to orotate and orotidylate (Iwahana, H. et al. (1996) Biochem. Biophys. Res. Commun. 219:249-255). Further steps then lead to the synthesis of uridine nucleotides from orotidylate. Cytidine nucleotides are derived from uridine-5'-triphosphate (UTP) by the amidation of UTP using glutamine as the amino donor and the enzyme CTP synthetase. Regulatory mutations in the human CTP synthetase are believed to confer multi-drug resistance to agents widely used in cancer therapy (Yamauchi, M. et al. (1990) EMBO J. 9:2095-2099).

Ligases forming carbon-carbon bonds

Ligases in this group are represented by the carboxylases acetyl-CoA carboxylase and pyruvate carboxylase. Acetyl-CoA carboxylase is a complex which includes a biotin carboxyl carrier protein, biotin carboxylase, and a carboxyl transferase made up of two alpha and two beta subunits. This complex catalyzes the carboxylation of Acetyl-CoA from $CO_2$ and $H_2O$ using the energy of ATP hydrolysis (PRINTS document PR01069). Acetyl-CoA carboxylase is the rate-limiting step in the biogenesis of long-chain fatty acids. Two isoforms of Acetyl-CoA carboxylase, types I and types II, are expressed in humans in a tissue-specific manner (Ha, J. et al. (1994) Eur. J. Biochem. 219:297-306). Pyruvate carboxylase is a nuclear-encoded mitochondrial enzyme that catalyzes the conversion of pyruvate to oxaloacetate, a key intermediate in the citric acid cycle.

Ligases forming phosphoric ester bonds

Ligases in this group are represented by the DNA ligases involved in both DNA replication and repair. DNA ligases seal phosphodiester bonds between two adjacent nucleotides in a DNA chain using the energy from ATP hydrolysis to first activate the free 5'-phosphate of one nucleotide and then react it with the 3'-OH group of the adjacent nucleotide. This resealing reaction is used in both DNA replication to join small DNA fragments called "Okazaki" fragments that are transiently formed in the process of replicating new DNA, and in DNA repair. DNA repair is the process by which accidental base changes, such as those produced by oxidative damage, hydrolytic attack, or uncontrolled methylation of DNA, are corrected before replication or transcription of the DNA can occur. Bloom's syndrome is an inherited human disease in which individuals are partially deficient in DNA ligation and consequently have an increased incidence of cancer (Alberts. B. et al. (1994) The Molecular Biology of the Cell, Garland Publishing Inc., New York, NY, p. 247).

The discovery of new synthetases and the polynucleotides encoding them satisfies a need in the art by providing new compositions which are useful in the diagnosis, prevention, and treatment of immune, neuronal, and reproductive disorders, and cell proliferative disorders including cancer.

## SUMMARY OF THE INVENTION

The invention features purified polypeptides, human synthetases, referred to collectively as "SYNT' and individually as "SYNT-1," "SYNT-2," "SYNT-3," "SYNT-4,""SYNT-5," "SYNT-6," "SYNT-7," "SYNT-8," "SYNT-9,""SYNT-10," "SYNT-11," "SYNT- 12," "SYNT-13," "SYNT-14," and "SYNT-15." In one aspect, the invention provides an isolated polypeptide comprising an amino acid sequence selected from the group consisting of a) an amino acid sequence selected from the group consisting of SEQ ID NO: 1-15. b) a naturally occurring amino acid sequence having at least 90% sequence identity to an amino acid sequence selected from the group consisting of SEQ ID NO:1-15, c) a biologically active fragment of an amino acid sequence selected from the group consisting of SEQ-ID NO:1-15, and d) an immunogenic fragment of an amino acid sequence selected from the group consisting of SEQ ID NO:1-15. In one alternative, the invention provides an isolated polypeptide comprising the amino acid sequence of SEQ ID NO:1-15.

The invention further provides an isolated polynucleotide encoding a polypeptide comprising an amino acid sequence selected from the group consisting of a) an amino acid sequence selected from the group consisting of SEQ ID NO:1-15, b) a naturally occurring amino acid sequence having at least 90% sequence identity to an amino acid sequence selected from the group consisting of SEQ ID NO:1-15, c) a biologically active fragment of an amino acid sequence selected from the group consisting of SEQ ID NO:1-15, and d) an immunogenic fragment of an amino acid sequence selected from the group consisting of SEQ ID NO:1-15. In one alternative, the polynucleotide encodes a polypeptide selected from the group consisting of SEQ ID NO:1-15. In another alternative, the polynucleotide is selected from the group consisting of SEQ ID NO:16-30.

Additionally, the invention provides a recombinant polynucleotide comprising a promoter sequence operably linked to a polynucleotide encoding a polypeptide comprising an amino acid sequence selected from the group consisting of a) an amino acid sequence selected from the group consisting of SEQ ID NO:1-15, b) a naturally occurring amino acid sequence having at least 90% sequence identity to an amino acid sequence selected from the group consisting of SEQ ID NO: 1-15, c) a biologically active fragment of an amino acid sequence selected from the group consisting of SEQ ID NO:1-15, and d) an immunogenic fragment of an amino acid sequence selected from the group consisting of SEQ ID NO:1-15. In one alternative, the invention provides a cell transformed with the recombinant polynucleotide. In another alternative, the invention provides a transgenic organism comprising the recombinant polynucleotide.

The invention also provides a method for producing a polypeptide comprising an amino acid sequence selected from the group consisting of a) an amino acid sequence selected from the group consisting of SEQ ID NO:1-15, b) a naturally occurring amino acid sequence having at least 90% sequence identity to an amino acid sequence selected from the group consisting of SEQ ID NO:1-15, c) a biologically active fragment of an amino acid sequence selected from the group consisting of SEQ ID NO:1-15, and d) an immunogenic fragment of an amino acid sequence selected from the group consisting of SEQ ID NO:1-15. The method comprises a) culturing a cell under conditions suitable for expression of the polypeptide, wherein said cell is transformed with a recombinant polynucleotide comprising a promoter sequence operably linked to a polynucleotide encoding the polypeptide, and b) recovering the polypeptide so expressed.

Additionally, the invention provides an isolated antibody which specifically binds to a polypeptide comprising an amino acid sequence selected from the group consisting of a) an amino acid sequence selected from the group consisting of SEQ ID NO:1-15, b) a naturally occurring amino acid sequence having at least 90% sequence identity to an amino acid sequence selected from the group consisting of SEQ ID NO:1-15, c) a biologically active fragment of an amino acid sequence selected from the group consisting of SEQ ID NO:1-15, and d) an immunogenic fragment of an amino acid sequence selected from the group consisting of SEQ ID NO:1-15.

The invention further provides an isolated polynucleotide comprising a polynucleotide sequence selected from the group consisting of a) a polynucleotide sequence selected from the group consisting of SEQ ID NO:16-30, b) a naturally occurring polynucleotide sequence having at least 90% sequence identity to a polynucleotide sequence selected from the group consisting of SEQ ID NO:16-30, c) a polynucleotide sequence complementary to a), d) a polynucleotide sequence complementary to b), and e) an RNA equivalent of a)-d). In one alternative, the polynucleotide comprises at least 60 contiguous nucleotides.

Additionally, the invention provides a method for detecting a target polynucleotide in a sample, said target polynucleotide having a sequence of a polynucleotide comprising a polynucleotide sequence selected from the group consisting of a) a polynucleotide sequence selected from the group consisting of SEQ ID NO: 16-30, b) a naturally occurring polynucleotide sequence having at least 90% sequence identity to a polynucleotide sequence selected from the group consisting of SEQ ID NO:16-30, c) a polynucleotide sequence complementary to a), d) a polynucleotide sequence complementary to b), and e) an RNA equivalent of a)-d). The method comprises a) hybridizing the sample with a probe comprising at least 20 contiguous nucleotides comprising a sequence complementary to said target polynucleotide in the sample, and which probe specifically hybridizes to said target polynucleotide, under conditions whereby a hybridization complex is formed between said probe and said target polynucleotide or fragments thereof, and b) detecting the presence or absence of said hybridization complex, and optionally, if present, the amount thereof. In one alternative, the probe comprises at least 60 contiguous nucleotides.

The invention further provides a method for detecting a target polynucleotide in a sample, said target polynucleotide having a sequence of a polynucleotide comprising a polynucleotide sequence selected from the group consisting of a) a polynucleotide sequence selected from the group consisting of SEQ ID NO: 16-30, b) a naturally occurring polynucleotide sequence having at least 90% sequence identity to a polynucleotide sequence selected from the group consisting of SEQ ID NO: 16-30, c) a polynucleotide sequence complementary to a), d) a polynucleotide sequence complementary to b), and e) an RNA equivalent of a)-d). The method comprises a) amplifying said target polynucleotide or fragment thereof using polymerase chain reaction amplification, and b) detecting the presence or absence of said amplified target polynucleotide or fragment thereof, and, optionally, if present, the amount thereof.

The invention further provides a pharmaceutical composition comprising an effective amount of a polypeptide comprising an amino acid sequence selected from the group consisting of a) an amino acid sequence selected from the group consisting of SEQ ID NO:1-15, b) a naturally occurring amino acid sequence having at least 90% sequence identity to an amino acid sequence selected from the group consisting of SEQ ID NO:1-15, c) a biologically active fragment of an amino acid sequence selected from the group consisting of SEQ ID NO:1-15, and d) an immunogenic fragment of an amino acid sequence selected from the group consisting of SEQ ID NO:1-15, and a pharmaceutically acceptable excipient. In one embodiment, the pharmaceutical composition comprises an amino acid sequence selected from the group consisting of SEQ ID NO:1-15. The invention additionally provides a method of treating a disease or condition associated with decreased expression of functional SYNT, comprising administering to a patient in need of such treatment the pharmaceutical composition.

The invention also provides a method for screening a compound for effectiveness as an agonist of a polypeptide comprising an amino acid sequence selected from the group consisting of a) an amino acid sequence selected from the group consisting of SEQ ID NO:1-15, b) a naturally occurring amino acid sequence having at least 90% sequence identity to an amino acid sequence selected from the group consisting of SEQ ID NO:1-15, c) a biologically active fragment of an amino acid sequence selected from the group consisting of SEQ ID NO:1-15, and d) an immunogenic fragment of an amino acid sequence selected from the group consisting of SEQ ID NO:1-15. The method comprises a) exposing a sample comprising the polypeptide to a compound, and b) detecting agonist activity in the sample. In one alternative, the invention provides a pharmaceutical composition comprising an agonist compound identified by the method and a pharmaceutically acceptable excipient. In another alternative, the invention provides a method of treating a disease or condition associated with decreased expression of functional SYNT, comprising administering to a patient in need of such treatment the pharmaceutical composition.

Additionally, the invention provides a method for screening a compound for effectiveness as an antagonist of a polypeptide comprising an amino acid sequence selected from the group consisting of a) an amino acid sequence selected from the group consisting of SEQ ID NO: 1-15, b) a naturally occurring amino acid sequence having at least 90% sequence identity to an amino acid sequence selected from the group consisting of SEQ ID NO:1-15, c) a biologically active fragment of an amino acid sequence selected from the group consisting of SEQ ID NO:1-15, and d) an immunogenic fragment of an amino acid sequence selected from the group consisting of SEQ ID NO:1-15. The method comprises a) exposing a sample comprising the polypeptide to a compound, and b) detecting antagonist activity in the sample. In one alternative, the invention provides a pharmaceutical composition comprising an antagonist compound identified by the method and a pharmaceutically acceptable excipient. In another alternative, the invention provides a method of treating a disease or condition associated with overexpression of functional SYNT, comprising administering to a patient in need of such treatment the pharmaceutical composition.

The invention further provides a method of screening for a compound that specifically binds to a polypeptide comprising an amino acid sequence selected from the group consisting of a) an amino acid sequence selected from the group consisting of SEQ ID NO:1-15, b) a naturally occurring amino acid sequence having at least 90% sequence identity to an amino acid sequence selected from the group consisting of SEQ ID NO:1-15, c) a biologically active fragment of an amino acid sequence selected from the group consisting of SEQ ID NO:1-15, and d) an immunogenic fragment of an amino acid sequence selected from the group consisting of SEQ ID NO:1-15. The method comprises a) combining the polypeptide with at least one test compound under suitable conditions, and b) detecting binding of the polypeptide to the test compound, thereby identifying a compound that specifically binds to the polypeptide.

The invention further provides a method of screening for a compound that modulates the activity of a polypeptide comprising an amino acid sequence selected from the group consisting of a) an amino acid sequence selected from the group consisting of SEQ ID NO:1-15, b) a naturally occurring amino acid sequence having at least 90% sequence identity to an amino acid sequence selected from the group consisting of SEQ ID NO:1-15, c) a biologically active fragment of an amino acid sequence selected from the group consisting of SEQ ID NO:1-15, and d) an immunogenic fragment of an amino acid sequence selected from the group consisting of SEQ ID NO:1-15. The method comprises a) combining the polypeptide with at least one test compound under conditions permissive for the activity of the polypeptide, b) assessing the activity of the polypeptide in the presence of the test compound, and c) comparing the activity of the polypeptide in the presence of the test compound with the activity of the polypeptide in the absence of the test compound, wherein a change in the activity of the polypeptide in the presence of the test compound is indicative of a compound that modulates the activity of the polypeptide.

The invention further provides a method for screening a compound for effectiveness in altering expression of a target polynucleotide, wherein said target polynucleotide comprises a sequence selected from the group consisting of SEQ ID NO: 16-30, the method comprising a) exposing a sample comprising the target polynucleotide to a compound, and b) detecting altered expression of the target polynucleotide.

The invention further provides a method for assessing toxicity of a test compound, said method comprising a) treating a biological sample containing nucleic acids with the test compound; b) hybridizing the nucleic acids of the treated biological sample with a probe comprising at least 20 contiguous nucleotides of a polynucleotide comprising a polynucleotide sequence selected from the group consisting of i) a polynucleotide sequence selected from the group consisting of SEQ ID NO: 16-30. ii) a naturally occurring polynucleotide sequence having at least 90% sequence identity to a polynucleotide sequence selected from the group consisting of SEQ ID NO: 16-30, iii) a polynucleotide sequence complementary to i), iv) a polynucleotide sequence complementary to ii), and v) an RNA equivalent of i)-iv). Hybridization occurs under conditions whereby a specific hybridization complex is formed between said probe and a target polynucleotide in the biological sample, said target polynucleotide comprising a polynucleotide sequence selected from the group consisting of SEQ ID NO:) 16-30, ii) a naturally occurring polynucleotide sequence having at least 90% sequence identity to a polynucleotide sequence selected from the group consisting of SEQ ID NO:16-30, iii) a polynucleotide sequence complementary to i), iv) a polynucleotide sequence complementary to ii), and v) an RNA equivalent of i)-iv). Alternatively, the target polynucleotide comprises a fragment of the above polynucleotide sequence; c) quantifying the amount of hybridization complex; and d) comparing the amount of hybridization complex in the treated biological sample with the amount of hybridization complex in an untreated biological sample, wherein a difference in the amount of hybridization complex in the treated biological sample is indicative of toxicity of the test compound.

## BRIEF DESCRIPTION OF THE TABLES

Table 1 shows polypeptide and nucleotide sequence identification numbers (SEQ ID NOs), clone identification numbers (clone IDs), cDNA libraries, and cDNA fragments used to assemble full-length sequences encoding SYNT.

Table 2 shows features of each polypeptide sequence, including potential motifs, homologous sequences, and methods, algorithms, and searchable databases used for analysis of SYNT.

Table 3 shows selected fragments of each nucleic acid sequence; the tissue-specific expression patterns of each nucleic acid sequence as determined by northern analysis; diseases. disorders, or conditions associated with these tissues; and the vector into which each cDNA was cloned.

Table 4 describes the tissues used to construct the cDNA libraries from which cDNA clones encoding SYNT were isolated.

Table 5 shows the tools, programs, and algorithms used to analyze the polynucleotides and polypeptides of the invention, along with applicable descriptions, references, and threshold parameters.

## DESCRIPTION OF THE INVENTION

Before the present proteins, nucleotide sequences, and methods are described, it is understood that this invention is not limited to the particular machines. materials and methods described, as these may vary. It is also to be understood that the terminology used herein is for the purpose of describing particular embodiments only, and is not intended to limit the scope of the present invention which will be limited only by the appended claims.

It must be noted that as used herein and in the appended claims, the singular forms "a," "an," and "the" include plural reference unless the context clearly dictates otherwise. Thus, for example, a reference to "a host-cell" includes a plurality of such host cells, and a reference to "an antibody" is a reference to one or more antibodies and equivalents thereof known to those skilled in the art, and so forth.

Unless defined otherwise, all technical and scientific terms used herein have the same meanings as commonly understood by one of ordinary skill in the art to which this invention belongs. Although any machines, materials, and methods similar or equivalent to those described herein can be used to practice or test the present invention, the preferred machines, materials and methods are now described. All publications mentioned herein are cited for the purpose of describing and disclosing the cell lines, protocols, reagents and vectors which are reported in the publications and which might be used in connection with the invention. Nothing herein is to be construed as an admission that the invention is not entitled to antedate such disclosure by virtue of prior invention.

## DEFINITIONS

*'SYNT"' refers to the amino acid sequences of substantially purified SYNT obtained from any species, particularly a mammalian species, including bovine, ovine, porcine, murine, equine, and human, and from any source, whether natural, synthetic, semi-synthetic, or recombinant.

The term "agonist" refers to a molecule which intensifies or mimics the biological activity of SYNT. Agonists may include proteins, nucleic acids, carbohydrates, small molecules, or any other compound or composition which modulates the activity of SYNT either by directly interacting with SYNT or by acting on components of the biological pathway in which SYNT participates.

An "allelic variant" is an alternative form of the gene encoding SYNT. Allelic variants may result from at least one mutation in the nucleic acid sequence and may result in altered mRNAs or in polypeptides whose structure or function may or may not be altered. A gene may have none, one, or many allelic variants of its naturally occurring form. Common mutational changes which give rise to allelic variants are generally ascribed to natural deletions, additions, or substitutions of nucleotides. Each of these types of changes may occur alone, or in combination with the others, one or more times in a given sequence.

"Altered" nucleic acid sequences encoding SYNT include those sequences with deletions, insertions, or substitutions of different nucleotides, resulting in a polypeptide the same as SYNT or a polypeptide with at least one functional characteristic of SYNT. Included within this definition are polymorphisms which may or may not be readily detectable using a particular oligonucleotide probe of the polynucleotide encoding SYNT, and improper or unexpected hybridization to allelic variants, with a locus other than the normal chromosomal locus for the polynucleotide sequence encoding SYNT. The encoded protein may also be "altered," and may contain deletions, insertions, or substitutions of amino acid residues which produce a silent change and result in a functionally equivalent SYNT. Deliberate amino acid substitutions may be made on the basis of similarity in polarity, charge, solubility, hydrophobicity, hydrophilicity, and/or the amphipathic nature of the residues, as long as the biological or immunological activity of SYNT is retained. For example, negatively charged amino acids may include aspartic acid and glutamic acid, and positively charged amino acids may include lysine and arginine. Amino acids with uncharged polar side chains having similar hydrophilicity values may include: asparagine and glutamine; and serine and threonine. Amino acids with uncharged side chains having similar hydrophilicity values may include: leucine, isoleucine, and valine; glycine and alanine; and phenylalanine and tyrosine.

The terms "amino acid" and "amino acid sequence" refer to an oligopeptide, peptide, polypeptide, or protein sequence, or a fragment of any of these, and to naturally occurring or synthetic molecules. Where "amino acid sequence" is recited to refer to a sequence of a naturally occurring protein molecule, "amino acid sequence" and like terms are not meant to limit the amino acid sequence to the complete native amino acid sequence associated with the recited protein molecule.

"Amplification" relates to the production of additional copies of a nucleic acid sequence. Amplification is generally carried out using polymerase chain reaction (PCR) technologies well known in the art.

The term "antagonist" refers to a molecule which inhibits or attenuates the biological activity of SYNT. Antagonists may include proteins such as antibodies, nucleic acids, carbohydrates, small molecules, or any other compound or composition which modulates the activity of SYNT either by directly interacting with SYNT or by acting on components of the biological pathway in which SYNT participates.

The term "antibody" refers to intact immunoglobulin molecules as well as to fragments thereof, such as Fab, $F(ab')_2$, and Fv fragments, which are capable of binding an epitopic determinant. Antibodies that bind SYNT polypeptides can be prepared using intact polypeptides or using fragments containing small peptides of interest as the immunizing antigen. The polypeptide or oligopeptide used to immunize an animal (e.g., a mouse, a rat, or a rabbit) can be derived from the translation of RNA, or synthesized chemically, and can be conjugated to a carrier protein if desired. Commonly used carriers that are chemically coupled to peptides include bovine serum albumin, thyroglobulin, and keyhole limpet hemocyanin (KLH). The coupled peptide is then used to immunize the animal.

The term "antigenic determinant" refers to that region of a molecule (i.e., an epitope) that makes contact with a particular antibody. When a protein or a fragment of a protein is used to immunize a host animal, numerous regions of the protein may induce the production of antibodies which bind specifically to antigenic determinants (particular regions or three-dimensional structures on the protein). An antigenic determinant may compete with the intact antigen (i.e., the immunogen used to elicit the immune response) for binding to an antibody.

The term "antisense" refers to any composition capable of base-pairing with the "sense" (coding) strand of a specific nucleic acid sequence. Antisense compositions may include DNA; RNA; peptide nucleic acid (PNA); oligonucleotides having modified backbone linkages such as phosphorothioates, methylphosphonates, or benzylphosphonates; oligonucleotides having modified sugar groups such as 2'-methoxyethyl sugars or 2'-methoxyethoxy sugars; or oligonucleotides having modified bases such as 5-methyl cytosine, 2'-deoxyuracil, or 7-deaza-2'-deoxyguanosine. Antisense molecules may be produced by any method including chemical synthesis or transcription. Once introduced into a cell, the complementary antisense molecule base-pairs with a naturally occurring nucleic acid sequence produced by the cell to form duplexes which block either transcription or translation. The designation "negative" or "minus" can refer to the antisense strand, and the designation "positive" or "plus" can refer to the sense strand of a reference DNA molecule.

The term "biologically active" refers to a protein having structural, regulatory, or biochemical functions of a naturally occurring molecule. Likewise, "immunologically active" or "immunogenic" refers to the capability of the natural, recombinant, or synthetic SYNT, or of any oligopeptide thereof, to induce a specific immune response in appropriate animals or cells and to bind with specific antibodies.

"Complementary" describes the relationship between two single-stranded nucleic acid sequences that anneal by base-pairing. For example, 5'-AGT-3' pairs with its complement,

3'-TCA-5'.

A "composition comprising a given polynucleotide sequence" and a "composition comprising a given amino acid sequence" refer broadly to any composition containing the given polynucleotide or amino acid sequence. The composition may comprise a dry formulation or an aqueous solution. Compositions comprising polynucleotide sequences encoding SYNT or fragments of SYNT may be employed as hybridization probes. The probes may be stored in freeze-dried form and may be associated with a stabilizing agent such as a carbohydrate. In hybridizations, the probe may be deployed in an aqueous solution containing salts (e.g., NaCl), detergents (e.g., sodium dodecyl sulfate; SDS), and other components (e.g., Denhardt's solution, dry milk, salmon sperm DNA, etc.).

"Consensus sequence" refers to a nucleic acid sequence which has been subjected to repeated DNA sequence analysis to resolve uncalled bases, extended using the XL-PCR kit (PE Biosystems, Foster City CA) in the 5' and/or the 3' direction, and resequenced, or which has been assembled from one or more overlapping cDNA, EST, or genomic DNA fragments using a computer program for fragment assembly, such as the GELVIEW fragment assembly system (GCG, Madison WI) or Phrap (University of Washington, Seattle WA). Some sequences have been both extended and assembled to produce the consensus sequence.

"Conservative amino acid substitutions" are those substitutions that are predicted to least interfere with the properties of the original protein, i.e., the structure and especially the function of the protein is conserved and not significantly changed by such substitutions. The table below shows amino acids which may be substituted for an original amino acid in a protein and which are regarded as conservative amino acid substitutions.

| Original Residue | Conservative Substitution |
|---|---|
| Ala | Gly, Ser |
| Arg | His, Lys |
| Asn | Asp, Gln, His |
| Asp | Asn, Glu |
| Cys | Ala, Ser |
| Gln | Asn, Glu, His |
| Glu | Asp, Gln, His |
| Gly | Ala |
| His | Asn, Arg, Gln, Glu |
| Ile | Leu, Val |

(continued)

| Original Residue | Conservative Substitution |
|---|---|
| Leu | Ile, Val |
| Lys | Arg, Gin, Glu |
| Met | Leu, Ile |
| Phe | His, Met, Leu, Trp, Tyr |
| Ser | Cys, Thr |
| Thr | Ser, Val |
| Trp | Phe, Tyr |
| Tyr | His, Phe, Trp |
| Val | Ile, Leu, Thr |

Conservative amino acid substitutions generally maintain (a) the structure of the polypeptide backbone in the area of the substitution, for example, as a beta sheet or alpha helical conformation, (b) the charge or hydrophobicity of the molecule at the site of the substitution, and/or (c) the bulk of the side chain.

A "deletion" refers to a change in the amino acid or nucleotide sequence that results in the absence of one or more amino acid residues or nucleotides.

The term "derivative" refers to a chemically modified polynucleotide or polypeptide. Chemical modifications of a polynucleotide sequence can include, for example, replacement of hydrogen by an alkyl, acyl, hydroxyl, or amino group. A derivative polynucleotide encodes a polypeptide which retains at least one biological or immunological function of the natural molecule. A derivative polypeptide is one modified by glycosylation, pegylation, or any similar process that retains at least one biological or immunological function of the polypeptide from which it was derived.

A "detectable label" refers to a reporter molecule or enzyme that is capable of generating a measurable signal and is covalently or noncovalently joined to a polynucleotide or polypeptide.

A "fragment" is a unique portion of SYNT or the polynucleotide encoding SYNT which is identical in sequence to but shorter in length than the parent sequence. A fragment may comprise up to the entire length of the defined sequence, minus one nucleotide/amino acid residue. For example, a fragment may comprise from 5 to 1000 contiguous nucleotides or amino acid residues. A fragment used as a probe, primer, antigen, therapeutic molecule, or for other purposes, may be at least 5, 10, 15, 16, 20, 25, 30, 40, 50, 60, 75, 100, 150, 250 or at least 500 contiguous nucleotides or amino acid residues in length. Fragments may be preferentially selected from certain regions of a molecule. For example, a polypeptide fragment may comprise a certain length of contiguous amino acids selected from the first 250 or 500 amino acids (or first 25% or 50% of a polypeptide) as shown in a certain defined sequence. Clearly these lengths are exemplary, and any length that is supported by the specification, including the Sequence Listing, tables, and figures, may be encompassed by the present embodiments.

A fragment of SEQ ID NO:16-30 comprises a region of unique polynucleotide sequence that specifically identifies SEQ ID NO:16-30, for example, as distinct from any other sequence in the genome from which the fragment was obtained. A fragment of SEQ ID NO:16-30 is useful, for example, in hybridization and amplification technologies and in analogous methods that distinguish SEQ ID NO:16-30 from related polynucleotide sequences. The precise length of a fragment of SEQ ID NO:16-30 and the region of SEQ ID NO:16-30 to which the fragment corresponds are routinely determinable by one of ordinary skill in the art based on the intended purpose for the fragment.

A fragment of SEQ ID NO:1-15 is encoded by a fragment of SEQ ID NO:16-30. A fragment of SEQ ID NO:1-15 comprises a region of unique amino acid sequence that specifically identifies SEQ ID NO:1-15. For example, a fragment of SEQ ID NO:1-15 is useful as an immunogenic peptide for the development of antibodies that specifically recognize SEQ ID NO:1-15. The precise length of a fragment of SEQ ID NO:1-15 and the region of SEQ ID NO:1-15 to which the fragment corresponds are routinely determinable by one of ordinary skill in the art based on the intended purpose for the fragment.

A "full-length" polynucleotide sequence is one containing at least a translation initiation codon (e.g., methionine) followed by an open reading frame and a translation termination codon. A "full-length" polynucleotide sequence encodes a "full-length" polypeptide sequence.

"Homology" refers to sequence similarity or, interchangeably, sequence identity, between two or more polynucleotide sequences or two or more polypeptide sequences.

The terms "percent identity" and "% identity," as applied to polynucleotide sequences, refer to the percentage of residue matches between at least two polynucleotide sequences aligned using a standardized algorithm. Such an algorithm may insert, in a standardized and reproducible way, gaps in the sequences being compared in order to optimize alignment between two sequences, and therefore achieve a more meaningful comparison of the two sequences.

Percent identity between polynucleotide sequences may be determined using the default parameters of the CLUSTAL V algorithm as incorporated into the MEGALIGN version 3.12e sequence alignment program. This program is part of the LASERGENE software package, a suite of molecular biological analysis programs (DNASTAR, Madison WI). CLUSTAL V is described in Higgins, D.G. and P.M. Sharp (1989) CABIOS 5:151-153 and in Higgins. D.G. et al. (1992) CABIOS 8: 189-19 1. For pairwise alignments of polynucleotide sequences, the default parameters are set as follows: Ktuple=2, gap penalty=5, window=4, and "diagonals saved"=4. The "weighted" residue weight table is selected as the default. Percent identity is reported by CLUSTAL V as the "percent similarity" between aligned polynucleotide sequences.

Alternatively, a suite of commonly used and freely available sequence comparison algorithms is provided by the National Center for Biotechnology Information (NCBI) Basic Local Alignment Search Tool (BLAST) (Altschul, S.F. et al. (1990) J. Mol. Biol. 215:403-4 10), which is available from several sources, including the NCBI, Bethesda. MD, and on the Internet at http://www.ncbi.nlm.nih.gov/BLAST/. The BLAST software suite includes various sequence analysis programs including "blastn," that is used to align a known polynucleotide sequence with other polynucleotide sequences from a variety of databases. Also available is a tool called "BLAST 2 Sequences" that is used for direct pairwise comparison of two nucleotide sequences. "BLAST 2 Sequences" can be accessed and used interactively at http://www.ncbi.nlm.nih.gov/gorf/b12.html. The "BLAST 2 Sequences" tool can be used for both blastn and blastp (discussed below). BLAST programs are commonly used with gap and other parameters set to default settings. For example, to compare two nucleotide sequences, one may use blastn with the "BLAST 2 Sequences" tool Version 2.0.12 (April-21-2000) set at default parameters. Such default parameters may be, for example:

*Matrix: BLOSUM62*
*Reward for match: 1*
*Penalty for mismatch: -2*
*Open Gap: 5 and Extension Gap: 2 penalties*
*Gap x drop-off: 50*
*Expect: 10*
*Word Size: 1*
*Filter: on*

Percent identity may be measured over the length of an entire defined sequence, for example, as defined by a particular SEQ ID number, or may be measured over a shorter length, for example, over the length of a fragment taken from a larger, defined sequence, for instance, a fragment of at least 20, at least 30, at least 40, at least 50, at least 70, at least 100, or at least 200 contiguous nucleotides. Such lengths are exemplary only, and it is understood that any fragment length supported by the sequences shown herein, in the tables, figures, or Sequence Listing, may be used to describe a length over which percentage identity may be measured.

Nucleic acid sequences that do not show a high degree of identity may nevertheless encode similar amino acid sequences due to the degeneracy of the genetic code. It is understood that changes in a nucleic acid sequence can be made using this degeneracy to produce multiple nucleic acid sequences that all encode substantially the same protein.

The phrases "percent identity" and "% identity," as applied to polypeptide sequences, refer to the percentage of residue matches between at least two polypeptide sequences aligned using a standardized algorithm. Methods of polypeptide sequence alignment are well-known. Some alignment methods take into account conservative amino acid substitutions. Such conservative substitutions, explained in more detail above, generally preserve the charge and hydrophobicity at the site of substitution, thus preserving the structure (and therefore function) of the polypeptide,

Percent identity between polypeptide sequences may be determined using the default parameters of the CLUSTAL V algorithm as incorporated into the MEGALIGN version 3.12e sequence alignment program (described and referenced above). For pairwise alignments of polypeptide sequences using CLUSTAL V, the default parameters are set as follows: Ktuple=1, gap penalty=3, window=5, and "diagonals saved"=5. The PAM250 matrix is selected as the default residue weight table. As with polynucleotide alignments, the percent identity is reported by CLUSTAL V as the "percent similarity" between aligned polypeptide sequence pairs.

Alternatively the NCBI BLAST software suite may be used. For example, for a pairwise comparison of two polypeptide sequences, one may use the "BLAST 2 Sequences" tool Version 2.0.12 (Apr-21-2000) with blastp set at default parameters. Such default parameters may be, for example:

*Matrix: BLOSUM62*
*Open Gap: 11 and Extension Gap: 1 penalties*
*Gap x drop-off: 50*

*Expect: 10*
*Word Size: 3*
*Filter: on*

Percent identity may be measured over the length of an entire defined polypeptide sequence, for example, as defined by a particular SEQ ID number, or may be measured over a shorter length, for example, over the length of a fragment taken from a larger, defined polypeptide sequence, for instance, a fragment of at least 15, at least 20, at least 30, at least 40, at least 50, at least 70 or at least 150 contiguous residues. Such lengths are exemplary only, and it is understood that any fragment length supported by the sequences shown herein, in the tables, figures or Sequence Listing, may be used to describe a length over which percentage identity may be measured.
"Human artificial chromosomes" (HACs) are linear microchromosomes which may contain DNA sequences of about 6 kb to 10 Mb in size, and which contain all of the elements required for chromosome replication, segregation and maintenance.
The term "humanized antibody" refers to an antibody molecule in which the amino acid sequence in the non-antigen binding regions has been altered so that the antibody more closely resembles a human antibody, and still retains its original binding ability.
"Hybridization" refers to the process by which a polynucleotide strand anneals with a complementary strand through base pairing under defined hybridization conditions. Specific hybridization is an indication that two nucleic acid sequences share a high degree of complementarity. Specific hybridization complexes form under permissive annealing conditions and remain hybridized after the "washing" step(s). The washing step(s) is particularly important in determining the stringency of the hybridization process, with more stringent conditions allowing less non-specific binding, i.e., binding between pairs of nucleic acid strands that are not perfectly matched. Permissive conditions for annealing of nucleic acid sequences are routinely determinable by one of ordinary skill in the art and may be consistent among hybridization experiments. whereas wash conditions may be varied among experiments to achieve the desired stringency, and therefore hybridization specificity. Permissive annealing conditions occur, for example, at 68°C in the presence of about 6 x SSC, about 1% (w/v) SDS, and about 100 μg/ml sheared, denatured salmon sperm DNA.
Generally, stringency of hybridization is expressed, in part, with reference to the temperature under which the wash step is carried out. Such wash temperatures are typically selected to be about 5°C to 20°C lower than the thermal melting point ($T_m$) for the specific sequence at a defined ionic strength and pH. The $T_m$ is the temperature (under defined ionic strength and pH) at which 50% of the target sequence hybridizes to a perfectly matched probe. An equation for calculating $T_m$ and conditions for nucleic acid hybridization are well known and can be found in Sambrook, J. et al., 1989, Molecular Cloning: A Laboratory Manual, 2nd ed., vol. 1-3, Cold Spring Harbor Press, Plainview NY; specifically see volume 2, chapter 9.
High stringency conditions for hybridization between polynucleotides of the present invention include wash conditions of 68°C in the presence of about 0.2 x SSC and about 0.1% SDS. for 1 hour. Alternatively, temperatures of about 65°C, 60°C, 55°C, or 42°C may be used. SSC concentration may be varied from about 0.1 to 2 x SSC, with SDS being present at about 0.1%. Typically, blocking reagents are used to block non-specific hybridization. Such blocking reagents include, for instance, sheared and denatured salmon sperm DNA at about 100-200 μg/ml. Organic solvent, such as formamide at a concentration of about 35-50% v/v, may also be used under particular circumstances, such as for RNA:DNA hybridizations. Useful variations on these wash conditions will be readily apparent to those of ordinary skill in the art. Hybridization, particularly under high stringency conditions, may be suggestive of evolutionary similarity between the nucleotides. Such similarity is strongly indicative of a similar role for the nucleotides and their encoded polypeptides.
The term "hybridization complex" refers to a complex formed between two nucleic acid sequences by virtue of the formation of hydrogen bonds between complementary bases. A hybridization complex may be formed in solution (e.g., $C_0t$ or $R_0t$ analysis) or formed between one nucleic acid sequence present in solution and another nucleic acid sequence immobilized on a solid support (e.g., paper, membranes, filters, chips, pins or glass slides, or any other appropriate substrate to which cells or their nucleic acids have been fixed).
The words "-insertion" and "addition" refer to changes in an amino acid or nucleotide sequence resulting in the addition of one or more amino acid residues or nucleotides, respectively.
"Immune response" can refer to conditions associated with inflammation, trauma, immune disorders, or infectious or genetic disease, etc. These conditions can be characterized by expression of various factors, e.g., cytokines, chemokines, and other signaling molecules, which may affect cellular and systemic defense systems.
An "immunogenic fragment" is a polypeptide or oligopeptide fragment of SYNT which is capable of eliciting an immune response when introduced into a living organism, for example, a mammal. The term "immunogenic fragment" also includes any polypeptide or oligopeptide fragment of SYNT which is useful in any of the antibody production methods disclosed herein or known in the art.
The term "microarray" refers to an arrangement of a plurality of polynucleotides, polypeptides, or other chemical compounds on a substrate.

The terms "element" and "array element" refer to a polynucleotide, polypeptide, or other chemical compound having a unique and defined position on a microarray.

The term "modulate" refers to a change in the activity of SYNT. For example, modulation may cause an increase or a decrease in protein activity, binding characteristics, or any other biological, functional, or immunological properties of SYNT.

The phrases "nucleic acid" and "nucleic acid sequence" refer to a nucleotide, oligonucleotide, polynucleotide, or any fragment thereof. These phrases also refer to DNA or RNA of genomic or synthetic origin which may be single-stranded or double-stranded and may represent the sense or the antisense strand, to peptide nucleic acid (PNA), or to any DNA-like or RNA-like material.

"Operably linked" refers to the situation in which a first nucleic acid sequence is placed in a functional relationship with a second nucleic acid sequence. For instance, a promoter is operably linked to a coding sequence if the promoter affects the transcription or expression of the coding sequence. Operably linked DNA sequences may be in close proximity or contiguous and, where necessary to join two protein coding regions, in the same reading frame.

"Peptide nucleic acid" (PNA) refers to an antisense molecule or anti-gene agent which comprises an oligonucleotide of at least about 5 nucleotides in length linked to a peptide backbone of amino acid residues ending in lysine. The terminal lysine confers solubility to the composition. PNAs preferentially bind complementary single stranded DNA or RNA and stop transcript elongation, and may be pegylated to extend their lifespan in the cell.

"Post-translational modification" of an SYNT may involve lipidation, glycosylation, phosphorylation, acetylation, racemization, proteolytic cleavage, and other modifications known in the art. These processes may occur synthetically or biochemically. Biochemical modifications will vary by cell type depending on the enzymatic milieu of SYNT.

"Probe" refers to nucleic acid sequences encoding SYNT, their complements, or fragments thereof, which are used to detect identical, allelic or related nucleic acid sequences. Probes are isolated oligonucleotides or polynucleotides attached to a detectable label or reporter molecule. Typical labels include radioactive isotopes, ligands, chemiluminescent agents, and enzymes. "Primers" are short nucleic acids, usually DNA oligonucleotides, which may be annealed to a target polynucleotide by complementary base-pairing. The primer may then be extended along the target DNA strand by a DNA polymerase enzyme. Primer pairs can be used for amplification (and identification) of a nucleic acid sequence, e.g., by the polymerase chain reaction (PCR).

Probes and primers as used in the present invention typically comprise at least 15 contiguous nucleotides of a known sequence. In order to enhance specificity, longer probes and primers may also be employed, such as probes and primers that comprise at least 20, 25, 30, 40, 50, 60, 70, 80, 90, 100, or at least 150 consecutive nucleotides of the disclosed nucleic acid sequences. Probes and primers may be considerably longer than these examples, and it is understood that any length supported by the specification, including the tables, figures, and Sequence Listing, may be used.

Methods for preparing and using probes and primers are described in the references, for example Sambrook, J. et al., 1989, <u>Molecular Cloning: A Laboratory Manual,</u> $2^{nd}$ ed., vol. 1-3, Cold Spring Harbor Press, Plainview NY; Ausubel, F.M. et al.,1987, <u>Current Protocols in Molecular Biology,</u> Greene Publ. Assoc. & Wiley-Intersciences, New York NY; Innis, M. et al., 1990, <u>PCR Protocols, A Guide to Methods and Applications</u>, Academic Press, San Diego CA. PCR primer pairs can be derived from a known sequence, for example, by using computer programs intended for that purpose such as Primer (Version 0.5, 1991, Whitehead Institute for Biomedical Research, Cambridge MA).

Oligonucleotides for use as primers are selected using software known in the art for such purpose. For example, OLIGO 4.06 software is useful for the selection of PCR primer pairs of up to 100 nucleotides each, and for the analysis of oligonucleotides and larger polynucleotides of up to 5,000 nucleotides from an input polynucleotide sequence of up to 32 kilobases. Similar primer selection programs have incorporated additional features for expanded capabilities. For example, the PrimOU primer selection program (available to the public from the Genome Center at University of Texas South West Medical Center, Dallas TX) is capable of choosing specific primers from megabase sequences and is thus useful for designing primers on a genome-wide scope. The Primer3 primer selection program (available to the public from the Whitehead Institute/MIT Center for Genome Research, Cambridge MA) allows the user to input a "mispriming library," in which sequences to avoid as primer binding sites are user-specified. Primer3 is useful, in particular, for the selection of oligonucleotides for microarrays. (The source code for the latter two primer selection programs may also be obtained from their respective sources and modified to meet the user's specific needs.) The PrimeGen program (available to the public from the UK Human Genome Mapping Project Resource Centre, Cambridge UK) designs primers based on multiple sequence alignments, thereby allowing selection of primers that hybridize to either the most conserved or least conserved regions of aligned nucleic acid sequences. Hence, this program is useful for identification of both unique and conserved oligonucleotides and polynucleotide fragments. The oligonucleotides and polynucleotide fragments identified by any of the above selection methods are useful in hybridization technologies, for example, as PCR or sequencing primers, microarray elements, or specific probes to identify fully or partially complementary polynucleotides in a sample of nucleic acids. Methods of oligonucleotide selection are not limited to those described above.

A "recombinant nucleic acid" is a sequence that is not naturally occurring or has a sequence that is made by an artificial combination of two or more otherwise separated segments of sequence. This artificial combination is often accomplished by chemical synthesis or, more commonly, by the artificial manipulation of isolated segments of nucleic acids, e.g., by genetic engineering techniques such as those described in Sambrook, supra. The term recombinant includes nucleic acids that have been altered solely by addition, substitution, or deletion of a portion of the nucleic acid. Frequently, a recombinant nucleic acid may include a nucleic acid sequence operably linked to a promoter sequence. Such a recombinant nucleic acid may be part of a vector that is used, for example, to transform a cell.

Alternatively, such recombinant nucleic acids may be part of a viral vector, e.g., based on a vaccinia virus, that could be use to vaccinate a mammal wherein the recombinant nucleic acid is expressed, inducing a protective immunological response in the mammal.

A "regulatory element" refers to a nucleic acid sequence usually derived from untranslated regions of a gene and includes enhancers, promoters, introns, and 5' and 3' untranslated regions (UTRs). Regulatory elements interact with host or viral proteins which control transcription, translation, or RNA stability.

"Reporter molecules" are chemical or biochemical moieties used for labeling a nucleic acid, amino acid, or antibody. Reporter molecules include radionuclides; enzymes; fluorescent, chemiluminescent, or chromogenic agents; substrates; cofactors; inhibitors; magnetic particles; and other moieties known in the art.

An "RNA equivalent," in reference to a DNA sequence, is composed of the same linear sequence of nucleotides as the reference DNA sequence with the exception that all occurrences of the nitrogenous base thymine are replaced with uracil, and the sugar backbone is composed of ribose instead of deoxyribose.

The term "sample" is used in its broadest sense. A sample suspected of containing nucleic acids encoding SYNT, or fragments thereof, or SYNT itself, may comprise a bodily fluid; an extract from a cell, chromosome, organelle, or membrane isolated from a cell; a cell; genomic DNA, RNA, or cDNA, in solution or bound to a substrate; a tissue; a tissue print; etc.

The terms "specific binding" and "specifically binding" refer to that interaction between a protein or peptide and an agonist, an antibody, an antagonist, a small molecule, or any natural or synthetic binding composition. The interaction is dependent upon the presence of a particular structure of the protein, e.g., the antigenic determinant or epitope, recognized by the binding molecule. For example, if an antibody is specific for epitope "A," the presence of a polypeptide comprising the epitope A, or the presence of free unlabeled A, in a reaction containing free labeled A and the antibody will reduce the amount of labeled A that binds to the antibody.

The term "substantially purified" refers to nucleic acid or amino acid sequences that are removed from their natural environment and are isolated or separated, and are at least 60% free, preferably at least 75% free, and most preferably at least 90% free from other components with which they are naturally associated.

A "substitution" refers to the replacement of one or more amino acid residues or nucleotides by different amino acid residues or nucleotides, respectively.

"Substrate" refers to any suitable rigid or semi-rigid support including membranes, filters, chips, slides, wafers, fibers, magnetic or nonmagnetic beads, gels, tubing, plates, polymers, microparticles and capillaries. The substrate can have a variety of surface forms, such as wells, trenches, pins, channels and pores, to which polynucleotides or polypeptides are bound.

A "transcript image" refers to the collective pattern of gene expression by a particular cell type or tissue under given conditions at a given time.

"Transformation" describes a process by which exogenous DNA is introduced into a recipient cell. Transformation may occur under natural or artificial conditions according to various methods well known in the art, and may rely on any known method for the insertion of foreign nucleic acid sequences into a prokaryotic or eukaryotic host cell. The method for transformation is selected based on the type of host cell being transformed and may include, but is not limited to, bacteriophage or viral infection, electroporation, heat shock, lipofection, and particle bombardment. The term "transformed" cells includes stably transformed cells in which the inserted DNA is capable of replication either as an autonomously replicating plasmid or as part of the host chromosome, as well as transiently transformed cells which express the inserted DNA or RNA for limited periods of time.

A "transgenic organism," as used herein, is any organism, including but not limited to animals and plants, in which one or more of the cells of the organism contains heterologous nucleic acid introduced by way of human intervention, such as by transgenic techniques well known in the art. The nucleic acid is introduced into the cell, directly or indirectly by introduction into a precursor of the cell, by way of deliberate genetic manipulation, such as by microinjection or by infection with a recombinant virus. The term genetic manipulation does not include classical cross-breeding, or in vitro fertilization, but rather is directed to the introduction of a recombinant DNA molecule. The transgenic organisms contemplated in accordance with the present invention include bacteria, cyanobacteria, fungi, plants, and animals. The isolated DNA of the present invention can be introduced into the host by methods known in the art, for example infection, transfection, transformation or transconjugation. Techniques for transferring the DNA of the present invention into such organisms are widely known and provided in references such as Sambrook et al. (1989), supra.

A "variant" of a particular nucleic acid sequence is defined as a nucleic acid sequence having at least 40% sequence identity to the particular nucleic acid sequence over a certain length of one of the nucleic acid sequences using blastn with the "BLAST 2 Sequences" tool Version 2.0.9 (May-07-1999) set at default parameters. Such a pair of nucleic acids may show, for example, at least 50%, at least 60%, at least 70%, at least 80%, at least 85%, at least 90%, at least 95% or at least 98% or greater sequence identity over a certain defined length. A variant may be described as, for example, an "allelic" (as defined above), "splice," "species," or "polymorphic" variant. A splice variant may have significant identity to a reference molecule, but will generally have a greater or lesser number of polynucleotides due to alternative splicing of exons during mRNA processing. The corresponding polypeptide may possess additional functional domains or lack domains that are present in the reference molecule. Species variants are polynucleotide sequences that vary from one species to another. The resulting polypeptides generally will have significant amino acid identity relative to each other. A polymorphic variant is a variation in the polynucleotide sequence of a particular gene between individuals of a given species. Polymorphic variants also may encompass "single nucleotide polymorphisms" (SNPs) in which the polynucleotide sequence varies by one nucleotide base. The presence of SNPs may be indicative of, for example, a certain population, a disease state, or a propensity for a disease state.

A "variant" of a particular polypeptide sequence is defined as a polypeptide sequence having at least 40% sequence identity to the particular polypeptide sequence over a certain length of one of the polypeptide sequences using blastp with the "BLAST 2 Sequences" tool Version 2.0.9 (May-07-1999) set at default parameters. Such a pair of polypeptides may show, for example, at least 50%, at least 60%, at least 70%, at least 80%, at least 90%, at least 95%, or at least 98% or greater sequence identity over a certain defined length of one of the polypeptides.

## THE INVENTION

The invention is based on the discovery of new human synthetases (SYNT), the polynucleotides encoding SYNT, and the use of these compositions for the diagnosis, treatment, or prevention of immune, neuronal, and reproductive disorders, and cell proliferative disorders including cancer.

Table 1 lists the Incyte clones used to assemble full length nucleotide sequences encoding SYNT. Columns 1 and 2 show the sequence identification numbers (SEQ ID NOs) of the polypeptide and nucleotide sequences, respectively. Column 3 shows the clone IDs of the Incyte clones in which nucleic acids encoding each SYNT were identified. and column 4 shows the cDNA libraries from which these clones were isolated. Column 5 shows Incyte clones and their corresponding cDNA libraries. Clones for which cDNA libraries are not indicated were derived from pooled cDNA libraries. In some cases, GenBank sequence identifiers are also shown in column 5. The Incyte clones and GenBank cDNA sequences, where indicated, in column 5 were used to assemble the consensus nucleotide sequence of each SYNT and are useful as fragments in hybridization technologies.

The columns of Table 2 show various properties of each of the polypeptides of the invention: column 1 references the SEQ ID NO: column 2 shows the number of amino acid residues in each polypeptide; column 3 shows potential phosphorylation sites; column 4 shows potential glycosylation sites; column 5 shows the amino acid residues comprising signature sequences and motifs; column 6 shows homologous sequences as identified by BLAST analysis; and column 7 shows analytical methods and in some cases, searchable databases to which the analytical methods were applied. The methods of column 7 were used to characterize each polypeptide through sequence homology and protein motifs.

The columns of Table 3 show the tissue-specificity and diseases, disorders, or conditions associated with nucleotide sequences encoding SYNT. The first column of Table 3 lists the nucleotide SEQ ID NOs. Column 2 lists fragments of the nucleotide sequences of column 1. These fragments are useful, for example, in hybridization or amplification technologies to identify SEQ ID NO:16, SEQ ID NO:17, SEQ ID NO:18, SEQ ID NO:19 SEQ ID NO:20, SEQ ID NO:21, SEQ ID NO:22. SEQ ID NO:23, SEQ ID NO:25. SEQ ID NO:26, SEQ ID NO:27, SEQ ID NO:29, and SEQ ID NO:30 and to distinguish between SEQ ID NO:16, SEQ ID NO:17, SEQ ID NO:18, SEQ ID NO:19 SEQ ID NO:20. SEQ ID NO:21, SEQ ID NO:22. SEQ ID NO:23. SEQ ID NO:25, SEQ ID NO:26. SEQ ID NO:27, SEQ ID NO:29, and SEQ ID NO:30 and related polynucleotide sequences. The polypeptides encoded by these fragments are useful, for example, as immunogenic peptides. Column 3 lists tissue categories which express SYNT as a fraction of total tissues expressing SYNT. Column 4 lists diseases, disorders, or conditions associated with those tissues expressing SYNT as a fraction of total tissues expressing SYNT. Column 5 lists the vectors used to subclone each cDNA library.

The columns of Table 4 show descriptions of the tissues used to construct the cDNA libraries from which cDNA clones encoding SYNT were isolated. Column 1 references the nucleotide SEQ ID NOs, column 2 shows the cDNA libraries from which these clones were isolated, and column 3 shows the tissue origins and other descriptive information relevant to the cDNA libraries in column 2.

SEQ ID NO: 16 maps to chromosome 5 within the interval from 147.10 to 150.00 centiMorgans. SEQ ID NO: 17 maps to chromosome 10 within the interval from 137.60 to 139.20 centiMorgans. This interval also contains gene MXI1, a member of the MYC family. SEQ ID NO: 18 maps to chromosome 2 within the interval from 228.80 to 230.10 centiMorgans. This interval also contains a gene for a proto-oncogene encoding a tyrosine-protein kinase. SEQ ID NO:21 maps to chromosome 5 within the interval from 172.6 to 184.7 centiMorgans. SEQ ID NO:24 maps to chromosome 2 within the interval from 118.0 to 127.4 centiMorgans. SEQ ID NO:26 maps to chromosome 3 within the interval from 157.4 to 162.0 centiMorgans. SEQ ID NO:27 maps to chromosome 12 within the interval from 97.1 to 116.6 centiMorgans. SEQ ID NO:28 maps to chromosome 4 within the interval from 77.3 to 99.2 centiMorgans and to chromosome 5 within the intervals from 79.2 to 92.3 centiMorgans, from 116.3 to 127.9 centiMorgans, and from 157.6 to 163.0 centiMorgans. SEQ ID NO:29 maps to chromosome 1 within the interval from 242.5 to 258.7 centiMorgans and to chromosome 19 within the interval from 69.9 to 104.9 centiMorgans. SEQ ID NO:30 maps to chromosome 1 within the interval from 57.2 to 57.5 centiMorgans.

The invention also encompasses SYNT variants. A preferred SYNT variant is one which has at least about 80%, or alternatively at least about 90%, or even at least about 95% amino acid sequence identity to the SYNT amino acid sequence, and which contains at least one functional or structural characteristic of SYNT.

The invention also encompasses polynucleotides which encode SYNT. In a particular embodiment, the invention encompasses a polynucleotide sequence comprising a sequence selected from the group consisting of SEQ ID NO:16-30, which encodes SYNT. The polynucleotide sequences of SEQ ID NO:16-30, as presented in the Sequence Listing, embrace the equivalent RNA sequences, wherein occurrences of the nitrogenous base thymine are replaced with uracil, and the sugar backbone is composed of ribose instead of deoxyribose.

The invention also encompasses a variant of a polynucleotide sequence encoding SYNT. In particular, such a variant polynucleotide sequence will have at least about 70%. or alternatively at least about 85%, or even at least about 95% polynucleotide sequence identity to the polynucleotide sequence encoding SYNT. A particular aspect of the invention encompasses a variant of a polynucleotide sequence comprising a sequence selected from the group consisting of SEQ ID NO:16-30 which has at least about 70%, or alternatively at least about 85%, or even at least about 95% polynucleotide sequence identity to a nucleic acid sequence selected from the group consisting of SEQ ID NO:16-30. Any one of the polynucleotide variants described above can encode an amino acid sequence which contains at least one functional or structural characteristic of SYNT.

It will be appreciated by those skilled in the art that as a result of the degeneracy of the genetic code, a multitude of polynucleotide sequences encoding SYNT, some bearing minimal similarity to the polynucleotide sequences of any known and naturally occurring gene, may be produced. Thus, the invention contemplates each and every possible variation of polynucleotide sequence that could be made by selecting combinations based on possible codon choices. These combinations are made in accordance with the standard triplet genetic code as applied to the polynucleotide sequence of naturally occurring SYNT, and all such variations are to be considered as being specifically disclosed.

Although nucleotide sequences which encode SYNT and its variants are generally capable of hybridizing to the nucleotide sequence of the naturally occurring SYNT under appropriately selected conditions of stringency, it may be advantageous to produce nucleotide sequences encoding SYNT or its derivatives possessing a substantially different codon usage. e.g., inclusion of non-naturally occurring codons. Codons may be selected to increase the rate at which expression of the peptide occurs in a particular prokaryotic or eukaryotic host in accordance with the frequency with which particular codons are utilized by the host. Other reasons for substantially altering the nucleotide sequence encoding SYNT and its derivatives without altering the encoded amino acid sequences include the production of RNA transcripts having more desirable properties, such as a greater half-life, than transcripts produced from the naturally occurring sequence.

The invention also encompasses production of DNA sequences which encode SYNT and SYNT derivatives, or fragments thereof, entirely by synthetic chemistry. After production, the synthetic sequence may be inserted into any of the many available expression vectors and cell systems using reagents well known in the art. Moreover, synthetic chemistry may be used to introduce mutations into a sequence encoding SYNT or any fragment thereof.

Also encompassed by the invention are polynucleotide sequences that are capable of hybridizing to the claimed polynucleotide sequences, and, in particular, to those shown in SEQ ID NO:16-30 and fragments thereof under various conditions of stringency. (See, e.g., Wahl, G.M. and S.L. Berger (1987) Methods Enzymol. 152:399-407; Kimmel, A.R. (1987) Methods Enzymol. 152:507-511.) Hybridization conditions, including annealing and wash conditions, are described in "Definitions."

Methods for DNA sequencing are well known in the art and may be used to practice any of the embodiments of the invention. The methods may employ such enzymes as the Klenow fragment of DNA polymerase I. SEQUENASE (US Biochemical, Cleveland OH), Taq polymerase (PE Biosystems, Foster City CA), thermostable T7 polymerase (Amersham Pharmacia Biotech, Piscataway NJ), or combinations of polymerases and proofreading exonucleases such as those found in the ELONGASE amplification system (Life Technologies, Gaithersburg MD). Preferably, sequence preparation is automated with machines such as the MICROLAB 2200 liquid transfer system (Hamilton, Reno NV), PTC200 thermal cycler (MJ Research, Watertown MA) and ABI CATALYST 800 thermal cycler (PE Biosystems). Sequencing is then carried out using either the ABI 373 or 377 DNA sequencing system (PE Biosystems), the MEGABACE 1000 DNA sequencing system (Molecular Dynamics, Sunnyvale CA), or other systems known in the art. The resulting sequences are analyzed using a variety of algorithms which are well known in the art. (See, e.g., Ausubel, F.M. (1997) Short Protocols in Molecular Biology, John Wiley & Sons, New York NY, unit 7.7: Meyers, R.A. (1995) Molecular Biology and Biotechnology, Wiley VCH. New York NY, pp. 856-853.)

The nucleic acid sequences encoding SYNT may be extended utilizing a partial nucleotide sequence and employing various PCR-based methods known in the art to detect upstream sequences, such as promoters and regulatory elements. For example, one method which may be employed, restriction-site PCR, uses universal and nested primers to amplify unknown sequence from genomic DNA within a cloning vector. (See, e.g., Sarkar, G. (1993) PCR Methods Applic. 2:318-322.) Another method, inverse PCR, uses primers that extend in divergent directions to amplify unknown sequence from a circularized template. The template is derived from restriction fragments comprising a known genomic locus and surrounding sequences. (See, e.g., Triglia, T. et al. (1988) Nucleic Acids Res. 16:8186.) A third method, capture PCR, involves PCR amplification of DNA fragments adjacent to known sequences in human and yeast artificial chromosome DNA. (See, e.g., Lagerstrom, M. et al. (1991) PCR Methods Applic. 1:111-119.) In this method, multiple restriction enzyme digestions and ligations may be used to insert an engineered double-stranded sequence into a region of unknown sequence before performing PCR. Other methods which may be used to retrieve unknown sequences are known in the art. (See, e.g., Parker. J.D. et al. (1991) Nucleic Acids Res. 19:3055-3060). Additionally, one may use PCR, nested primers, and PROMOTERFINDER libraries (Clontech, Palo Alto CA) to walk genomic DNA. This procedure avoids the need to screen libraries and is useful in finding intron/exon junctions. For all PCR-based methods, primers may be designed using commercially available software, such as OLIGO 4.06 Primer Analysis software (National Biosciences, Plymouth MN) or another appropriate program, to be about 22 to 30 nucleotides in length, to have a GC content of about 50% or more, and to anneal to the template at temperatures of about 68°C to 72°C.

When screening for full-length cDNAs, it is preferable to use libraries that have been size-selected to include larger cDNAs. In addition, random-primed libraries, which often include sequences containing the 5' regions of genes, are preferable for situations in which an oligo d(T) library does not yield a full-length cDNA. Genomic libraries may be useful for extension of sequence into 5' non-transcribed regulatory regions.

Capillary electrophoresis systems which are commercially available may be used to analyze the size or confirm the nucleotide sequence of sequencing or PCR products. In particular, capillary sequencing may employ flowable polymers for electrophoretic separation, four different nucleotide-specific, laser-stimulated fluorescent dyes, and a charge coupled device camera for detection of the emitted wavelengths. Output/light intensity may be converted to electrical signal using appropriate software (e.g., GENOTYPER and SEQUENCE NAVIGATOR, PE Biosystems), and the entire process from loading of samples to computer analysis and electronic data display may be computer controlled. Capillary electrophoresis is especially preferable for sequencing small DNA fragments which may be present in limited amounts in a particular sample.

In another embodiment of the invention, polynucleotide sequences or fragments thereof which encode SYNT may be cloned in recombinant DNA molecules that direct expression of SYNT, or fragments or functional equivalents thereof, in appropriate host cells. Due to the inherent degeneracy of the genetic code, other DNA sequences which encode substantially the same or a functionally equivalent amino acid sequence may be produced and used to express SYNT.

The nucleotide sequences of the present invention can be engineered using methods generally known in the art in order to alter SYNT-encoding sequences for a variety of purposes including, but not limited to, modification of the cloning, processing, and/or expression of the gene product. DNA shuffling by random fragmentation and PCR reassembly of gene fragments and synthetic oligonucleotides may be used to engineer the nucleotide sequences. For example, oligonucleotide-mediated site-directed mutagenesis may be used to introduce mutations that create new restriction sites, alter glycosylation patterns, change codon preference, produce splice variants, and so forth.

The nucleotides of the present invention may be subjected to DNA shuffling techniques such as MOLECULARBREEDING (Maxygen Inc., Santa Clara CA: described in U.S. Patent Number 5.837.458: Chang, C.-C. et al. (1999) Nat. Biotechnol. 17:793-797: Christians. F.C. et al. (1999) Nat. Biotechnol. 17:259-264; and Crameri. A. et al. ( 1996) Nat. Biotechnol. 14:315-319) to alter or improve the biological properties of SYNT, such as its biological or enzymatic activity or its ability to bind to other molecules or compounds. DNA shuffling is a process by which a library of gene variants is produced using PCR-mediated recombination of gene fragments. The library is then subjected to selection or screening procedures that identify those gene variants with the desired properties. These preferred variants may then be pooled and further subjected to recursive rounds of DNA shuffling and selection/screening. Thus, genetic diversity is created through "artificial" breeding and rapid molecular evolution. For example, fragments of a single gene containing random point mutations may be recombined, screened, and then reshuffled until the desired properties are optimized. Alternatively, fragments of a given gene may be recombined with fragments of homologous genes in the same gene family, either from the same or different species, thereby maximizing the genetic diversity of multiple naturally occurring genes in a directed and controllable manner.

In another embodiment, sequences encoding SYNT may be synthesized, in whole or in part, using chemical methods well known in the art. (See, e.g., Caruthers. M.H. et al. (1980) Nucleic Acids Symp. Ser. 7:215-223; and Horn. T. et al. (1980) Nucleic Acids Symp. Ser. 7:225-232.) Alternatively. SYNT itself or a fragment thereof may be synthesized using chemical methods. For example, peptide synthesis can be performed using various solution-phase or solid-phase techniques. (See, e.g., Creighton. T. (1984) Proteins, Structures and Molecular Properties, WH Freeman, New York NY, pp. 55-60: and Roberge, J.Y. et al. (1995) Science 269:202-204.) Automated synthesis may be achieved using the ABI 431A peptide synthesizer (PE Biosystems). Additionally, the amino acid sequence of SYNT, or any part thereof, may be altered during direct synthesis and/or combined with sequences from other proteins, or any part thereof, to produce a variant polypeptide or a polypeptide having a sequence of a naturally occurring polypeptide.

The peptide may be substantially purified by preparative high performance liquid chromatography. (See, e. g., Chiez. R.M. and F.Z. Regnier (1990) Methods Enzymol. 182:392-421.) The composition of the synthetic peptides may be confirmed by amino acid analysis or by sequencing. (See, e.g., Creighton, supra, pp. 28-53.)

In order to express a biologically active SYNT, the nucleotide sequences encoding SYNT or derivatives thereof may be inserted into an appropriate expression vector, i.e., a vector which contains the necessary elements for transcriptional and translational control of the inserted coding sequence in a suitable host. These elements include regulatory sequences, such as enhancers, constitutive and inducible promoters, and 5' and 3' untranslated regions in the vector and in polynucleotide sequences encoding SYNT. Such elements may vary in their strength and specificity. Specific initiation signals may also be used to achieve more efficient translation of sequences encoding SYNT. Such signals include the ATG initiation codon and adjacent sequences, e.g. the Kozak sequence. In cases where sequences encoding SYNT and its initiation codon and upstream regulatory sequences are inserted into the appropriate expression vector, no additional transcriptional or translational control signals may be needed. However, in cases where only coding sequence, or a fragment thereof, is inserted, exogenous translational control signals including an in-frame ATG initiation codon should be provided by the vector. Exogenous translational elements and initiation codons may be of various origins, both natural and synthetic. The efficiency of expression may be enhanced by the inclusion of enhancers appropriate for the particular host cell system used. (See, e.g., Scharf, D. et al. (1994) Results Probl. Cell Differ. 20: 125-162.)

Methods which are well known to those skilled in the art may be used to construct expression vectors containing sequences encoding SYNT and appropriate transcriptional and translational control elements. These methods include in vitro recombinant DNA techniques, synthetic techniques, and in vivo genetic recombination. (See, e.g., Sambrook, J. et al. (1989) Molecular Cloning, A Laboratory Manual, Cold Spring Harbor Press, Plainview NY, ch. 4, 8, and 16-17; Ausubel, F.M. et al. (1995) Current Protocols in Molecular Biology, John Wiley & Sons, New York NY, ch. 9, 13, and 16.)

A variety of expression vector/host systems may be utilized to contain and express sequences encoding SYNT. These include, but are not limited to, microorganisms such as bacteria transformed with recombinant bacteriophage, plasmid, or cosmid DNA expression vectors; yeast transformed with yeast expression vectors; insect cell systems infected with viral expression vectors (e.g., baculovirus); plant cell systems transformed with viral expression vectors (e.g., cauliflower mosaic virus, CaMV, or tobacco mosaic virus, TMV) or with bacterial expression vectors (e. g., Ti or pBR322 plasmids); or animal cell systems. (See, e.g., Sambrook. supra; Ausubel, supra: Van Heeke, G. and S.M. Schuster (1989) J. Biol. Chem. 264:5503-5509-. Bitter. G.A. et al. (1987) Methods Enzymol. 153:516-544; Scorer. C.A. et al. (1994) Bio/Technology 12:181-184; Engelhard, E.K. et al. (1994) Proc. Natl. Acad. Sci. USA 91:3224-3227; Sandig. V. et al. (1996) Hum. Gene Ther. 7:1937-1945. Takamatsu. N. (1987) EMBO J. 6:307-311; Coruzzi. G. et al. (1984) EMBO J. 3:1671-1680: Broglie, R. et al. (1984) Science 224:838-843; Winter, J. et al. (1991) Results Probl. Cell Differ. 17:85-105: The McGraw Hill Yearbook of Science and Technology (1992) McGraw Hill. New York NY, pp. 191-196; Logan, J. and T. Shenk (1984) Proc. Natl. Acad. Sci. USA 81:3655-3659: and Harrington. J.J. et al. (1997) Nat. Genet. 15:345-355.) Expression vectors derived from retroviruses, adenoviruses, or herpes or vaccinia viruses. or from various bacterial plasmids, may be used for delivery of nucleotide sequences to the targeted organ, tissue, or cell population. (See, e.g.. Di Nicola, M. et al. (1998) Cancer Gen. Ther. 5(6):350-356; Yu, M. et al., (1993) Proc. Natl. Acad. Sci. USA 90(13):6340-6344: Buller, R.M. et al. (1985) Nature 317(6040):813-815; McGregor. D.P. et al. (1994) Mol. Immunol. 31(3):219-226; and Verma, I.M. and N. Somia (1997) Nature 389:239-242.) The invention is not limited by the host cell employed.

In bacterial systems, a number of cloning and expression vectors may be selected depending upon the use intended for polynucleotide sequences encoding SYNT. For example, routine cloning, subcloning, and propagation of polynucleotide sequences encoding SYNT can be achieved using a multifunctional E. coli vector such as PBLUESCRIPT (Stratagene, La Jolla CA) or PSPORT1 plasmid (Life Technologies). Ligation of sequences encoding SYNT into the vector's multiple cloning site disrupts the *lacZ* gene, allowing a colorimetric screening procedure for identification of transformed bacteria containing recombinant molecules. In addition, these vectors may be useful for in vitro transcription, dideoxy sequencing, single strand rescue with helper phage, and creation of nested deletions in the cloned sequence. (See, e.g., Van Heeke, G. and S.M. Schuster (1989) J. Biol. Chem. 264:5503-5509.) When large quantities of SYNT are needed, e.g. for the production of antibodies, vectors which direct high level expression of SYNT may be used. For example, vectors containing the strong, inducible T5 or T7 bacteriophage promoter may be used.

Yeast expression systems may be used for production of SYNT. A number of vectors containing constitutive or inducible promoters, such as alpha factor, alcohol oxidase, and PGH promoters, may be used in the yeast Saccharomyces cerevisiae or Pichia pastoris. In addition, such vectors direct either the secretion or intracellular retention of expressed proteins and enable integration of foreign sequences into the host genome for stable propagation. (See, e. g., Ausubel, 1995, supra; Bitter, supra; and Scorer, supra.)

Plant systems may also be used for expression of SYNT. Transcription of sequences encoding SYNT may be driven viral promoters, e.g., the 35S and 19S promoters of CaMV used alone or in combination with the omega leader sequence from TMV (Takamatsu, N. (1987) EMBO J. 6:307-311). Alternatively, plant promoters such as the small subunit of RUBISCO or heat shock promoters may be used. (See. e.g., Coruzzi, supra: Broglie. supra: and Winter, supra.) These constructs can be introduced into plant cells by direct DNA transformation or pathogen-mediated transfection. (See, e.g., The McGraw Hill Yearbook of Science and Technology (1992) McGraw Hill, New York NY, pp. 191-196.)

In mammalian cells, a number of viral-based expression systems may be utilized. In cases where an adenovirus is used as an expression vector, sequences encoding SYNT may be ligated into an adenovirus transcription/ translation complex consisting of the late promoter and tripartite leader sequence. Insertion in a non-essential E1 or E3 region of the viral genome may be used to obtain infective virus which expresses SYNT in host cells. (See, e.g., Logan, J. and T. Shenk (1984) Proc. Natl. Acad. Sci. USA 81:3655-3659.) In addition, transcription enhancers, such as the Rous sarcoma virus (RSV) enhancer, may be used to increase expression in mammalian host cells. SV40 or EBV-based vectors may also be used for high-level protein expression.

Human artificial chromosomes (HACs) may also be employed to deliver larger fragments of DNA than can be contained in and expressed from a plasmid. HACs of about 6 kb to 10.Mb are constructed and delivered via conventional delivery methods (liposomes, polycationic amino polymers, or vesicles) for therapeutic purposes. (See, e.g., Harrington, JJ. et al. (1997) Nat. Genet. 15:345-355.)

For long term production of recombinant proteins in mammalian systems, stable expression of SYNT in cell lines is preferred. For example, sequences encoding SYNT can be transformed into cell lines using expression vectors which may contain viral origins of replication and/or endogenous expression elements and a selectable marker gene on the same or on a separate vector. Following the introduction of the vector, cells may be allowed to grow for about 1 to 2 days in enriched media before being switched to selective media. The purpose of the selectable marker is to confer resistance to a selective agent, and its presence allows growth and recovery of cells which successfully express the introduced sequences. Resistant clones of stably transformed cells may be propagated using tissue culture techniques appropriate to the cell type.

Any number of selection systems may be used to recover transformed cell lines. These include, but are not limited to, the herpes simplex virus thymidine kinase and adenine phosphoribosyltransferase genes. for use in *tk*$^-$ and *apr*$^-$ cells, respectively. (See, e.g., Wigler, M. et al. (1977) Cell 11:223-232; Lowy, I. et al. (1980) Cell 22:817-823.) Also, antimetabolite, antibiotic, or herbicide resistance can be used as the basis for selection. For example, *dhfr* confers resistance to methotrexate; *neo* confers resistance to the aminoglycosides neomycin and G-418; and *als* and *pat* confer resistance to chlorsulfuron and phosphinotricin acetyltransferase, respectively. (See, e.g., Wigler, M. et al. (1980) Proc. Natl. Acad. Sci. USA 77:3567-3570; Colbere-Garapin, F. et al, (1981) J. Mol. Biol. 150:1-14.) Additional selectable genes have been described. e.g.. *trpB* and *hisD,* which alter cellular requirements for metabolites. (See. e.g., Hartman. S.C. and R.C. Mulligan (1988) Proc. Natl. Acad. Sci. USA 85:8047-8051.) Visible markers, e.g., anthocyanins, green fluorescent proteins (GFP: Clontech), β glucuronidase and its substrate β-glucuronide, or luciferase and its substrate luciferin may be used. These markers can be used not only to identify transformants, but also to quantify the amount of transient or stable protein expression attributable to a specific vector system. (See. e.g., Rhodes. C.A. (1995) Methods Mol. Biol. 55:121-131.)

Although the presence/absence of marker gene expression suggests that the gene of interest is also present, the presence and expression of the gene may need to be confirmed. For example, if the sequence encoding SYNT is inserted within a marker gene sequence, transformed cells containing sequences encoding SYNT can be identified by the absence of marker gene function. Alternatively, a marker gene can be placed in tandem with a sequence encoding SYNT under the control of a single promoter. Expression of the marker gene in response to induction or selection usually indicates expression of the tandem gene as well.

In general, host cells that contain the nucleic acid sequence encoding SYNT and that express SYNT may be identified by a variety of procedures known to those of skill in the art. These procedures include, but are not limited to, DNA-DNA or DNA-RNA hybridizations, PCR amplification, and protein bioassay or immunoassay techniques which include membrane, solution, or chip based technologies for the detection and/or quantification of nucleic acid or protein sequences.

Immunological methods for detecting and measuring the expression of SYNT using either specific polyclonal or monoclonal antibodies are known in the art. Examples of such techniques include enzyme-linked immunosorbent assays (ELISAs), radioimmunoassays (RIAs), and fluorescence activated cell sorting (FACS). A two-site, monoclonal-based immunoassay utilizing monoclonal antibodies reactive to two non-interfering epitopes on SYNT is preferred, but a competitive binding assay may be employed. These and other assays are well known in the art. (See, e.g., Hampton, R. et al. (1990) Serological Methods, a Laboratory Manual, APS Press, St. Paul MN, Sect. IV; Coligan, J.E. et al. (1997) Current Protocols in Immunology, Greene Pub. Associates and Wiley-Interscience, New York NY; and Pound, J.D. (1998) Immunochemical Protocols, Humana Press. Totowa NJ.)

A wide variety of labels and conjugation techniques are known by those skilled in the art and may be used in various nucleic acid and amino acid assays. Means for producing labeled hybridization or PCR probes for detecting sequences related to polynucleotides encoding SYNT include oligolabeling, nick translation, end-labeling, or PCR amplification using a labeled nucleotide. Alternatively, the sequences encoding SYNT, or any fragments thereof, may be cloned into a vector for the production of an mRNA probe. Such vectors are known in the art, are commercially available, and may be used to synthesize RNA probes in vitro by addition of an appropriate RNA polymerase such as T7, T3, or SP6 and labeled nucleotides. These procedures may be conducted using a variety of commercially available kits, such as those provided by Amersham Pharmacia Biotech, Promega (Madison WI), and US Biochemical. Suitable reporter molecules or labels which may be used for ease of detection include radionuclides, enzymes, fluorescent, chemiluminescent, or chromogenic agents, as well as substrates, cofactors, inhibitors, magnetic particles, and the like.

Host cells transformed with nucleotide sequences encoding SYNT may be cultured under conditions suitable for the expression and recovery of the protein from cell culture. The protein produced by a transformed cell may be secreted or retained intracellularly depending on the sequence and/or the vector used. As will be understood by those of skill in the art, expression vectors containing polynucleotides which encode SYNT may be designed to contain signal sequences which direct secretion of SYNT through a prokaryotic or eukaryotic cell membrane.

In addition, a host cell strain may be chosen for its ability to modulate expression of the inserted sequences or to process the expressed protein in the desired fashion. Such modifications of the polypeptide include, but are not limited to, acetylation, carboxylation, glycosylation, phosphorylation, lipidation, and acylation. Post-translational processing which cleaves a "prepro" or "pro" form of the protein may also be used to specify protein targeting, folding, and/or activity. Different host cells which have specific cellular machinery and characteristic mechanisms for post-translational activities (e.g., CHO, HeLa, MDCK, HEK293, and WI38) are available from the American Type Culture Collection (ATCC, Manassas VA) and may be chosen to ensure the correct modification and processing of the foreign protein.

In another embodiment of the invention, natural, modified, or recombinant nucleic acid sequences encoding SYNT may be ligated to a heterologous sequence resulting in translation of a fusion protein in any of the aforementioned host systems. For example, a chimeric SYNT protein containing a heterologous moiety that can be recognized by a commercially available antibody may facilitate the screening of peptide libraries for inhibitors of SYNT activity. Heterologous protein and peptide moieties may also facilitate purification of fusion proteins using commercially available affinity matrices. Such moieties include, but are not limited to, glutathione S-transferase (GST), maltose binding protein (MBP), thioredoxin (Trx), calmodulin binding peptide (CBP), 6-His, FLAG, *c-myc,* and hemagglutinin (HA), GST, MBP, Trx, CBP, and 6-His enable purification of their cognate fusion proteins on immobilized glutathione, maltose, phenylarsine oxide, calmodulin, and metal-chelate resins, respectively. FLAG, *c-myc,* and hemagglutinin (HA) enable immunoaffinity purification of fusion proteins using commercially available monoclonal and polyclonal antibodies that specifically recognize these epitope tags. A fusion protein may also be engineered to contain a proteolytic cleavage site located between the SYNT encoding sequence and the heterologous protein sequence, so that SYNT may be cleaved away from the heterologous moiety following purification. Methods for fusion protein expression and purification are discussed in Ausubel (1995, supra, ch. 10). A variety of commercially available kits may also be used to facilitate expression and purification of fusion proteins.

In a further embodiment of the invention, synthesis of radiolabeled SYNT may be achieved in vitro using the TNT rabbit reticulocyte lysate or wheat germ extract system (Promega). These systems couple transcription and translation of protein-coding sequences operably associated with the T7, T3, or SP6 promoters. Translation takes place in the presence of a radiolabeled amino acid precursor, for example, $^{35}$S-methionine.

SYNT of the present invention or fragments thereof may be used to screen for compounds that specifically bind to SYNT. At least one and up to a plurality of test compounds may be screened for specific binding to SYNT. Examples of test compounds include antibodies, oligonucleotides, proteins (e.g., receptors), or small molecules.

In one embodiment, the compound thus identified is closely related to the natural ligand of SYNT, e.g., a ligand or fragment thereof, a natural substrate, a structural or functional mimetic, or a natural binding partner. (See, Coligan, J.E. et al. (1991) Current Protocols in Immunology 1(2): Chapter 5.) Similarly, the compound can be closely related to the natural receptor to which SYNT binds, or to at least a fragment of the receptor, e.g., the ligand binding site. In either case, the compound can be rationally designed using known techniques. In one embodiment, screening for these compounds involves producing appropriate cells which express SYNT, either as a secreted protein or on the cell membrane. Preferred cells include cells from mammals, yeast, Drosophila, or E. coli. Cells expressing SYNT or cell membrane fractions which contain SYNT are then contacted with a test compound and binding, stimulation, or inhibition of activity of either SYNT or the compound is analyzed.

An assay may simply test binding of a test compound to the polypeptide, wherein binding is detected by a fluorophore, radioisotope, enzyme conjugate, or other detectable label. For example, the assay may comprise the steps of combining at least one test compound with SYNT, either in solution or affixed to a solid support, and detecting the binding of SYNT to the compound. Alternatively, the assay may detect or measure binding of a test compound in the presence of a labeled competitor. Additionally, the assay may be carried out using cell-free preparations, chemical libraries, or natural product mixtures, and the test compound(s) may be free in solution or affixed to a solid support.

SYNT of the present invention or fragments thereof may be used to screen for compounds that modulate the activity of SYNT. Such compounds may include agonists, antagonists, or partial or inverse agonists. In one embodiment, an assay is performed under conditions permissive for SYNT activity, wherein SYNT is combined with at least one test compound, and the activity of SYNT in the presence of a test compound is compared with the activity of SYNT in the absence of the test compound. A change in the activity of SYNT in the presence of the test compound is indicative of a compound that modulates the activity of SYNT. Alternatively, a test compound is combined with an in vitro or cell-free system comprising SYNT under conditions suitable for SYNT activity, and the assay is performed. In either of these assays, a test compound which modulates the activity of SYNT may do so indirectly and need not come in direct contact with the test compound. At least one and up to a plurality of test compounds may be screened.

In another embodiment, polynucleotides encoding SYNT or their mammalian homologs may be "knocked out" in an animal model system using homologous recombination in embryonic stem (ES) cells. Such techniques are well known in the art and are useful for the generation of animal models of human disease. (See, e.g., U.S. Patent No. 5,175,383 and U.S. Patent No. 5,767,337.) For example, mouse ES cells, such as the mouse 129/SvJ cell line, are derived from the early mouse embryo and grown in culture. The ES cells are transformed with a vector containing the gene of interest disrupted by a marker gene, e.g., the neomycin phosphotransferase gene (neo; Capecchi, M.R. (1989) Science 244:1288-1292). The vector integrates into the corresponding region of the host genome by homologous recombination. Alternatively, homologous recombination takes place using the Cre-loxP system to knockout a gene of interest in a tissue- or developmental stage-specific manner (Marth, J.D. (1996) Clin. Invest. 97:1999-2002: Wagner, K.U. et al. (1997) Nucleic Acids Res. 25:4323-4330). Transformed ES cells are identified and microinjected into mouse cell blastocysts such as those from the C57BL/6 mouse strain. The blastocysts are surgically transferred to pseudopregnant dams, and the resulting chimeric progeny are genotyped and bred to produce heterozygous or homozygous strains. Transgenic animals thus generated may be tested with potential therapeutic or toxic agents.

Polynucleotides encoding SYNT may also be manipulated in vitro in ES cells derived from human blastocysts. Human ES cells have the potential to differentiate into at least eight separate cell lineages including endoderm, mesoderm, and ectodermal cell types. These cell lineages differentiate into, for example, neural cells, hematopoietic lineages, and cardiomyocytes (Thomson. J.A. et al. (1998) Science 282:1145-1147).

Polynucleotides encoding SYNT can also be used to create "knockin" humanized animals (pigs) or transgenic animals (mice or rats) to model human disease. With knockin technology, a region of a polynucleotide encoding SYNT is injected into animal ES cells, and the injected sequence integrates into the animal cell genome. Transformed cells are injected into blastulae, and the blastulae are implanted as described above. Transgenic progeny or inbred lines are studied and treated with potential pharmaceutical agents to obtain information on treatment of a human disease. Alternatively, a mammal inbred to overexpress SYNT, e.g., by secreting SYNT in its milk, may also serve as a convenient source of that protein (Janne, J. et al. (1998) Biotechnol. Annu. Rev. 4:55-74).

## THERAPEUTICS

Chemical and structural similarity, e.g., in the context of sequences and motifs, exists between regions of SYNT and human synthetases. In addition, the expression of SYNT is closely associated with hematopoietic/immune, cancerous, proliferating, inflamed, immune, nervous, gastrointestinal and reproductive tissues. Therefore, SYNT appears to play a role in an immune disorder such as inflammation, actinic keratosis, acquired immunodeficiency syndrome (AIDS). Addison's disease, adult respiratory distress syndrome, allergies, ankylosing spondylitis, amyloidosis, anemia, arteriosclerosis, asthma, atherosclerosis, autoimmune hemolytic anemia, autoimmune thyroiditis, bronchitis, bursitis, cholecystitis, cirrhosis, contact dermatitis, Crohn's disease, atopic dermatitis, dermatomyositis, diabetes mellitus, emphysema, erythroblastosis fetalis, erythema nodosum, atrophic gastritis, glomerulonephritis, Goodpasture's syndrome, gout, Graves' disease, Hashimoto's thyroiditis, paroxysmal nocturnal hemoglobinuria, hepatitis. hypereosinophilia, irritable bowel syndrome, episodic lymphopenia with lymphocytotoxins, mixed connective tissue disease (MCTD), multiple sclerosis, myasthenia gravis, myocardial or pericardial inflammation, myelofibrosis, osteoarthritis, osteoporosis, pancreatitis, polycythemia vera, polymyositis, psoriasis, Reiter's syndrome, rheumatoid arthritis, scleroderma, Sjögren's syndrome, systemic anaphylaxis, systemic lupus erythematosus, systemic sclerosis, primary thrombocythemia, thrombocytopenic purpura, ulcerative colitis, uveitis, Werner syndrome, complications of cancer, hemodialysis, and extracorporeal circulation, trauma, viral, bacterial, fungal, parasitic, protozoal, and helminthic infections, and hematopoietic cancer including lymphoma, leukemia, and myeloma; a neuronal disorder, such as akathesia, Alzheimer's disease, amnesia, amyotrophic lateral sclerosis, bipolar disorder, catatonia, cerebral neoplasms, dementia, depression, diabetic neuropathy, Down's syndrome, tardive dyskinesia, dystonias, epilepsy, Huntington's disease, peripheral neuropathy, multiple sclerosis, neurofibromatosis, Parkinson's disease, paranoid psychoses, postherpetic neuralgia, schizophrenia, and Tourette's disorder; a reproductive disorder, such as a disorder of prolactin production, infertility, including tubal disease, ovulatory defects, and endometriosis, a disruption of the estrous cycle, a disruption of the menstrual cycle, polycystic ovary syndrome, ovarian hyperstimulation syndrome, an endometrial or ovarian tumor, a uterine fibroid, autoimmune disorders, an ectopic pregnancy, and teratogenesis; cancer of the breast, fibrocystic breast disease, and galactorrhea; a disruption of spermatogenesis, abnormal sperm physiology, cancer of the testis, cancer of the prostate, benign prostatic hyperplasia, prostatitis, Peyronie's disease, impotence, carcinoma of the male breast, and gynecomastia; and a cell proliferative disorder, such as actinic keratosis, arteriosclerosis, atherosclerosis, bursitis, cirrhosis, hepatitis, mixed connective tissue disease (MCTD), myelofibrosis, paroxysmal nocturnal hemoglobinuria, polycythemia vera, psoriasis, primary thrombocythemia, and cancers including adenocarcinoma, leukemia, lymphoma, melanoma, myeloma, sarcoma, teratocarcinoma, and, in particular, a cancer of the adrenal gland, bladder, bone, bone marrow, brain, breast, cervix, gall bladder, ganglia, gastrointestinal tract, heart, kidney, liver, lung, muscle, ovary, pancreas, parathyroid, penis, prostate, salivary glands, skin, spleen, testis, thymus, thyroid, and uterus.

In another embodiment. a vector capable of expressing SYNT or a fragment or derivative thereof may be administered to a subject to treat or prevent a disorder associated with decreased expression or activity of SYNT including, but not limited to, those described above.

In a further embodiment, a pharmaceutical composition comprising a substantially purified SYNT in conjunction with a suitable pharmaceutical carrier may be administered to a subject to treat or prevent a disorder associated with decreased expression or activity of SYNT including, but not limited to, those provided above.

In still another embodiment, an agonist which modulates the activity of SYNT may be administered to a subject to treat or prevent a disorder associated with decreased expression or activity of SYNT including, but not limited to, those listed above.

In a further embodiment, an antagonist of SYNT may be administered to a subject to treat or prevent a disorder associated with increased expression or activity of SYNT. Examples of such disorders include, but are not limited to, those immune, neuronal, reproductive, and cell proliferative disorders described above. In one aspect, an antibody which specifically binds SYNT may be used directly as an antagonist or indirectly as a targeting or delivery mechanism for bringing a pharmaceutical agent to cells or tissues which express SYNT.

In an additional embodiment, a vector expressing the complement of the polynucleotide encoding SYNT may be administered to a subject to treat or prevent a disorder associated with increased expression or activity of SYNT including, but not limited to, those described above.

In other embodiments, any of the proteins, antagonists, antibodies. agonists, complementary sequences, or vectors of the invention may be administered in combination with other appropriate therapeutic agents. Selection of the appropriate agents for use in combination therapy may be made by one of ordinary skill in the art, according to conventional pharmaceutical principles. The combination of therapeutic agents may act synergistically to effect the treatment or prevention of the various disorders described above. Using this approach, one may be able to achieve therapeutic efficacy with lower dosages of each agent, thus reducing the potential for adverse side effects.

An antagonist of SYNT may be produced using methods which are generally known in the art. In particular, purified SYNT may be used to produce antibodies or to screen libraries of pharmaceutical agents to identify those which specifically bind SYNT. Antibodies to SYNT may also be generated using methods that are well known in the art. Such antibodies may include, but are not limited to, polyclonal, monoclonal, chimeric, and single chain antibodies. Fab fragments, and fragments produced by a Fab expression library. Neutralizing antibodies (i.e., those which inhibit dimer formation) are generally preferred for therapeutic use.

For the production of antibodies, various hosts including goats, rabbits, rats, mice, humans, and others may be immunized by injection with SYNT or with any fragment or oligopeptide thereof which has immunogenic properties. Depending on the host species, various adjuvants may be used to increase immunological response. Such adjuvants include, but are not limited to, Freund's, mineral gels such as aluminum hydroxide, and surface active substances such as lysolecithin, pluronic polyols, polyanions, peptides, oil emulsions, KLH, and dinitrophenol. Among adjuvants used in humans, BCG (bacilli Calmette-Guerin) and Corynebacterium parvum are especially preferable.

It is preferred that the oligopeptides, peptides, or fragments used to induce antibodies to SYNT have an amino acid sequence consisting of at least about 5 amino acids, and generally will consist of at least about 10 amino acids. It is also preferable that these oligopeptides, peptides, or fragments are identical to a portion of the amino acid sequence of the natural protein. Short stretches of SYNT amino acids may be fused with those of another protein, such as KLH, and antibodies to the chimeric molecule may be produced.

Monoclonal antibodies to SYNT may be prepared using any technique which provides for the production of antibody molecules by continuous cell lines in culture. These include, but are not limited to, the hybridoma technique, the human B-cell hybridoma technique, and the EBV-hybridoma technique. (See, e.g., Kohler, G. et al. (1975) Nature 256:495-497; Kozbor, D. et al. (1985) J. Immunol. Methods 81:31-42; Cote, R.J. et al. (1983) Proc. Natl. Acad. Sci. USA 80:2026-2030; and Cole, S.P. et al. (1984) Mol. Cell Biol. 62:109-120.)

In addition, techniques developed for the production of "chimeric antibodies," such as the splicing of mouse antibody genes to human antibody genes to obtain a molecule with appropriate antigen specificity and biological activity, can be used. (See, e.g., Morrison, S.L. et al. (1984) Proc. Natl. Acad. Sci. USA 81:6851-6855; Neuberger, M.S. et al. (1984) Nature 312:604-608; and Takeda, S. et al. (1985) Nature 314:452-454.) Alternatively, techniques described for the production of single chain antibodies may be adapted, using methods known in the art, to produce SYNT-specific single chain antibodies. Antibodies with related specificity, but of distinct idiotypic composition, may be generated by chain shuffling from random combinatorial immunoglobulin libraries. (See, e.g., Burton. D.R. (1991) Proc. Natl. Acad. Sci. USA 88:10134-10137.)

Antibodies may also be produced by inducing in vivo production in the lymphocyte population or by screening immunoglobulin libraries or panels of highly specific binding reagents as disclosed in the literature. (See, e.g., Orlandi. R. et al. (1989) Proc. Natl. Acad. Sci. USA 86:3833-3837; Winter, G. et al. (1991) Nature 349:293-299.)

Antibody fragments which contain specific binding sites for SYNT may also be generated. For example, such fragments include, but are not limited to, $F(ab')_2$ fragments produced by pepsin digestion of the antibody molecule and Fab fragments generated by reducing the disulfide bridges of the F(ab')2 fragments. Alternatively, Fab expression libraries may be constructed to allow rapid and easy identification of monoclonal Fab fragments with the desired specificity. (See, e.g., Huse, W.D. et al. (1989) Science 246:1275-1281.)

Various immunoassays may be used for screening to identify antibodies having the desired specificity. Numerous protocols for competitive binding or immunoradiometric assays using either polyclonal or monoclonal antibodies with established specificities are well known in the art. Such immunoassays typically involve the measurement of complex formation between SYNT and its specific antibody. A two-site, monoclonal-based immunoassay utilizing monoclonal antibodies reactive to two non-interfering SYNT epitopes is generally used, but a competitive binding assay may also be employed (Pound, supra).

Various methods such as Scatchard analysis in conjunction with radioimmunoassay techniques may be used to assess the affinity of antibodies for SYNT. Affinity is expressed as an association constant, $K_a$, which is defined as the molar concentration of SYNT-antibody complex divided by the molar concentrations of free antigen and free antibody under equilibrium conditions. The $K_a$ determined for a preparation of polyclonal antibodies, which are heterogeneous in their affinities for multiple SYNT epitopes, represents the average affinity, or avidity, of the antibodies for SYNT. The $K_a$, determined for a preparation of monoclonal antibodies, which are monospecific for a particular SYNT epitope, represents a true measure of affinity. High-affinity antibody preparations with $K_a$ ranging from about $10^9$ to $10^{12}$ L/mole are preferred for use in immunoassays in which the SYNT-antibody complex must withstand rigorous manipulations. Low-affinity antibody preparations with $K_a$, ranging from about $10^6$ to $10^7$ L/mole are preferred for use in immunopurification and similar procedures which ultimately require dissociation of SYNT, preferably in active form, from the antibody (Catty, D. (1988) Antibodies. Volume I: A Practical Approach, IRL Press, Washington DC; Liddell, J. E. and A. Cryer ( 1991 ) A Practical Guide to Monoclonal Antibodies, John Wiley & Sons, New York NY).

The titer and avidity of polyclonal antibody preparations may be further evaluated to determine the quality and suitability of such preparations for certain downstream applications. For example, a polyclonal antibody preparation containing at least 1-2 mg specific antibody/ml, preferably 5-10 mg specific antibody/ml, is generally employed in procedures requiring precipitation of SYNT-antibody complexes. Procedures for evaluating antibody specificity, titer, and avidity, and guidelines for antibody quality and usage in various applications, are generally available. (See, e.g., Catty, supra, and Coligan et al., supra.)

In another embodiment of the invention, the polynucleotides encoding SYNT, or any fragment or complement thereof, may be used for therapeutic purposes. In one aspect, modifications of gene expression can be achieved by designing complementary sequences or antisense molecules (DNA, RNA, PNA, or modified oligonucleotides) to the coding or regulatory regions of the gene encoding SYNT. Such technology is well known in the art, and antisense oligonucleotides or larger fragments can be designed from various locations along the coding or control regions of sequences encoding SYNT. (See, e.g., Agrawal, S., ed. (1996) Antisense Therapeutics, Humana Press Inc., Totawa NJ.)

In therapeutic use, any gene delivery system suitable for introduction of the antisense sequences into appropriate target cells can be used. Antisense sequences can be delivered intracellularly in the form of an expression plasmid which, upon transcription, produces a sequence complementary to at least a portion of the cellular sequence encoding the target protein. (See, e.g.. Slater. J.E. et al, (1998) J. Allergy Clin. Immunol, 102(3):469-475; and Scanlon, K.J. et al. (1995) 9(13):1288-1296.) Antisense sequences can also be introduced intracellularly through the use of viral vectors, such as retrovirus and adeno-associated virus vectors. (See, e.g.. Miller. A.D. (1990) Blood 76:271: Ausubel, supra; Uckert, W. and W. Walther (1994) Pharmacol. Ther. 63(3):323-347.) Other gene delivery mechanisms include liposome-derived systems, artificial viral envelopes, and other systems known in the art. (See, e.g., Rossi, J.J. (1995) Br. Med. Bull. 51(1):217-225: Boado, R.J. et al. (1998) J. Pharm. Sci. 87(11):1308-1315: and Morris, M.C. et al. (1997) Nucleic Acids Res. 25(14):2730-2736.)

In another embodiment of the invention, polynucleotides encoding SYNT may be used for somatic or germline gene therapy. Gene therapy may be performed to (i) correct a genetic deficiency (e.g., in the cases of severe combined immunodeficiency (SCID)-X1 disease characterized by X-linked inheritance (Cavazzana-Calvo, M. et al. (2000) Science 288:669-672), severe combined immunodeficiency syndrome associated with an inherited adenosine deaminase (ADA) deficiency (Blaese, R.M. et al, (1995) Science 270:475-480; Bordignon, C. et al, (1995) Science 270:470-475), cystic fibrosis (Zabner, J. et al. (1993) Cell 75:207-216; Crystal. R.G. et al. (1995) Hum. Gene Therapy 6:643-666; Crystal, R.G. et al, (1995) Hum. Gene Therapy 6:667-703), thalassamias, familial hypercholesterolemia, and hemophilia resulting from Factor VIII or Factor IX deficiencies (Crystal, R.G. (1995) Science 270:404-410; Verma, I.M. and Somia, N. (1997) Nature 389:239-242)), (ii) express a conditionally lethal gene product (e.g., in the case of cancers which result from unregulated cell proliferation), or (iii) express a protein which affords protection against intracellular parasites (e.g., against human retroviruses, such as human immunodeficiency virus (HIV) (Baltimore, D. (1988) Nature 335:395-396; Poeschla, E. et al. (1996) Proc. Natl. Acad. Sci. USA. 93:11395-11399), hepatitis B or C virus (HBV, HCV); fungal parasites, such as Candida albicans and Paracoccidioides brasiliensis; and protozoan parasites such as Plasmodium falciparum and Trypanosoma cruzi). In the case where a genetic deficiency in SYNT expression or regulation causes disease, the expression of SYNT from an appropriate population of transduced cells may alleviate the clinical manifestations caused by the genetic deficiency.

In a further embodiment of the invention, diseases or disorders caused by deficiencies in SYNT are treated by constructing mammalian expression vectors encoding SYNT and introducing these vectors by mechanical means into SYNT-deficient cells. Mechanical transfer technologies for use with cells in vivo or ex vitro include (i) direct DNA microinjection into individual cells, (ii) ballistic gold particle delivery, (iii) liposome-mediated transfection, (iv) receptor-mediated gene transfer, and (v) the use of DNA transposons (Morgan, R.A. and W.F. Anderson (1993) Annu. Rev. Biochem. 62:191-217; Ivics, Z. (1997) Cell 91:501-5 10: Boulay, J-L. and H. Récipon (1998) Curr. Opin. Biotechnol. 9: 445-450).

Expression vectors that may be effective for the expression of SYNT include, but are not limited to, the PCDNA 3.1, EPITAG, PRCCMV2, PREP, PVAX vectors (Invitrogen, Carlsbad CA), PCMV-SCRIPT, PCMV-TAG, PEGSH/PERV (Stratagene, La Jolla CA), and PTET-OFF, PTET-ON, PTRE2, PTRE2-LUC, PTK-HYG (Clontech, Palo Alto CA). SYNT may be expressed using (i) a constitutively active promoter, (e.g., from cytomegalovirus (CMV), Rous sarcoma virus (RSV), SV40 virus, thymidine kinase (TK), or β-actin genes), (ii) an inducible promoter (e.g., the tetracycline-regulated promoter (Gossen, M. and H. Bujard (1992) Proc. Natl. Acad. Sci. USA 89:5547-5551; Gossen. M. et al. (1995) Science 268:1766-1769; Rossi, F.M.V. and H.M. Blau (1998) Curr. Opin. Biotechnol. 9:451-456), commercially available in the T-REX plasmid (Invitrogen)); the ecdysone-inducible promoter (available in the plasmids PVGRXR and FIND; Invitrogen); the FK506/rapamycin inducible promoter; or the RU486/mifepristone inducible promoter (Rossi, F.M.V. and H. M. Blau, supra)), or (iii) a tissue-specific promoter or the native promoter of the endogenous gene encoding SYNT from a normal individual.

Commercially available liposome transformation kits (e.g., the PERFECT LIPID TRANSFECTION KIT, available from Invitrogen) allow one with ordinary skill in the art to deliver polynucleotides to target cells in culture and require minimal effort to optimize experimental parameters. In the alternative, transformation is performed using the calcium phosphate method (Graham, F.L. and A.J. Eb (1973) Virology 52:456-467), or by electroporation (Neumann, E. et al, (1982) EMBO J. 1:841-845), The introduction of DNA to primary cells requires modification of these standardized mammalian transfection protocols.

In another embodiment of the invention, diseases or disorders caused by genetic defects with respect to SYNT expression are treated by constructing a retrovirus vector consisting of (i) the polynucleotide encoding SYNT under the control of an independent promoter or the retrovirus long terminal repeat (LTR) promoter, (ii) appropriate RNA packaging signals, and (iii) a Rev-responsive element (RRE) along with additional retrovirus cis-acting RNA sequences and coding sequences required for efficient vector propagation. Retrovirus vectors (e.g., PFB and PFBNEO) are commercially available (Stratagene) and are based on published data (Riviere, I. et al. (1995) Proc. Natl. Acad. Sci. USA 92:6733-6737). incorporated by reference herein. The vector is propagated in an appropriate vector producing cell line (VPCL) that expresses an envelope gene with a tropism for receptors on the target cells or a promiscuous envelope protein such as VSVg (Armentano, D. et al. (1987) J. Virol. 61:1647-1650; Bender, M.A. et al. (1987) J. Virol. 61:1639-1646; Adam, M.A. and A.D. Miller (1988) J. Virol. 62:3802-3806; Dull. T. et al. (1998) J. Virol. 72:8463-8471; Zufferey, R. et al. (1998) J. Virol. 72:9873-9880). U.S. Patent Number 5.910,434 to Rigg ("Method for obtaining retrovirus packaging cell lines producing high transducing efficiency retroviral supernatant") discloses a method for obtaining retrovirus packaging cell lines and is hereby incorporated by reference. Propagation of retrovirus vectors, transduction of a population of cells (e.g., CD4+ T-cells), and the return of transduced cells to a patient are procedures well known to persons skilled in the art of gene therapy and have been well documented (Ranga, U. et al. (1997) J. Virol, 71: 7020-7029; Bauer. G. et al, (1997) Blood 89:2259-2267; Bonyhadi, M.L. (1997) J. Virol, 71:4707-4716: Ranga, U. et al. (1998) Proc. Natl. Acad. Sci. USA 95:1201-1206; Su. L. (1997) Blood 89:2283-2290).

In the alternative, an adenovirus-based gene therapy delivery system is used to deliver polynucleotides encoding SYNT to cells which have one or more genetic abnormalities with respect to the expression of SYNT. The construction and packaging of adenovirus-based vectors are well known to those with ordinary skill in the art. Replication defective adenovirus vectors have proven to be versatile for importing genes encoding immunoregulatory proteins into intact islets in the pancreas (Csete. M.E. et al. (1995) Transplantation 27:263-268). Potentially useful adenoviral vectors are described in U.S. Patent Number 5.707,618 to Armentano ("Adenovirus vectors for gene therapy"), hereby incorporated by reference. For adenoviral vectors, see also Antinozzi, P.A. et al. (1999) Annu. Rev. Nutr. 19: 511-544; and Verma, I.M. and N. Somia (1997) Nature 18:389:239-242, both incorporated by reference herein.

In another alternative, a herpes-based, gene therapy delivery system is used to deliver polynucleotides encoding SYNT to target cells which have one or more genetic abnormalities with respect to the expression of SYNT. The use of herpes simplex virus (HSV)-based vectors may be especially valuable for introducing SYNT to cells of the central nervous system, for which HSV has a tropism. The construction and packaging of herpes-based vectors are well known to those with ordinary skill in the art. A replication-competent herpes simplex virus (HSV) type 1-based vector has been used to deliver a reporter gene to the eyes of primates (Liu, X. et al. (1999) Exp. Eye Res. 169: 385-395). The construction of a HSV-1 virus vector has also been disclosed in detail in U.S. Patent Number 5,804,413 to DeLuca ("Herpes simplex virus strains for gene transfer"), which is hereby incorporated by reference. U.S. Patent Number 5,804,413 teaches the use of recombinant HSV d92 which consists of a genome containing at least one exogenous gene to be transferred to a cell under the control of the appropriate promoter for purposes including human gene therapy. Also taught by this patent are the construction and use of recombinant HSV strains deleted for ICP4, ICP27 and ICP22. For HSV vectors, see also Coins. W.F. et al. (1999) J. Virol. 73:519-532 and Xu, H. et al. (1994) Dev. Biol. 163:152-161, hereby incorporated by reference. The manipulation of cloned herpesvirus sequences, the generation of recombinant virus following the transfection of multiple plasmids containing different segments of the large herpesvirus genomes, the growth and propagation of herpesvirus, and the infection of cells with herpesvirus are techniques well known to those of ordinary skill in the art.

In another alternative, an alphavirus (positive, single-stranded RNA virus) vector is used to deliver polynucleotides encoding SYNT to target cells. The biology of the prototypic alphavirus, Semliki Forest Virus (SFV), has been studied extensively and gene transfer vectors have been based on the SFV genome (Garoff, H. and K.-J. Li (1998) Curr. Opin. Biotech. 9:464-469). During alphavirus RNA replication, a subgenomic RNA is generated that normally encodes the viral capsid proteins. This subgenomic RNA replicates to higher levels than the full-length genomic RNA, resulting in the overproduction of capsid proteins relative to the viral proteins with enzymatic activity (e.g., protease and polymerase). Similarly, inserting the coding sequence for SYNT into the alphavirus genome in place of the capsid-coding region results in the production of a large number of SYNT-coding RNAs and the synthesis of high levels of SYNT in vector transduced cells. While alphavirus infection is typically associated with cell lysis within a few days, the ability to establish a persistent infection in hamster normal kidney cells (BHK-21) with a variant of Sindbis virus (SIN) indicates that the lytic replication of alphaviruses can be altered to suit the needs of the gene therapy application (Dryga, S.A. et al. (1997) Virology 228:74-83). The wide host range of alphaviruses will allow the introduction of SYNT into a variety of cell types. The specific transduction of a subset of cells in a population may require the sorting of cells prior to transduction. The methods of manipulating infectious cDNA clones of alphaviruses, performing alphavirus cDNA and RNA transfections, and performing alphavirus infections, are well known to those with ordinary skill in the art.

Oligonucleotides derived from the transcription initiation site, e.g., between about positions - 10 and +10 from the start site, may also be employed to inhibit gene expression. Similarly, inhibition can be achieved using triple helix base-pairing methodology. Triple helix pairing is useful because it causes inhibition of the ability of the double helix to open sufficiently for the binding of polymerases, transcription factors, or regulatory molecules. Recent therapeutic advances using triplex DNA have been described in the literature. (See, e.g., Gee, J.E. et al. (1994) in Huber, B.E. and B.I. Carr, Molecular and Immunologic Approaches, Futura Publishing, Mt. Kisco NY, pp. 163-177.) A complementary sequence or antisense molecule may also be designed to block translation of mRNA by preventing the transcript from binding to ribosomes.

Ribozymes, enzymatic RNA molecules, may also be used to catalyze the specific cleavage of RNA. The mechanism of ribozyme action involves sequence-specific hybridization of the ribozyme molecule to complementary target RNA, followed by endonucleolytic cleavage. For example, engineered hammerhead motif ribozyme molecules may specifically and efficiently catalyze endonucleolytic cleavage of sequences encoding SYNT.

Specific ribozyme cleavage sites within any potential RNA target are initially identified by scanning the target molecule for ribozyme cleavage sites, including the following sequences: GUA, GUU, and GUC. Once identified, short RNA sequences of between 15 and 20 ribonucleotides, corresponding to the region of the target gene containing the cleavage site, may be evaluated for secondary structural features which may render the oligonucleotide inoperable. The suitability of candidate targets may also be evaluated by testing accessibility to hybridization with complementary oligonucleotides using ribonuclease protection assays.

Complementary ribonucleic acid molecules and ribozymes of the invention may be prepared by any method known in the art for the synthesis of nucleic acid molecules. These include techniques for chemically synthesizing oligonucleotides such as solid phase phosphoramidite chemical synthesis. Alternatively, RNA molecules may be generated by in vitro and in vivo transcription of DNA sequences encoding SYNT. Such DNA sequences may be incorporated into a wide variety of vectors with suitable RNA polymerase promoters such as T7 or SP6. Alternatively, these cDNA constructs that synthesize complementary RNA, constitutively or inducibly, can be introduced into cell lines, cells, or tissues.

RNA molecules may be modified to increase intracellular stability and half-life. Possible modifications include, but are not limited to, the addition of flanking sequences at the 5' and/or 3' ends of the molecule, or the use of phosphorothioate or 2' O-methyl rather than phosphodiesterase linkages within the backbone of the molecule. This concept is inherent in the production of PNAs and can be extended in all of these molecules by the inclusion of nontraditional bases such as inosine, queosine, and wybutosine, as well as acetyl-, methyl-, thio-, and similarly modified forms of adenine, cytidine, guanine, thymine, and uridine which are not as easily recognized by endogenous endonucleases.

An additional embodiment of the invention encompasses a method for screening for a compound which is effective in altering expression of a polynucleotide encoding SYNT. Compounds which may be effective in altering expression of a specific polynucleotide may include, but are not limited to, oligonucleotides, antisense oligonucleotides, triple helix-forming oligonucleotides, transcription factors and other polypeptide transcriptional regulators, and non-macromolecular chemical entities which are capable of interacting with specific polynucleotide sequences. Effective compounds may alter polynucleotide expression by acting as either inhibitors or promoters of polynucleotide expression. Thus, in the treatment of disorders associated with increased SYNT expression or activity, a compound which specifically inhibits expression of the polynucleotide encoding SYNT may be therapeutically useful, and in the treament of disorders associated with decreased SYNT expression or activity, a compound which specifically promotes expression of the polynucleotide encoding SYNT may be therapeutically useful.

At least one, and up to a plurality, of test compounds may be screened for effectiveness in altering expression of a specific polynucleotide. A test compound may be obtained by any method commonly known in the art, including chemical modification of a compound known to be effective in altering polynucleotide expression: selection from an existing, commercially-available or proprietary library of naturally-occurring or non-natural chemical compounds; rational design of a compound based on chemical and/or structural properties of the target polynucleotide; and selection from a library of chemical compounds created combinatorially or randomly. A sample comprising a polynucleotide encoding SYNT is exposed to at least one test compound thus obtained. The sample may comprise, for example, an intact or permeabilized cell, or an in vitro cell-free or reconstituted biochemical system. Alterations in the expression of a polynucleotide encoding SYNT are assayed by any method commonly known in the art. Typically, the expression of a specific nucleotide is detected by hybridization with a probe having a nucleotide sequence complementary to the sequence of the polynucleotide encoding SYNT. The amount of hybridization may be quantified, thus forming the basis for a comparison of the expression of the polynucleotide both with and without exposure to one or more test compounds. Detection of a change in the expression of a polynucleotide exposed to a test compound indicates that the test compound is effective in altering the expression of the polynucleotide. A screen for a compound effective in altering expression of a specific polynucleotide can be carried out, for example, using a Schizosaccharomyces pombe gene expression system (Atkins, D. et al. (1999) U.S. Patent No. 5,932,435; Arndt, G.M. et al. (2000) Nucleic Acids Res. 28:E15) or a human cell line such as HeLa cell (Clarke, M.L. et al. (2000) Biochem. Biophys. Res. Commun. 268:8-13). A particular embodiment of the present invention involves screening a combinatorial library of oligonucleotides (such as deoxyribonucleotides, ribonucleotides, peptide nucleic acids, and modified oligonucleotides) for antisense activity against a specific polynucleotide sequence (Bruice, T.W. et al. (1997) U.S. Patent No. 5.686.242; Bruice, T.W. et al. (2000) U.S. Patent No. 6.022,691).

Many methods for introducing vectors into cells or tissues are available and equally suitable for use in vivo, in vitro, and ex vivo. For ex vivo therapy, vectors may be introduced into stem cells taken from the patient and clonally propagated for autologous transplant back into that same patient. Delivery by transfection, by liposome injections, or by polycationic amino polymers may be achieved using methods which are well known in the art. (See, e.g., Goldman, C.K. et al. (1997) Nat. Biotechnol. 15:462-466.)

Any of the therapeutic methods described above may be applied to any subject in need of such therapy, including, for example, mammals such as humans, dogs, cats, cows, horses, rabbits, and monkeys.

An additional embodiment of the invention relates to the administration of a pharmaceutical composition which generally comprises an active ingredient formulated with a pharmaceutically acceptable excipient. Excipients may include, for example, sugars, starches, celluloses, gums, and proteins. Various formulations are commonly known and are thoroughly discussed in the latest edition of Remington's Pharmaceutical Sciences (Maack Publishing, Easton PA).

Such pharmaceutical compositions may consist of SYNT, antibodies to SYNT, and mimetics, agonists, antagonists, or inhibitors of SYNT.

The pharmaceutical compositions utilized in this invention may be administered by any number of routes including, but not limited to, oral, intravenous, intramuscular, intra-arterial. intramedullary, intrathecal, intraventricular, pulmonary, transdermal, subcutaneous, intraperitoneal. intranasal, enteral, topical, sublingual, or rectal means.

Pharmaceutical compositions for pulmonary administration may be prepared in liquid or dry powder form. These compositions are generally aerosolized immediately prior to inhalation by the patient. In the case of small molecules (e.g. traditional low molecular weight organic drugs), aerosol delivery of fast-acting formulations is well-known in the art. In the case of macromolecules (e.g. larger peptides and proteins), recent developments in the field of pulmonary delivery via the alveolar region of the lung have enabled the practical delivery of drugs such as insulin to blood circulation (see. e.g., Patton, J.S. et al., U.S. Patent No. 5.997,848). Pulmonary delivery has the advantage of administration without needle injection, and obviates the need for potentially toxic penetration enhancers.

Pharmaceutical compositions suitable for use in the invention include compositions wherein the active ingredients are contained in an effective amount to achieve the intended purpose. The determination of an effective dose is well within the capability of those skilled in the art.

Specialized forms of pharmaceutical compositions may be prepared for direct intracellular delivery of macromolecules comprising SYNT or fragments thereof. For example, liposome preparations containing a cell-impermeable macromolecule may promote cell fusion and intracellular delivery of the macromolecule. Alternatively, SYNT or a fragment thereof may be joined to a short cationic N-terminal portion from the HIV Tat-1 protein. Fusion proteins thus generated have been found to transduce into the cells of all tissues, including the brain, in a mouse model system (Schwarze, S.R. et al. (1999) Science 285:1569-1572).

For any compound, the therapeutically effective dose can be estimated initially either in cell culture assays, e.g., of neoplastic cells, or in animal models such as mice, rats, rabbits, dogs, monkeys, or pigs. An animal model may also be used to determine the appropriate concentration range and route of administration. Such information can then be used to determine useful doses and routes for administration in humans.

A therapeutically effective dose refers to that amount of active ingredient, for example SYNT or fragments thereof, antibodies of SYNT, and agonists, antagonists or inhibitors of SYNT, which ameliorates the symptoms or condition. Therapeutic efficacy and toxicity may be determined by standard pharmaceutical procedures in cell cultures or with experimental animals, such as by calculating the $ED_{50}$ (the dose therapeutically effective in 50% of the population) or $LD_{50}$ (the dose lethal to 50% of the population) statistics. The dose ratio of toxic to therapeutic effects is the therapeutic index, which can be expressed as the $LD_{50}/ED_{50}$ ratio. Pharmaceutical compositions which exhibit large therapeutic indices are preferred. The data obtained from cell culture assays and animal studies are used to formulate a range of dosage for human use. The dosage contained in such compositions is preferably within a range of circulating concentrations that includes the $ED_{50}$ with little or no toxicity. The dosage varies within this range depending upon the dosage form employed, the sensitivity of the patient, and the route of administration.

The exact dosage will be determined by the practitioner, in light of factors related to the subject requiring treatment. Dosage and administration are adjusted to provide sufficient levels of the active moiety or to maintain the desired effect. Factors which may be taken into account include the severity of the disease state, the general health of the subject, the age, weight, and gender of the subject, time and frequency of administration, drug combinations), reaction sensitivities, and response to therapy. Long-acting pharmaceutical compositions may be administered every 3 to 4 days, every week, or biweekly depending on the half-life and clearance rate of the particular formulation.

Normal dosage amounts may vary from about 0.1 μg to 100,000 μg, up to a total dose of about 1 gram, depending upon the route of administration. Guidance as to particular dosages and methods of delivery is provided in the literature and generally available to practitioners in the art. Those skilled in the art will employ different formulations for nucleotides than for proteins or their inhibitors. Similarly, delivery of polynucleotides or polypeptides will be specific to particular cells, conditions, locations, etc.

**DIAGNOSTICS**

In another embodiment, antibodies which specifically bind SYNT may be used for the diagnosis of disorders characterized by expression of SYNT, or in assays to monitor patients being treated with SYNT or agonists, antagonists, or inhibitors of SYNT. Antibodies useful for diagnostic purposes may be prepared in the same manner as described above for therapeutics. Diagnostic assays for SYNT include methods which utilize the antibody and a label to detect SYNT in human body fluids or in extracts of cells or tissues. The antibodies may be used with or without modification, and may be labeled by covalent or non-covalent attachment of a reporter molecule. A wide variety of reporter molecules, several of which are described above, are known in the art and may be used.

A variety of protocols for measuring SYNT, including ELISAs, RIAs, and FACS, are known in the art and provide a basis for diagnosing altered or abnormal levels of SYNT expression. Normal or standard values for SYNT expression are established by combining body fluids or cell extracts taken from normal mammalian subjects, for example, human subjects, with antibody to SYNT under conditions suitable for complex formation. The amount of standard complex formation may be quantitated by various methods, such as photometric means. Quantities of SYNT expressed in subject, control, and disease samples from biopsied tissues are compared with the standard values. Deviation between standard and subject values establishes the parameters for diagnosing disease.

In another embodiment of the invention, the polynucleotides encoding SYNT may be used for diagnostic purposes. The polynucleotides which may be used include oligonucleotide sequences, complementary RNA and DNA molecules. and PNAs. The polynucleotides may be used to detect and quantify gene expression in biopsied tissues in which expression of SYNT may be correlated with disease. The diagnostic assay may be used to determine absence, presence, and excess expression of SYNT, and to monitor regulation of SYNT levels during therapeutic intervention.

In one aspect, hybridization with PCR probes which are capable of detecting polynucleotide sequences, including genomic sequences, encoding SYNT or closely related molecules may be used to identify nucleic acid sequences which encode SYNT. The specificity of the probe, whether it is made from a highly specific region, e.g., the 5'regulatory region, or from a less specific region, e.g., a conserved motif, and the stringency of the hybridization or amplification will determine whether the probe identifies only naturally occurring sequences encoding SYNT, allelic variants, or related sequences.

Probes may also be used for the detection of related sequences, and may have at least 50% sequence identity to any of the SYNT encoding sequences. The hybridization probes of the subject invention may be DNA or RNA and may be derived from the sequence of SEQ ID NO: 16-30 or from genomic sequences including promoters, enhancers, and introns of the SYNT gene.

Means for producing specific hybridization probes for DNAs encoding SYNT include the cloning of polynucleotide sequences encoding SYNT or SYNT derivatives into vectors for the production of mRNA probes. Such vectors are known in the art, are commercially available, and may be used to synthesize RNA probes in vitro by means of the addition of the appropriate RNA polymerases and the appropriate labeled nucleotides. Hybridization probes may be labeled by a variety of reporter groups, for example, by radionuclides such as $^{32}P$ or $^{35}S$, or by enzymatic labels, such as alkaline phosphatase coupled to the probe via avidin/biotin coupling systems, and the like.

Polynucleotide sequences encoding SYNT may be used for the diagnosis of disorders associated with expression of SYNT. Examples of such disorders include, but are not limited to, an immune disorder such as inflammation, actinic keratosis, acquired immunodeficiency syndrome (AIDS), Addison's disease, adult respiratory distress syndrome, allergies, ankylosing spondylitis, amyloidosis, anemia, arteriosclerosis, asthma, atherosclerosis, autoimmune hemolytic anemia, autoimmune thyroiditis, bronchitis, bursitis, cholecystitis, cirrhosis, contact dermatitis. Crohn's disease, atopic dermatitis, dermatomyositis, diabetes mellitus, emphysema, erythroblastosis fetalis, erythema nodosum, atrophic gastritis, glomerulonephritis. Goodpasture's syndrome, gout, Graves' disease, Hashimoto's thyroiditis, paroxysmal nocturnal hemoglobinuria, hepatitis, hypereosinophilia, irritable bowel syndrome, episodic lymphopenia with lymphocytotoxins, mixed connective tissue disease (MCTD), multiple sclerosis, myasthenia gravis, myocardial or pericardial inflammation, myelofibrosis, osteoarthritis, osteoporosis, pancreatitis, polycythemia vera, polymyositis, psoriasis. Reiter's syndrome, rheumatoid arthritis, scleroderma, Sjögren's syndrome, systemic anaphylaxis, systemic lupus erythematosus, systemic sclerosis, primary thrombocythemia, thrombocytopenic purpura, ulcerative colitis, uveitis, Werner syndrome, complications of cancer, hemodialysis, and extracorporeal circulation, trauma, viral. bacterial, fungal, parasitic, protozoal, and helminthic infections, and hematopoietic cancer including lymphoma, leukemia, and myeloma; a neuronal disorder, such as akathesia, Alzheimer's disease, amnesia, amyotrophic lateral sclerosis, bipolar disorder, catatonia, cerebral neoplasms, dementia, depression, diabetic neuropathy, Down's syndrome, tardive dyskinesia, dystonias, epilepsy, Huntington's disease, peripheral neuropathy, multiple sclerosis, neurofibromatosis, Parkinson's disease, paranoid psychoses, postherpetic neuralgia, schizophrenia, and Tourette's disorder; a reproductive disorder, such as a disorder of prolactin production, infertility, including tuba) disease, ovulatory defects, and endometriosis, a disruption of the estrous cycle, a disruption of the menstrual cycle, polycystic ovary syndrome, ovarian hyperstimulation syndrome, an endometrial or ovarian tumor, a uterine fibroid, autoimmune disorders, an ectopic pregnancy, and teratogenesis; cancer of the breast, fibrocystic breast disease, and galactorrhea; a disruption of spermatogenesis, abnormal sperm physiology, cancer of the testis, cancer of the prostate, benign prostatic hyperplasia, prostatitis, Peyronie's disease, impotence, carcinoma of the male breast, and gynecomastia; and a cell proliferative disorder, such as actinic keratosis, arteriosclerosis, atherosclerosis, bursitis, cirrhosis, hepatitis, mixed connective tissue disease (MCTD), myelofibrosis, paroxysmal nocturnal hemoglobinuria, polycythemia vera, psoriasis, primary thrombocythemia, and cancers including adenocarcinoma, leukemia, lymphoma, melanoma, myeloma, sarcoma, teratocarcinoma, and, in particular, a cancer of the adrenal gland, bladder, bone, bone marrow, brain, breast, cervix, gall bladder, ganglia, gastrointestinal tract, heart, kidney, liver, lung, muscle, ovary, pancreas, parathyroid, penis, prostate, salivary glands, skin, spleen, testis, thymus, thyroid, and uterus. The polynucleotide sequences encoding SYNT may be used in Southern or northern analysis, dot blot, or other membrane-based technologies; in PCR technologies; in dipstick, pin, and multiformat ELISA-like assays; and in microarrays utilizing fluids or tissues from patients to detect altered SYNT expression. Such qualitative or quantitative methods are well known in the art.

In a particular aspect, the nucleotide sequences encoding SYNT may be useful in assays that detect the presence of associated disorders, particularly those mentioned above. The nucleotide sequences encoding SYNT may be labeled by standard methods and added to a fluid or tissue sample from a patient under conditions suitable for the formation of hybridization complexes. After a suitable incubation period, the sample is washed and the signal is quantified and compared with a standard value. If the amount of signal in the patient sample is significantly altered in comparison to a control sample then the presence of altered levels of nucleotide sequences encoding SYNT in the sample indicates the presence of the associated disorder. Such assays may also be used to evaluate the efficacy of a particular therapeutic treatment regimen in animal studies, in clinical trials, or to monitor the treatment of an individual patient.

In order to provide a basis for the diagnosis of a disorder associated with expression of SYNT, a normal or standard profile for expression is established. This may be accomplished by combining body fluids or cell extracts taken from normal subjects, either animal or human, with a sequence, or a fragment thereof, encoding SYNT, under conditions suitable for hybridization or amplification. Standard hybridization may be quantified by comparing the values obtained from normal subjects with values from an experiment in which a known amount of a substantially purified polynucleotide is used. Standard values obtained in this manner may be compared with values obtained from samples from patients who are symptomatic for a disorder. Deviation from standard values is used to establish the presence of a disorder.

Once the presence of a disorder is established and a treatment protocol is initiated, hybridization assays may be repeated on a regular basis to determine if the level of expression in the patient begins to approximate that which is observed in the normal subject. The results obtained from successive assays may be used to show the efficacy of treatment over a period ranging from several days to months.

With respect to cancer, the presence of an abnormal amount of transcript (either under- or overexpressed) in biopsied tissue from an individual may indicate a predisposition for the development of the disease, or may provide a means for detecting the disease prior to the appearance of actual clinical symptoms. A more definitive diagnosis of this type may allow health professionals to employ preventative measures or aggressive treatment earlier thereby preventing the development or further progression of the cancer.

Additional diagnostic uses for oligonucleotides designed from the sequences encoding SYNT may involve the use of PCR. These oligomers may be chemically synthesized, generated enzymatically, or produced in vitro. Oligomers will preferably contain a fragment of a polynucleotide encoding SYNT, or a fragment of a polynucleotide complementary to the polynucleotide encoding SYNT, and will be employed under optimized conditions for identification of a specific gene or condition. Oligomers may also be employed under less stringent conditions for detection or quantification of closely related DNA or RNA sequences.

In a particular aspect, oligonucleotide primers derived from the polynucleotide sequences encoding SYNT may be used to detect single nucleotide polymorphisms (SNPs). SNPs are substitutions, insertions and deletions that are a frequent cause of inherited or acquired genetic disease in humans. Methods of SNP detection include, but are not limited to, single-stranded conformation polymorphism (SSCP) and fluorescent SSCP (fSSCP) methods. In SSCP, oligonucleotide primers derived from the polynucleotide sequences encoding SYNT are used to amplify DNA using-the polymerase chain reaction (PCR). The DNA may be derived, for example, from diseased or normal tissue, biopsy samples, bodily fluids, and the like. SNPs in the DNA cause differences in the secondary and tertiary structures of PCR products in single-stranded form, and these differences are detectable using gel electrophoresis in non-denaturing gels. In fSCCP, the oligonucleotide primers are fluorescently labeled, which allows detection of the amplimers in high-throughput equipment such as DNA sequencing machines. Additionally, sequence database analysis methods, termed in silico SNP (isSNP), are capable of identifying polymorphisms by comparing the sequence of individual overlapping DNA fragments which assemble into a common consensus sequence. These computer-based methods filter out sequence variations due to laboratory preparation of DNA and sequencing errors using statistical models and automated analyses of DNA sequence chromatograms. In the alternative, SNPs may be detected and characterized by mass spectrometry using, for example, the high throughput MASSARRAY system (Sequenom, Inc., San Diego CA).

Methods which may also be used to quantify the expression of SYNT include radiolabeling or biotinylating nucleotides, coamplification of a control nucleic acid, and interpolating results from standard curves. (See, e.g., Melby, P.C. et al. (1993) J. Immunol. Methods 159:235-244: Duplaa, C. et al. (1993) Anal. Biochem. 212:229-236.) The speed of quantitation of multiple samples may be accelerated by running the assay in a high-throughput format where the oligomer or polynucleotide of interest is presented in various dilutions and a spectrophotometric or colorimetric response gives rapid quantitation.

In further embodiments, oligonucleotides or longer fragments derived from any of the polynucleotide sequences described herein may be used as elements on a microarray. The microarray can be used in transcript imaging techniques which monitor the relative expression levels of large numbers of genes simultaneously as described in Seilhamer, J.J. et al., "Comparative Gene Transcript Analysis," U.S. Patent No. 5,840,484, incorporated herein by reference. The microarray may also be used to identify genetic variants, mutations, and polymorphisms. This information may be used to determine gene function, to understand the genetic basis of a disorder, to diagnose a disorder, to monitor progression/regression of disease as a function of gene expression, and to develop and monitor the activities of therapeutic agents in the treatment of disease. In particular, this information may be used to develop a pharmacogenomic profile of a patient in order to select the most appropriate and effective treatment regimen for that patient. For example, therapeutic agents which are highly effective and display the fewest side effects may be selected for a patient based on his/her pharmacogenomic profile.

In another embodiment, antibodies specific for SYNT, or SYNT or fragments thereof may be used as elements on a microarray. The microarray may be used to monitor or measure protein-protein interactions, drug-target interactions, and gene expression profiles, as described above.

A particular embodiment relates to the use of the polynucleotides of the present invention to generate a transcript image of a tissue or cell type. A transcript image represents the global pattern of gene expression by a particular tissue or cell type. Global gene expression patterns are analyzed by quantifying the number of expressed genes and their relative abundance under given conditions and at a given time. (See Seilhamer et al., "Comparative Gene Transcript Analysis." U.S. Patent Number 5,840,484, expressly incorporated by reference herein.) Thus a transcript image may be generated by hybridizing the polynucleotides of the present invention or their complements to the totality of transcripts or reverse transcripts of a particular tissue or cell type. In one embodiment, the hybridization takes place in high-throughput format, wherein the polynucleotides of the present invention or their complements comprise a subset of a plurality of elements on a microarray. The resultant transcript image would provide a profile of gene activity.

Transcript images may be generated using transcripts isolated from tissues, cell lines, biopsies, or other biological samples. The transcript image may thus reflect gene expression in vivo, as in the case of a tissue or biopsy sample, or in vitro, as in the case of a cell line.

Transcript images which profile the expression of the polynucleotides of the present invention may also be used in conjunction with in vitro model systems and preclinical evaluation of pharmaceuticals. as well as toxicological testing of industrial and naturally-occurring environmental compounds. All compounds induce characteristic gene expression patterns, frequently termed molecular fingerprints or toxicant signatures, which are indicative of mechanisms of action and toxicity (Nuwaysir, E.F. et al. (1999) Mol. Carcinog. 24:153-159; Steiner, S. and N.L. Anderson (2000) Toxicol. Lett. 112-113:467-471, expressly incorporated by reference herein). If a test compound has a signature similar to that of a compound with known toxicity, it is likely to share those toxic properties. These fingerprints or signatures are most useful and refined when they contain expression information from a large number of genes and gene families. Ideally, a genome-wide measurement of expression provides the highest quality signature. Even genes whose expression is not altered by any tested compounds are important as well, as the levels of expression of these genes are used to normalize the rest of the expression data. The normalization procedure is useful for comparison of expression data after treatment with different compounds. While the assignment of gene function to elements of a toxicant signature aids in interpretation of toxicity mechanisms, knowledge of gene function is not necessary for the statistical matching of signatures which leads to prediction of toxicity. (See, for example, Press Release 00-02 from the National Institute of Environmental Health Sciences, released February 29, 2000, available at http://www.niehs.nih.gov/oc/news/toxchip.htm.) Therefore, it is important and desirable in toxicological screening using toxicant signatures to include all expressed gene sequences.

In one embodiment, the toxicity of a test compound is assessed by treating a biological sample containing nucleic acids with the test compound. Nucleic acids that are expressed in the treated biological sample are hybridized with one or more probes specific to the polynucleotides of the present invention, so that transcript levels corresponding to the polynucleotides of the present invention may be quantified. The transcript levels in the treated biological sample are compared with levels in an untreated biological sample. Differences in the transcript levels between the two samples are indicative of a toxic response caused by the test compound in the treated sample.

Another particular embodiment relates to the use of the polypeptide sequences of the present invention to analyze the proteome of a tissue or cell type. The term proteome refers to the global pattern of protein expression in a particular tissue or cell type. Each protein component of a proteome can be subjected individually to further analysis. Proteome expression patterns, or profiles, are analyzed by quantifying the number of expressed proteins and their relative abundance under given conditions and at a given time. A profile of a cell's proteome may thus be generated by separating and analyzing the polypeptides of a particular tissue or cell type. In one embodiment, the separation is achieved using two-dimensional gel electrophoresis, in which proteins from a sample are separated by isoelectric focusing in the first dimension, and then according to molecular weight by sodium dodecyl sulfate slab gel electrophoresis in the second dimension (Steiner and Anderson, supra). The proteins are visualized in the gel as discrete and uniquely positioned spots, typically by staining the gel with an agent such as Coomassie Blue or silver or fluorescent stains. The optical density of each protein spot is generally proportional to the level of the protein in the sample. The optical densities of equivalently positioned protein spots from different samples, for example, from biological samples either treated or untreated with a test compound or therapeutic agent, are compared to identify any changes in protein spot density related to the treatment. The proteins in the spots are partially sequenced using, for example, standard methods employing chemical or enzymatic cleavage followed by mass spectrometry. The identity of the protein in a spot may be determined by comparing its partial sequence, preferably of at least 5 contiguous amino acid residues, to the polypeptide sequences of the present invention. In some cases, further sequence data may be obtained for definitive protein identification.

A proteomic profile may also be generated using antibodies specific for SYNT to quantify the levels of SYNT expression. In one embodiment, the antibodies are used as elements on a microarray, and protein expression levels are quantified by exposing the microarray to the sample and detecting the levels of protein bound to each array element (Lueking, A. et al. (1999) Anal. Biochem. 270:103-111; Mendoze, L.G. et at. (1999) Biotechniques 27:778-788). Detection may be performed by a variety of methods known in the art, for example, by reacting the proteins in the sample with a thiol- or amino-reactive fluorescent compound and detecting the amount of fluorescence bound at each array element.

Toxicant signatures at the proteome level are also useful for toxicological screening, and should be analyzed in parallel with toxicant signatures at the transcript level. There is a poor correlation between transcript and protein abundances for some proteins in some tissues (Anderson. N.L. and J. Seilhamer (1997) Electrophoresis 18:533-537), so proteome toxicant signatures may be useful in the analysis of compounds which do not significantly affect the transcript image, but which alter the proteomic profile. In addition, the analysis of transcripts in body fluids is difficult, due to rapid degradation of mRNA, so proteomic profiling may be more reliable and informative in such cases.

In another embodiment, the toxicity of a test compound is assessed by treating a biological sample containing proteins with the test compound. Proteins that are expressed in the treated biological sample are separated so that the amount of each protein can be quantified. The amount of each protein is compared to the amount of the corresponding protein in an untreated biological sample. A difference in the amount of protein between the two samples is indicative of a toxic response to the test compound in the treated sample. Individual proteins are identified by sequencing the amino acid residues of the individual proteins and comparing these partial sequences to the polypeptides of the present invention.

In another embodiment, the toxicity of a test compound is assessed by treating a biological sample containing proteins with the test compound. Proteins from the biological sample are incubated with antibodies specific to the polypeptides of the present invention. The amount of protein recognized by the antibodies is quantified. The amount of protein in the treated biological sample is compared with the amount in an untreated biological sample. A difference in the amount of protein between the two samples is indicative of a toxic response to the test compound in the treated sample.

Microarrays may be prepared, used, and analyzed using methods known in the art. (See, e.g., Brennan, T. M. et al. (1995) U.S. Patent No. 5,474,796; Schena, M. et al. (1996) Proc. Natl. Acad. Sci. USA 93:10614-10619; Baldeschweiler et al. (1995) PCT application WO95/251116; Shalon. D. et al. (1995) PCT application WO95/35505; Heller, R.A. et al. (1997) Proc. Natl. Acad. Sci. USA 94:2150-2155; and Heller, M.J. et al. (1997) U.S. Patent No. 5,605,662.) Various types of microarrays are well known and thoroughly described in DNA Microarrays: A Practical Approach, M. Schena, ed. (1999) Oxford University Press, London, hereby expressly incorporated by reference.

In another embodiment of the invention, nucleic acid sequences encoding SYNT may be used to generate hybridization probes useful in mapping the naturally occurring genomic sequence. Either coding or noncoding sequences may be used, and in some instances, noncoding sequences may be preferable over coding sequences. For example, conservation of a coding sequence among members of a multi-gene family may potentially cause undesired cross hybridization during chromosomal mapping. The sequences may be mapped to a particular chromosome, to a specific region of a chromosome, or to artificial chromosome constructions, e.g., human artificial chromosomes (HACs), yeast artificial chromosomes (YACs), bacterial artificial chromosomes (BACs), bacterial P1 constructions. or single chromosome cDNA libraries. (See, e.g., Harrington, J.J. et al. (1997) Nat. Genet. 15:345-355: Price. C.M. (1993) Blood Rev. 7:127-134: and Trask. B.J. (1991) Trends Genet. 7:149-154.) Once mapped, the nucleic acid sequences of the invention may be used to develop genetic linkage maps, for example, which correlate the inheritance of a disease state with the inheritance of a particular chromosome region or restriction fragment length polymorphism (RFLP). (See, e.g., Lander, E.S. and D. Botstein ( 1986) Proc. Natl, Acad. Sci. USA 83:7353-7357.)

Fluorescent in situ hybridization (FISH) may be correlated with other physical and genetic map data. (See, e.g., Heinz-Ulrich, et al, (1995) in Meyers, supra, pp. 965-968.) Examples of genetic map data can be found in various scientific journals or at the Online Mendelian Inheritance in Man (OMIM) World Wide Web site. Correlation between the location of the gene encoding SYNT on a physical map and a specific disorder, or a predisposition to a specific disorder, may help define the region of DNA associated with that disorder and thus may further positional cloning efforts.

In situ hybridization of chromosomal preparations and physical mapping techniques, such as linkage analysis using established chromosomal markers, may be used for extending genetic maps. Often the placement of a gene on the chromosome of another mammalian species, such as mouse, may reveal associated markers even if the exact chromosomal locus is not known. This information is valuable to investigators searching for disease genes using positional cloning or other gene discovery techniques. Once the gene or genes responsible for a disease or syndrome have been crudely localized by genetic linkage to a particular genomic region, e.g., ataxia-telangiectasia to 11q22-23. any sequences mapping to that area may represent associated or regulatory genes for further investigation. (See, e. g., Gatti, R.A. et al. (1988) Nature 336:577-580.) The nucleotide sequence of the instant invention may also be used to detect differences in the chromosomal location due to translocation, inversion, etc., among normal, carrier, or affected individuals.

In another embodiment of the invention, SYNT, its catalytic or immunogenic fragments, or oligopeptides thereof can be used for screening libraries of compounds in any of a variety of drug screening techniques. The fragment employed in such screening may be free in solution, affixed to a solid support, borne on a cell surface, or located intracellularly. The formation of binding complexes between SYNT and the agent being tested may be measured.

Another technique for drug screening provides for high throughput screening of compounds having suitable binding affinity to the protein of interest. (See, e.g., Geysen, et al. (1984) PCT application WO84/03564.) In this method, large numbers of different small test compounds are synthesized on a solid substrate. The test compounds are reacted with SYNT, or fragments thereof. and washed. Bound SYNT is then detected by methods well known in the art. Purified SYNT can also be coated directly onto plates for use in the aforementioned drug screening techniques. Alternatively, non-neutralizing antibodies can be used to capture the peptide and immobilize it on a solid support.

In another embodiment, one may use competitive drug screening assays in which neutralizing antibodies capable-of binding SYNT specifically compete with a test compound for binding SYNT. In this manner, antibodies can be used to detect the presence of any peptide which shares one or more antigenic determinants with SYNT.

In additional embodiments. the nucleotide sequences which encode SYNT may be used in any molecular biology techniques that have yet to be developed, provided the new techniques rely on properties of nucleotide sequences that are currently known, including, but not limited to, such properties as the triplet genetic code and specific base pair interactions.

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever.

The disclosures of all patents, applications, and publications mentioned above and below, in particular U.S. Ser. No. 60/144,992 and U.S. Ser. No. 60/168,858 are hereby expressly incorporated by reference.

## EXAMPLES

### I. Construction of cDNA Libraries

RNA was purchased from Clontech or isolated from tissues described in Table 4. Some tissues were homogenized and lysed in guanidinium isothiocyanate, while others were homogenized and lysed in phenol or in a suitable mixture of denaturants, such as TRIZOL (Life Technologies), a monophasic solution of phenol and guanidine isothiocyanate. The resulting lysates were centrifuged over CsCI cushions or extracted with chloroform. RNA was precipitated from the lysates with either isopropanol or sodium acetate and ethanol, or by other routine methods.

Phenol extraction and precipitation of RNA were repeated as necessary to increase RNA purity. In some cases, RNA was treated with DNase. For most libraries, poly(A+) RNA was isolated using oligo d(T)-coupled paramagnetic particles (Promega), OLIGOTEX latex particles (QIAGEN, Chatsworth CA), or an OLIGOTEX mRNA purification kit (QIAGEN). Alternatively, RNA was isolated directly from tissue lysates using other RNA isolation kits, e.g., the POLY(A)PURE mRNA purification kit (Ambion, Austin TX).

In some cases, Stratagene was provided with RNA and constructed the corresponding cDNA libraries. Otherwise, cDNA was synthesized and cDNA libraries were constructed with the UNIZAP vector system (Stratagene) or SUPERSCRIPT plasmid system (Life Technologies), using the recommended procedures or similar methods known in the art. (See, e.g., Ausubel, 1997, supra, units 5.1-6.6.) Reverse transcription was initiated using oligo d(T) or random primers. Synthetic oligonucleotide adapters were ligated to double stranded cDNA. and the cDNA was digested with the appropriate restriction enzyme or enzymes. For most libraries, the cDNA was size-selected (300-1000 bp) using SEPHACRYL S1000, SEPHAROSE CL2B, or SEPHAROSE CL4B column chromatography (Amersham Pharmacia Biotech) or preparative agarose gel electrophoresis. cDNAs were ligated into compatible restriction enzyme sites of the polylinker of a suitable plasmid, e.g., PBLUESCRIPT plasmid (Stratagene), PSPORT1 plasmid (Life Technologies), pcDNA2.1 plasmid (Invitrogen, Carlsbad CA), or pINCY plasmid (Incyte Genomics, Palo Alto CA). Recombinant plasmids were transformed into competent E. coli cells including XL1-Blue, XL1-BlueMRF, or SOLR from Stratagene or DH5$\alpha$. DH10B, or ElectroMAX DH10B from Life Technologies.

## II. Isolation of cDNA Clones

Plasmids obtained as described in Example I were recovered from host cells by in vivo excision using the UNIZAP vector system (Stratagene) or by cell lysis. Plasmids were purified using at least one of the following: a Magic or WIZARD Minipreps DNA purification system (Promega): an AGTC Miniprep purification kit (Edge Biosystems. Gaithersburg MD); and QIAWELL 8 Plasmid, QIAWELL 8 Plus Plasmid, QIAWELL 8 Ultra Plasmid purification systems or the R.E.A.L. PREP 96 plasmid purification kit from QIAGEN. Following precipitation, plasmids were resuspended in 0.1 ml of distilled water and stored, with or without lyophilization, at 4°C.

Alternatively, plasmid DNA was amplified from host cell lysates using direct link PCR in a high-throughput format (Rao, V.B. (1994) Anal. Biochem. 216:1-14). Host cell lysis and thermal cycling steps were carried out in a single reaction mixture. Samples were processed and stored in 384-well plates, and the concentration of amplified plasmid DNA was quantified fluorometrically using PICOGREEN dye (Molecular Probes, Eugene OR) and a FLUOROSKAN II fluorescence scanner (Labsystems Oy, Helsinki, Finland).

## III. Sequencing and Analysis

Incyte cDNA recovered in plasmids as described in Example II were sequenced as follows. Sequencing reactions were processed using standard methods or high-throughput instrumentation such as the ABI CATALYST 800 (PE Biosystems) thermal cycler or the PTC-200 thermal cycler (MJ Research) in conjunction with the HYDRA microdispenser (Robbins Scientific) or the MICROLAB 2200 (Hamilton) liquid transfer system. cDNA sequencing reactions were prepared using reagents provided by Amersham Pharmacia Biotech or supplied in ABI sequencing kits such as the ABI PRISM BIGDYE Terminator cycle sequencing ready reaction kit (PE Biosystems). Electrophoretic separation of cDNA sequencing reactions and detection of labeled polynucleotides were carried out using the MEGABACE 1000 DNA sequencing system (Molecular Dynamics); the ABI PRISM 373 or 377 sequencing system (PE Biosystems) in conjunction with standard ABI protocols and base calling software; or other sequence analysis systems known in the art. Reading frames within the cDNA sequences were identified using standard methods (reviewed in Ausubel. 1997, supra, unit 7.7). Some of the cDNA sequences were selected for extension using the techniques disclosed in Example VI.

The polynucleotide sequences derived from cDNA sequencing were assembled and analyzed using a combination of software programs which utilize algorithms well known to those skilled in the art. Table 5 summarizes the tools, programs, and algorithms used and provides applicable descriptions, references, and threshold parameters. The first column of Table 5 shows the tools. programs, and algorithms used, the second column provides brief descriptions thereof, the third column presents appropriate references, all of which are incorporated by reference herein in their entirety, and the fourth column presents, where applicable, the scores, probability values. and other parameters used to evaluate the strength of a match between two sequences (the higher the score, the greater the homology between two sequences). Sequences were analyzed using MACDNASIS PRO software (Hitachi Software Engineering, South San Francisco CA) and LASERGENE software (DNASTAR). Polynucleotide and polypeptide sequence alignments were generated using the default parameters specified by the clustal algorithm as incorporated into the MEGALIGN multisequence alignment program (DNASTAR), which also calculates the percent identity between aligned sequences.

The polynucleotide sequences were validated by removing vector, linker, and polyA sequences and by masking ambiguous bases, using algorithms and programs based on BLAST, dynamic programing, and dinucleotide nearest neighbor analysis. The sequences were then queried against a selection of public databases such as the GenBank primate, rodent, mammalian, vertebrate, and eukaryote databases, and BLOCKS, PRINTS, DOMO, PRODOM, and PFAM to acquire annotation using programs based on BLAST, PASTA, and BLIMPS. The sequences were assembled into full length polynucleotide sequences using programs based on Phred, Phrap, and Consed, and were screened for open reading frames using programs based on GeneMark, BLAST, and PASTA. The full length polynucleotide sequences were translated to derive the corresponding full length amino acid sequences, and these full length sequences were subsequently analyzed by querying against databases such as the GenBank databases (described above), SwissProt, BLOCKS, PRINTS, DOMO, PRODOM, Prosite, and Hidden Markov Model (HMM)-based protein family databases such as PFAM. HMM is a probabilistic approach which analyzes consensus primary structures of gene families. (See, e.g., Eddy, S.R. (1996) Curr. Opin. Struct. Biol. 6:361-365.)

The programs described above for the assembly and analysis of full length polynucleotide and amino acid sequences were also used to identify polynucleotide sequence fragments from SEQ ID NO: 16-30. Fragments from about 20 to about 4000 nucleotides which are useful in hybridization and amplification technologies were described in The Invention section above.

## IV. Analysis of Polynucleotide Expression

Northern analysis is a laboratory technique used to detect the presence of a transcript of a gene and involves the hybridization of a labeled nucleotide sequence to a membrane on which RNAs from a particular cell type or tissue have been bound. (See. e.g., Sambrook, supra, ch. 7: Ausubel. 1995, supra, ch. 4 and 16.)

Analogous computer techniques applying BLAST were used to search for identical or related molecules in cDNA databases such as GenBank or LIFESEQ (Incyte Genomics). This analysis is much faster than multiple membrane-based hybridizations. In addition, the sensitivity of the computer search can be modified to determine whether any particular match is categorized as exact or similar. The basis of the search is the product score, which is defined as:

$$\frac{\text{BLAST Score x Percent Identity}}{\text{5 x minimum \{length(Seq. 1). length(Seq. 2)\}}}$$

The product score takes into account both the degree of similarity between two sequences and the length of the sequence match. The product score is a normalized value between 0 and 100, and is calculated as follows: the BLAST score is multiplied by the percent nucleotide identity and the product is divided by (5 times the length of the shorter of the two sequences). The BLAST score is calculated by assigning a score of +5 for every base that matches in a high-scoring segment pair (HSP), and -4 for every mismatch. Two sequences may share more than one HSP (separated by gaps). If there is more than one HSP, then the pair with the highest BLAST score is used to calculate the product score. The product score represents a balance between fractional overlap and quality in a BLAST alignment. For example, a product score of 100 is produced only for 100% identity over the entire length of the shorter of the two sequences being compared. A product score of 70 is produced either by 100% identity and 70% overlap at one end, or by 88% identity and 100% overlap at the other. A product score of 50 is produced either by 100% identity and 50% overlap at one end, or 79% identity and 100% overlap.

The results of northern analyses are reported as a percentage distribution of libraries in which the transcript encoding SYNT occurred. Analysis involved the categorization of cDNA libraries by organ/tissue and disease. The organ/tissue categories included cardiovascular, dermatologic, developmental, endocrine, gastrointestinal, hematopoietic/immune, musculoskeletal, nervous, reproductive, and urologic. The disease/condition categories included cancer. inflammation, trauma, cell proliferation, neurological, and pooled. For each category, the number of libraries expressing the sequence of interest was counted and divided by the total number of libraries across all categories. Percentage values of tissue-specific and disease- or condition-specific expression are reported in Table 3.

## V. Extension of SYNT Encoding Polynucleotides

The full length nucleic acid sequences of SEQ ID NO:16-30 were produced by extension of an appropriate fragment of the full length molecule using oligonucleotide primers designed from this fragment. One primer was synthesized to initiate 5' extension of the known fragment, and the other primer, to initiate 3'extension of the known fragment. The initial primers were designed using OLIGO 4.06 software (National Biosciences), or another appropriate program, to be about 22 to 30 nucleotides in length, to have a GC content of about 50% or more, and to anneal to the target sequence at temperatures of about 68°C to about 72°C. Any stretch of nucleotides which would result in hairpin structures and primer-primer dimerizations was avoided.

Selected human cDNA libraries were used to extend the sequence. If more than one extension was necessary or desired, additional or nested sets of primers were designed.

High fidelity amplification was obtained by PCR using methods well known in the art. PCR was performed in 96-well plates using the PTC-200 thermal cycler (MJ Research, Inc.). The reaction mix contained DNA template, 200 nmol of each primer, reaction buffer containing $Mg^{2+}$, $(NH_4)_2SO_4$, and β-mercaptoethanol. Taq DNA polymerase (Amersham Pharmacia Biotech), ELONGASE enzyme (Life Technologies), and Pfu DNA polymerase (Stratagene), with the following parameters for primer pair PCI A and PCI B: Step 1: 94°C. 3 min; Step 2: 94°C, 15 sec: Step 3: 60°C, 1 min: Step 4: 68°C, 2 min: Step 5: Steps 2, 3, and 4 repeated 20 times; Step 6: 68°C. 5 min: Step 7: storage at 4°C. In the 5 alternative, the parameters for primer pair T7 and SK+ were as follows: Step 1: 94°C, 3 min: Step 2: 94°C. 15 sec; Step 3: 57°C, 1 min: Step 4: 68°C. 2 min; Step 5: Steps 2, 3, and 4 repeated 20 times; Step 6: 68°C. 5 min; Step 7: storage at 4°C.

The concentration of DNA in each well was determined by dispensing 100 µl PICOGREEN quantitation reagent (0.25% (v/v) PICOGREEN; Molecular Probes, Eugene OR) dissolved in 1X TE and 0.5 µl of undiluted PCR product into each well of an opaque fluorimeter plate (Coming Costar, Acton MA), allowing the DNA to bind to the reagent. The plate was scanned in a Fluoroskan II (Labsystems Oy, Helsinki, Finland) to measure the fluorescence of the sample and to quantify the concentration of DNA. A5 µl to 10 µl aliquot of the reaction mixture was analyzed by electrophoresis on a 1 % agarose mini-gel to determine which reactions were successful in extending the sequence.

The extended nucleotides were desalted and concentrated, transferred to 384-well plates. digested with CviJI cholera virus endonuclease (Molecular Biology Research. Madison WI), and sonicated or sheared prior to religation into pUC 18 vector (Amersham Pharmacia Biotech). For shotgun sequencing, the digested nucleotides were separated on low concentration (0.6 to 0.8%) agarose gels, fragments were excised, and agar digested with Agar ACE (Promega). Extended clones were religated using T4 ligase (New England Biolabs. Beverly MA) into pUC 18 vector (Amersham Pharmacia Biotech), treated with Pfu DNA polymerase (Stratagene) to fill-in restriction site overhangs, and transfected into competent E. coli cells. Transformed cells were selected on antibiotic-containing media, and individual colonies were picked and cultured overnight at 37 °C in 384-well plates in LB/2x carb liquid media.

The cells were lysed, and DNA was amplified by PCR using Taq DNA polymerase (Amersham Pharmacia Biotech) and Pfu DNA polymerase (Stratagene) with the following parameters: Step 1: 94°C. 3 min: Step 2: 94°C. 15 sec: Step 3: 60°C, 1 min: Step 4: 72°C, 2 min: Step 5: steps 2. 3, and 4 repeated 29 times: Step 6: 73°C, 5 min: Step 7: storage at 4°C. DNA was quantified by PICOGREEN reagent (Molecular Probes) as described above. Samples with low DNA recoveries were reamplified using the same conditions as described above. Samples were diluted with 20% dimethysulfoxide (1:2, v/v), and sequenced using DYENAMIC energy transfer sequencing primers and the DYENAMIC DIRECT kit (Amersham Pharmacia Biotech) or the ABI PRISM BIGDYE Terminator cycle sequencing ready reaction kit (PE Biosystems).

In like manner, the polynucleotide sequences of SEQ ID NO: 16-30 are used to obtain 5' regulatory sequences using the procedure above, along with oligonucleotides designed for such extension, and an appropriate genomic library.

### V. Chromosomal Mapping of SNYT Encoding Polynucleotides

The cDNA sequences which were used to assemble SEQ ID NO:16-30 were compared with sequences from the Incyte LIFESEQ database and public domain databases using BLAST and other implementations of the Smith-Waterman algorithm. Sequences from these databases that matched SEQ ID NO:16-30 were assembled into clusters of contiguous and overlapping sequences using assembly algorithms such as Phrap (Table 5). Radiation hybrid and genetic mapping data available from public resources such as the Stanford Human Genome Center (SHGC), Whitehead Institute for Genome Research (WIGR), and Généthon were used to determine if any of the clustered sequences had been previously mapped. Inclusion of a mapped sequence in a cluster resulted in the assignment of all sequences of that cluster, including its particular SEQ ID NO:, to that map location.

The genetic map locations of SEQ ID NO:16, SEQ ID NO:17, SEQ ID NO:18. SEQ ID NO:21, SEQ ID NO: 24. SEQ ID NO:26, SEQ ID NO:27, SEQ ID NO:28. SEQ ID NO:29, and SEQ ID NO:30 are described in The Invention as ranges, or intervals, of human chromosomes. More than one map location is reported for SEQ ID NO:28 and SEQ ID NO:29, indicating that previously mapped sequences having similarity, but not complete identity, to SEQ ID NO:28 and SEQ ID NO:29 were assembled into their respective clusters. The map position of an interval, in centiMorgans, is measured relative to the terminus of the chromosome's p-arm. (The centiMorgan (cM) is a unit of measurement based on recombination frequencies between chromosomal markers. On average, 1 cM is roughly equivalent to 1 megabase (Mb) of DNA in humans, although this can vary widely due to hot and cold spots of recombination.) The cM distances are based on genetic markers mapped by Généthon which provide boundaries for radiation hybrid markers whose sequences were included in each of the clusters. Diseases associated with the public and Incyte sequences located within the indicated intervals are also reported in the Invention where applicable. Human genome maps and other resources available to the public, such as the NCBI "GeneMap'99" World Wide Web site which can be accessed at http://www.ncbi.nlm.nih.gov/genemap, can be employed to determine if previously identified disease genes map within or in proximity to the intervals indicated above.

### VI. Labeling and Use of Individual Hybridization Probes

Hybridization probes derived from SEQ ID NO:16-30 are employed to screen cDNAs, genomic DNAs, or mRNAs. Although the labeling of oligonucleotides, consisting of about 20 base pairs, is specifically described, essentially the same procedure is used with larger nucleotide fragments. Oligonucleotides are designed using state-of-the-art software such as OLIGO 4.06 software (National Biosciences) and labeled by combining 50 pmol of each oligomer. 250 μCi of [$\gamma$-$^{32}$P] adenosine triphosphate (Amersham Pharmacia Biotech), and T4 polynucleotide kinase (DuPont NEN, Boston MA). The labeled oligonucleotides are substantially purified using a SEPHADEX G-25 superfine size exclusion dextran bead column (Amersham Pharmacia Biotech). An aliquot containing $10^7$ counts per minute of the labeled probe is used in a typical membrane-based hybridization analysis of human genomic DNA digested with one of the following endonucleases: Ase I, Bgl II, Eco RI, Pst I, Xba I, or Pvu II (DuPont NEN).

The DNA from each digest is fractionated on a 0.7% agarose gel and transferred to nylon membranes (Nytran Plus, Schleicher & Schuell, Durham NH). Hybridization is carried out for 16 hours at 40°C. To remove nonspecific signals, blots are sequentially washed at room temperature under conditions of up to, for example, 0.1 x saline sodium citrate and 0.5% sodium dodecyl sulfate. Hybridization patterns are visualized using autoradiography or an alternative imaging means and compared.

**VII. Microarrays**

The linkage or synthesis of array elements upon a microarray can be achieved utilizing photolithography, piezoelectric printing (ink-jet printing, See, e.g., Baldeschweiler, supra), mechanical microspotting technologies, and derivatives thereof. The substrate in each of the aforementioned technologies should be uniform and solid with a non-porous surface (Schena (1999), supra). Suggested substrates include silicon, silica, glass slides, glass chips, and silicon wafers. Alternatively, a procedure analogous to a dot or slot blot may also be used to arrange and link elements to the surface of a substrate using thermal, UV, chemical, or mechanical bonding procedures. A typical array may be produced using available methods and machines well known to those of ordinary skill in the art and may contain any appropriate number of elements. (See, e.g., Schena, M. et al.(1995) Science 270:467-470; Shalon, D. et al. (1996) Genome Res. 6:639-645; Marshall. A. and J. Hodgson (1998) Nat. Biotechnol. 16:27-31.)

Full length cDNAs. Expressed Sequence Tags (ESTs), or fragments or oligomers thereof may comprise the elements of the microarray. Fragments or oligomers suitable for hybridization can be selected using software well known in the art such as LASERGENE software (DNASTAR). The array elements are hybridized with polynucleotides in a biological sample. The polynucleotides in the biological sample are conjugated to a fluorescent label or other molecular tag for ease of detection. After hybridization, nonhybridized nucleotides from the biological sample are removed. and a fluorescence scanner is used to detect hybridization at each array element. Alternatively, laser desorbtion and mass spectrometry may be used for detection of hybridization. The degree of complementarity and the relative abundance of each polynucleotide which hybridizes to an element on the microarray may be assessed. In one embodiment, microarray preparation and usage is described in detail below.

Tissue or Cell Sample Preparation

Total RNA is isolated from tissue samples using the guanidinium thiocyanate method and poly(A)$^+$ RNA is purified using the oligo-(dT) cellulose method. Each poly(A)$^+$ RNA sample is reverse transcribed using MMLV reverse-transcriptase. 0.05 pg/µl oligo-(dT) primer (21mer), 1X first strand buffer, 0.03 units/µl RNase inhibitor, 500µM dATP, 500 µM dGTP, 500 µM dTTP, 40 µM dCTP, 40 µM dCTP-Cy3 (BDS) or dCTP-Cy5 (Amersham Pharmacia Biotech). The reverse transcription reaction is performed in a 25 ml volume containing 200 ng poly(A)$^+$ RNA with GEMBRIGHT kits (Incyte). Specific control poly(A)$^+$ RNAs are synthesized by in vitro transcription from non-coding yeast genomic DNA. After incubation at 37°C for 2 hr, each reaction sample (one with Cy3 and another with Cy5 labeling) is treated with 2.5 ml of 0.5M sodium hydroxide and incubated for 20 minutes at 85°C to the stop the reaction and degrade the RNA. Samples are purified using two successive CHROMA SPIN 30 gel filtration spin columns (CLONTECH Laboratories, Inc. (CLONTECH), Palo Alto CA) and after combining, both reaction samples are ethanol precipitated using 1ml of glycogen (1mg/ml), 60 ml sodium acetate, and 300 ml of 100% ethanol. The sample is then dried to completion using a SpeedVAC (Savant Instruments Inc., Holbrook NY) and resuspended in 14 µl 5X SSC/0.2% SDS.

Microarray Preparation

Sequences of the present invention are used to generate array elements. Each array element is amplified from bacterial cells containing vectors with cloned cDNA inserts. PCR amplification uses primers complementary to the vector sequences flanking the cDNA insert. Array elements are amplified in thirty cycles of PCR from an initial quantity of 1-2 ng to a final quantity greater than 5 µg. Amplified array elements are then purified using SEPHACRYL-400 (Amersham Pharmacia Biotech).

Purified array elements are immobilized on polymer-coated glass slides. Glass microscope slides (Coming) are cleaned by ultrasound in 0.1% SDS and acetone, with extensive distilled water washes between and after treatments. Glass slides are etched in 4% hydrofluoric acid (VWR Scientific Products Corporation (VWR), West Chester PA), washed extensively in distilled water. and coated with 0.05% aminopropyl silane (Sigma) in 95% ethanol. Coated slides are cured in a 110°C oven.

Array elements are applied to the coated glass substrate using a procedure described in US Patent No. 5.807.522. incorporated herein by reference. 1µl of the array element DNA, at an average concentration of 100 ng/µl, is loaded into the open capillary printing element by a high-speed robotic apparatus. The apparatus then deposits about 5 nl of array element sample per slide.

Microarrays are UV-crosslinked using a STRATALINKER UV-crosslinker (Stratagene). Microarrays are washed at room temperature once in 0.2% SDS and three times in distilled water. Non-specific binding sites are blocked by incubation of microarrays in 0.2% casein in phosphate buffered saline (PBS) (Tropix, Inc., Bedford MA) for 30 minutes at 60°C followed by washes in 0.2% SDS and distilled water as before.

Hybridization

Hybridization reactions contain 9 µl of sample mixture consisting of 0.2 µg each of Cy3 and Cy5 labeled cDNA synthesis products in 5X SSC, 0.2% SDS hybridization buffer. The sample mixture is heated to 65°C for 5 minutes and is aliquoted onto the microarray surface and covered with an 1.8 $cm^2$ coverslip. The arrays are transferred to a waterproof chamber having a cavity just slightly larger than a microscope slide. The chamber is kept at 100% humidity internally by the addition of 140 µl of 5X SSC in a comer of the chamber. The chamber containing the arrays is incubated for about 6.5 hours at 60°C. The arrays are washed for 10 min at 45°C in a first wash buffer (1 X SSC. 0.1% SDS), three times for 10 minutes each at 45°C in a second wash buffer (0.1X SSC), and dried.

Detection

Reporter-labeled hybridization complexes are detected with a microscope equipped with an Innova 70 mixed gas 10 W laser (Coherent, Inc., Santa Clara CA) capable of generating spectral lines at 488 nm for excitation of Cy3 and at 632 nm for excitation of Cy5. The excitation laser light is focused on the array using a 20X microscope objective (Nikon, Inc., Melville NY). The slide containing the array is placed on a computer-controlled X-Y stage on the microscope and raster-scanned past the objective. The 1.8 cm x 1.8 cm array used in the present example is scanned with a resolution of 20 micrometers.

In two separate scans, a mixed gas multiline laser excites the two fluorophores sequentially. Emitted light is split, based on wavelength, into two photomultiplier tube detectors (PMT R1477, Hamamatsu Photonics Systems. Bridgewater NJ) corresponding to the two fluorophores. Appropriate filters positioned between the array and the photomultiplier tubes are used to filter the signals. The emission maxima of the fluorophores used are 565 nm for Cy3 and 650 nm for Cy5. Each array is typically scanned twice, one scan per fluorophore using the appropriate filters at the laser source, although the apparatus is capable of recording the spectra from both fluorophores simultaneously.

The sensitivity of the scans is typically calibrated using the signal intensity generated by a cDNA control species added to the sample mixture at a known concentration. A specific location on the array contains a complementary DNA sequence, allowing the intensity of the signal at that location to be correlated with a weight ratio of hybridizing species of 1:100,000. When two samples from different sources (e.g., representing test and control cells), each labeled with a different fluorophore, are hybridized to a single array for the purpose of identifying genes that are differentially expressed, the calibration is done by labeling samples of the calibrating cDNA with the two fluorophores and adding identical amounts of each to the hybridization mixture.

The output of the photomultiplier tube is digitized using a 12-bit RTI-835H analog-to-digital (A/D) conversion board (Analog Devices, Inc., Norwood MA) installed in an IBM-compatible PC computer. The digitized data are displayed as an image where the signal intensity is mapped using a linear 20-color transformation to a pseudocolor scale ranging from blue (low signal) to red (high signal). The data is also analyzed quantitatively. Where two different fluorophores are excited and measured simultaneously, the data are first corrected for optical crosstalk (due to overlapping emission spectra) between the fluorophores using each fluorophore' emission spectrum.

A grid is superimposed over the fluorescence signal image such that the signal from each spot is centered in each element of the grid. The fluorescence signal within each element is then integrated to obtain a numerical value corresponding to the average intensity of the signal. The software used for signal analysis is the GEMTOOLS gene expression analysis program (Incyte).

**VIII. Complementary Polynucleotides**

Sequences complementary to the SYNT-encoding sequences, or any parts thereof, are used to detect, decrease, or inhibit expression of naturally occurring SYNT. Although use of oligonucleotides comprising from about 15 to 30 base pairs is described, essentially the same procedure is used with smaller or with larger sequence fragments. Appropriate oligonucleotides are designed using OLIGO 4.06 software (National Biosciences) and the coding sequence of SYNT. To inhibit transcription, a complementary oligonucleotide is designed from the most unique 5'sequence and used to prevent promoter binding to the coding sequence. To inhibit translation, a complementary oligonucleotide is designed to prevent ribosomal binding to the SYNT-encoding transcript.

**IX. Expression of SYNT**

Expression and purification of SYNT is achieved using bacterial or virus-based expression systems. For expression of SYNT in bacteria, cDNA is subcloned into an appropriate vector containing an antibiotic resistance gene and an inducible promoter that directs high levels of cDNA transcription. Examples of such promoters include, but are not limited to, the *trp-lac (tac)* hybrid promoter and the T5 or T7 bacteriophage promoter in conjunction with the *lac* operator regulatory element. Recombinant vectors are transformed into suitable bacterial hosts, e.g., BL21(DE3). Antibiotic resistant bacteria express SYNT upon induction with isopropyl beta-D-thiogalactopyranoside (IPTG). Expression of SYNT in eukaryotic cells is achieved by infecting insect or mammalian cell lines with recombinant Autographica californica nuclear polyhedrosis virus (AcMNPV), commonly known as baculovirus. The nonessential polyhedrin gene of baculovirus is replaced with cDNA encoding SYNT by either homologous recombination or bacterial-mediated transposition involving transfer plasmid intermediates. Viral infectivity is maintained and the strong polyhedrin promoter drives high levels of cDNA transcription. Recombinant baculovirus is used to infect Spodoptera frugiperda (Sf9) insect cells in most cases, or human hepatocytes, in some cases. Infection of the latter requires additional genetic modifications to baculovirus. (See Engelhard. E.K. et al. (1994) Proc. Natl. Acad. Sci. USA 91:3224-3227; Sandig, V. et al. (1996) Hum. Gene Ther. 7:1937-1945.)

In most expression systems, SYNT is synthesized as a fusion protein with, e.g., glutathione S-transferase (GST) or a peptide epitope tag, such as FLAG or 6-His, permitting rapid, single-step, affinity-based purification of recombinant fusion protein from crude cell lysates. GST, a 26-kilodalton enzyme from Schistosoma japonicum, enables the purification of fusion proteins on immobilized glutathione under conditions that maintain protein activity and antigenicity (Amersham Pharmacia Biotech). Following purification, the GST moiety can be proteolytically cleaved from SYNT at specifically engineered sites. FLAG, an 8-amino acid peptide, enables immunoaffinity purification using commercially available monoclonal and polyclonal anti-FLAG antibodies (Eastman Kodak). 6-His, a stretch of six consecutive histidine residues, enables purification on metal-chelate resins (QIAGEN). Methods for protein expression and purification are discussed in Ausubel (1995, supra, ch. 10 and 16). Purified SYNT obtained by these methods can be used directly in the assays shown in Examples X and XIV.

## X. Demonstration of SYNT Activity

An SYNT activity assay measures aminoacylation of tRNA in the presence of a radiolabeled substrate. A cell-free extract depleted of endogenous aminoacyl-tRNA synthetase is prepared from Escherichia coli. SYNT, either biochemically purified or recombinantly produced, is added to the cell free extract. The cell-free extract is incubated with [$^{14}$C]-labeled amino acid under conditions favorable for translation. Incorporation of the [$^{14}$C]-labeled amino acid into acid-precipitable aminoacyl-tRNA is measured using a radioisotope counter. The amount of the [$^{14}$C]-labeled amino acid incorporated into aminoacyl tRNA is proportional to the amount of SYNT activity. (See, for example. Ibba. M. et al. (1997) Science 278:1119-1 122).

Alternatively, SYNT activity may be assayed as follows. SYNT, or biologically active fragments thereof, are labeled with $^{125}$I Bolton-Hunter reagent. (See, e.g., Bolton et al. (1973) Biochem. J. 133:529.) Candidate molecules previously arrayed in the wells of a multi-well plate are incubated with the labeled SYNT, washed, and any wells with labeled SYNT complex are assayed. Data obtained using different concentrations of SYNT are used to calculate values for the number, affinity, and association of SYNT with the candidate molecules.

## XI. Functional Assays

SYNT function is assessed by expressing the sequences encoding SYNT at physiologically elevated levels in mammalian cell culture systems. cDNA is subcloned into a mammalian expression vector containing a strong promoter that drives high levels of cDNA expression. Vectors of choice include pCMV SPORT plasmid (Life Technologies) and pCR3.1 plasmid (Invitrogen), both of which contain the cytomegalovirus promoter. 5-10 /μg of recombinant vector are transiently transfected into a human cell line, for example, an endothelial or hematopoietic cell line, using either liposome formulations or electroporation. 1-2 μg of an additional plasmid containing sequences encoding a marker protein are co-transfected. Expression of a marker protein provides a means to distinguish transfected cells from nontransfected cells and is a reliable predictor of cDNA expression from the recombinant vector. Marker proteins of choice include, e.g., Green Fluorescent Protein (GFP; Clontech), CD64, or a CD64-GFP fusion protein. Flow cytometry (FCM), an automated, laser optics-based technique, is used to identify transfected cells expressing GFP or CD64-GFP and to evaluate the apoptotic state of the cells and other cellular properties. FCM detects and quantifies the uptake of fluorescent molecules that diagnose events preceding or coincident with cell death. These events include changes in nuclear DNA content as measured by staining of DNA with propidium iodide; changes in cell size and granularity as measured by forward light scatter and 90 degree side light scatter; down-regulation of DNA synthesis as measured by decrease in bromodeoxyuridine uptake; alterations in expression of cell surface and intracellular proteins as measured by reactivity with specific antibodies; and alterations in plasma membrane composition as measured by the binding of fluorescein-conjugated Annexin V protein to the cell surface. Methods in flow cytometry are discussed in Ormerod, M.

G. (1994) Flow Cytometry, Oxford, New York NY.

The influence of SYNT on gene expression can be assessed using highly purified populations of cells transfected with sequences encoding SYNT and either CD64 or CD64-GFP. CD64 and CD64-GFP are expressed on the surface of transfected cells and bind to conserved regions of human immunoglobulin G (IgG). Transfected cells are efficiently separated from nontransfected cells using magnetic beads coated with either human IgG or antibody against CD64 (DYNAL, Lake Success NY). mRNA can be purified from the cells using methods well known by those of skill in the art. Expression of mRNA encoding SYNT and other genes of interest can be analyzed by northern analysis or microarray techniques.

### XII. Production of SYNT Specific Antibodies

SYNT substantially purified using polyacrylamide gel electrophoresis (PAGE; see, e.g., Harrington, M.G. (1990) Methods Enzymol. 182:488-495), or other purification techniques, is used to immunize rabbits and to produce antibodies using standard protocols.

Alternatively, the SYNT amino acid sequence is analyzed using LASERGENE software (DNASTAR) to determine regions of high immunogenicity, and a corresponding oligopeptide is synthesized and used to raise antibodies by means known to those of skill in the art. Methods for selection of appropriate epitopes, such as those near the C-terminus or in hydrophilic regions are well described in the art. (See. e.g.. Ausubel, 1995, supra, ch. 11.)

Typically, oligopeptides of about 15 residues in length are synthesized using an ABI 431A peptide synthesizer (PE Biosystems) using FMOC chemistry and coupled to KLH (Sigma-Aldrich, St. Louis MO) by reaction with N-maleimidobenzoyl-N-hydroxysuccinimide ester (MBS) to increase immunogenicity. (See. e.g., Ausubel, 1995, supra.) Rabbits are immunized with the oligopeptide-KLH complex in complete Freund's adjuvant. Resulting antisera are tested for antipeptide and anti-SYNT activity by, for example, binding the peptide or SYNT to a substrate, blocking with 1% BSA. reacting with rabbit antisera, washing, and reacting with radio-iodinated goat anti-rabbit IgG.

### XIII. Purification of Naturally Occurring SYNT Using Specific Antibodies

Naturally occurring or recombinant SYNT is substantially purified by immunoaffinity chromatography using antibodies specific for SYNT. An immunoaffinity column is constructed by covalently coupling anti-SYNT antibody to an activated chromatographic resin, such as CNBr-activated SEPHAROSE (Amersham Pharmacia Biotech). After the coupling, the resin is blocked and washed according to the manufacturer's instructions.

Media containing SYNT are passed over the immunoaffinity column, and the column is washed under conditions that allow the preferential absorbance of SYNT (e.g., high ionic strength buffers in the presence of detergent). The column is eluted under conditions that disrupt antibody/SYNT binding (e.g., a buffer of pH 2 to pH 3, or a high concentration of a chaotrope, such as urea or thiocyanate ion), and SYNT is collected.

### XIV. Identification of Molecules Which Interact with SYNT

SYNT, or biologically active fragments thereof, are labeled with $^{125}$I Bolton-Hunter reagent. (See, e.g., Bolton A.E. and W.M. Hunter (1973) Biochem. J. 133:529-539.) Candidate molecules previously arrayed in the wells of a multi-well plate are incubated with the labeled SYNT, washed, and any wells with labeled SYNT complex are assayed. Data obtained using different concentrations of SYNT are used to calculate values for the number, affinity, and association of SYNT with the candidate molecules.

Alternatively, molecules interacting with SYNT are analyzed using the yeast two-hybrid system as described in Fields, S. and O. Song (1989. Nature 340:245-246), or using commercially available kits based on the two-hybrid system, such as the MATCHMAKER system (Clontech).

SYNT may also be used in the PATHCALLING process (CuraGen Corp., New Haven CT) which employs the yeast two-hybrid system in a high-throughput manner to determine all interactions between the proteins encoded by two large libraries of genes (Nandabalan. K. et al. (2000) U.S. Patent No. 6,057,101).

Various modifications and variations of the described methods and systems of the invention will be apparent to those skilled in the art without departing from the scope and spirit of the invention. Although the invention has been described in connection with certain embodiments, it should be understood that the invention as claimed should not be unduly limited to such specific embodiments. Indeed, various modifications of the described modes for carrying out the invention which are obvious to those skilled in molecular biology or related fields are intended to be within the scope of the following claims.

# Table 1

| Polypeptide SEQ ID NO: | Nucleotide SEQ ID NO: | Clone ID | Library | Fragments |
|---|---|---|---|---|
| 1 | 16 | 1806212 | SINTNOT13 | 268731R1 (HNT2NOT01), 410547R1 (BRSTNOT01), 642383F1 (BRSTNOT03), 642383R6 (BRSTNOT03), 1484778F6 (CORPNOT02), 1577276X13 (LNODNOT03), 1577276X14 (LNODNOT03), 1577276X16 (LNODNOT03), 1733808F6 (BRSTTUT08), 2057185R6 (BEPINOT01) |
| 2 | 17 | 2083883 | UTRSNOT08 | 1308705F1 (COLNFET02), 1337653F1 (COLNNOT13), 1430326F1 (SINTBST01), 1525049F6 (UCMCL5T01), 1525049T7 (UCMCL5T01), 1807032F6 (SINTNOT13), 2083883X31C1 (UTRSNOT08), 20838831X54C1, SAEA01919R1, SAEA00070F1 |
| 3 | 18 | 2454288 | ENDANOT01 | 1334395F6 (COLNNOT13), 1607276F6 (LUNGNOT15), 1930074F6 (COLNTUT03), 1978977R6 (LUNGTUT03), 2054860R6 (BEPINOT01), 2454288H1 (ENDANOT01), 2791177H2 (COLNTUT16), 4049241H1 (SINTNOT18), 4787755H1 (BRATNOT03) |
| 4 | 19 | 1513539 | PANCTUT01 | 285705F1 (EOSIHET02), 515506F1 (MMLR1DT01), 515506R1 (MMLR1DT01), 723201R1 (SYNOOAT01), 726178R1 (SYNOOAT01), 906091R1 (COLNNOT08), 1492309T1 (PROSNON01), 1513539H1 (PANCTUT01), 2632033H1 (COLNTUT15) |
| 5 | 20 | 2148623 | BRAINOT09 | 655968H1 (EOSINOT03), 820407T1 (KERANOT02), 1383948F1 (BRAITUT08), 1383996T6 (BRAITUT08), 1441048R1 (THYRNOT03), 1579330F6 (DUODNOT01), 2148623H1 (BRAINOT09), 3170589H1 (BRSTNOT18), 3251591H1 (SEMVNOT03), 3618285H1 (EPIPNOT01), 3637486H1 (LUNGNOT30), g1524807 |
| 6 | 21 | 2579405 | KIDNTUT13 | 2457R1 (U937NOT01), 270182R1 (HNT2NOT01), 730115H1 (LUNGNOT03), 991450H1 (COLNNOT11), 994055T1 (COLNNOT11), 1308496H1 (COLNFET02), 1808269F6 (SINTNOT13), 1875536T6 (LEUKNOT02), 1978055H1 (LUNGTUT03), 2579405H1 (KIDNTUT13), 2583944H1 (BRAITUT22) |
| 7 | 22 | 2662427 | ADRENOT08 | 283147F1 (CARDNOT01), 904594R2 (COLNNOT07), 2662427H1 (ADRENOT08), 2860066H1 (SININOT03), 3078653H1 (BRAIUNT01), 3852430F6 (BRAITUT12), 3852430T6 (BRAITUT12), SCDA09024V1, SCDA05143V1 |

## Table 1 (cont.)

| Polypeptide SEQ ID NO: | Nucleotide SEQ ID NO: | Clone ID | Library | Fragments |
|---|---|---|---|---|
| 8 | 23 | 2844928 | DRGLNOT01 | 1466601F1 (PANCTUT02), 1662986F6 (BRSTNOT09), 1732880F6 (BRSTTUT08), 1804107F6 (SINTNOT13), 2521851F6 (BRAITUT21), 2662837X303B1 (ADRENOT08), 2844928H1 (DRGLNOT01), 2967532H1 (SCORNOT04), 3774082H1 (BRSTNOT25), 5871970H1 (COLTDIT04) |
| 9 | 24 | 3231586 | COTRNOT01 | 319277H1 (EOSIHET02), 920940H1 (RATRNOT02), 1445995H1 (PLACNOT02), 1517438F1 (PANCTUT01), 1963042H1 (BRSTNOT04), 2023159H1 (CONNNOT01), 2932203H1 (UTRSNOR01), 3231586H1 (COTRNOT01), 3346128H1 (BRAITUT24), g1474393 |
| 10 | 25 | 3580770 | 293TF3T01 | 269986R1 (HNT2NOT01), 269986R6 (HNT2NOT01), 3580770H1 (293TF3T01), 3602575F6 (DRGTNOT01), 3602575T6 (DRGTNOT01), g1839725 |
| 11 | 26 | 3778612 | BRSTNOT27 | 3778612F6 (BRSTNOT27), 3778612H1 (BRSTNOT27), 4063060T6 (BRAINOT21) |
| 12 | 27 | 4574912 | PROSTMT02 | 224714F1 (PANCNOT01), 452757F1 (TLYMNOT02), 1210705R7 (BRSTNOT02), 1437821F6 (PANCNOT08), 2700694F6 (OVARTUT10), 3963634H1 (PROSNOT14), 3964731H1 (PROSNOT14), 4574912H1 (PROSTMT02), 5035739H1 (LIVRTUT13) |
| 13 | 28 | 5630806 | PLACFER01 | 1649584X20C1 (PROSTUT09), 1649855X12C1 (PROSTUT09), 1650519X14C1 (PROSTUT09), 2054061R6 (BEPINOT01), 2347805X304V1 (COLSUCT01), 3384595H1 (ESOGNOT04), 5630806H1 (PLACFER01) |
| 14 | 29 | 5854855 | FIBAUNT02 | 746434R1 (BRAITUT01), 815240R1 (OVARTUT01), 2515988F6 (LIVRTUT04), 3105953F6 (BRSTTUT15), 5854855H1 (FIBAUNT02) |
| 15 | 30 | 5993973 | FTUBTUT02 | 270363H1 (HNT2NOT01), 1863382F6 (PROSNOT19), 2696339F6 (UTRSNOT12), 3500696H1 (PROSTUT13), 4247086H1 (BRABDIT01), 5195020H1 (LUNLTUT04), 5591818H1 (COLCDIT03), 6023466H1 (TESTNOT11) |

# Table 2

| Polypeptide SEQ ID NO: | Amino Acid Residues | Potential Phosphorylation Sites | Potential Glycosylation Sites | Signature Sequences, Motifs, and Domains | Homologous Sequences | Analytical Methods and Databases |
|---|---|---|---|---|---|---|
| 1 | 1176 | T7 T238 T543 S35 S68 S167 S175 T224 S241 T379 S396 T444 S453 S518 T552 S593 S673 T749 T769 T791 S978 S1042 T1083 T26 T41 S128 S153 T293 T327 S368 T557 S697 S714 S783 T925 S1082 T1136 S1153 Y111 Y666 | N33 N333 N439 N882 | Leucine-tRNA ligase domain T303-I870<br>Amino-transfer RNA synthetase N36-F88 N713-N723 | similar to leucyl-tRNA synthetase [Arabidopsis thaliana] g2160156 | MOTIFS<br>BLAST-GenBank<br>BLAST-DOMO<br>PROFILESCAN<br>BLAST-PRODOM<br>BLIMPS-BLOCKS |
| 2 | 739 | S28 T132 S185 S282 S288 S307 T320 T352 S451 S661 T728 S732 S116 S163 S168 S261 T398 S436 T590 S633 T714 Y173 Y349 Y725 Y734 | N102 N588 N619 | AMP-binding domain S163-V629, N294-V345 E309-I320, T321-I329<br>Long-chain acyl-CoA synthetase P158-E280 | fatty acid coenzyme A ligase 5 [Homo sapiens] g6174680 | MOTIFS<br>BLAST-GenBank<br>BLAST-DOMO<br>PROFILESCAN<br>BLAST-PRODOM<br>BLIMPS-BLOCKS<br>HMMER-PFAM<br>BLIMPS-PRINTS |
| 3 | 589 | S179 T18 T20 S40 S138 T193 T256 T298 T354 S419 S485 T570 S579 S5 T114 T174 | N187 | Phenylalanyl-tRNA synthetase P2-Q556, N293-L589<br>Synthetase b chain D59-L84, R355-Y374 I439-N465 | phenylalanyl tRNA synthetase beta subunit [M. musculus] g4633656 | MOTIFS<br>BLAST-GenBank<br>BLAST-PRODOM<br>BLIMPS-PRODOM |

# Table 2 (cont.)

| Polypeptide SEQ ID NO: | Amino Acid Residues | Potential Phosphorylation Sites | Potential Glycosylation Sites | Signature Sequences, Motifs, and Domains | Homologous Sequences | Analytical Methods and Databases |
|---|---|---|---|---|---|---|
| 4 | 157 | S26 S69 T131 T132 S108 | N18 | Signal peptide: M1-V66<br>Acetyl-CoA carboxylase carboxyl transferase alpha subunit signature: G128-I139 | acetyl-coenzyme A synthetase [Penicillium chrysogenum] g265229 | BLAST-GenBank<br>SPSCAN<br>BLOCKS-PRINTS<br>MOTIFS |
| 5 | 643 | T308 T337 S402 S433 T454 S34 S101 T408 S530 S621 | N9 N33 N90 N104 N259 N306 N431 N571 N634 | Class II glutamine amidotransferase motif: L27-K36, G545-F560 (p<0.01)<br>Transferase/ligase motif: R30-A224, S258-F356, P404-Q484 | putative asparagine synthase [S. pombe] g3560144 | BLAST-GenBank<br>BLIMPS-BLOCKS<br>BLAST-PRODOM<br>MOTIFS |
| 6 | 660 | T282 S315 S329 T487 T596 S9 S276 T459 T624 Y230 | N140 | Class-I aminoacyl-tRNA synthetase signature: I74-M660, V183-L233, P201-L212, S519-N529<br>Arg-tRNA synthetase: K193-L224, V232-F245, T374-R395, H237-M660, S78-M660 | arginyl-tRNA synthetase, ArgRS [Homo sapiens] g1217668 | BLAST-GenBank<br>HMMER-PFAM<br>BLIMPS-BLOCKS<br>PROFILESCAN<br>BLIMPS-PFAM<br>BLAST-PRODOM<br>BLAST-DOMO<br>MOTIFS |

# Table 2 (cont.)

| Polypeptide SEQ ID NO: | Amino Acid Residues | Potential Phosphorylation Sites | Potential Glycosylation Sites | Signature Sequences, Motifs, and Domains | Homologous Sequences | Analytical Methods and Databases |
|---|---|---|---|---|---|---|
| 7 | 725 | S723 S80 S220 S238 T351 S371 T414 T465 S591 S641 T38 T45 T67 T165 S230 T252 T407 S413 S454 S499 S541 T554 T685 S688 T693 S719 | N48 N472 N546 N552 | Biotin-requiring enzyme signature: P652-F714, I657-R707<br>Biotin attachment site: K681<br>Acetyl-CoA biotin carboxyl carrier protein signature: F661-M675, V676-P689<br>Carbamoyl-PO4 synthase signature: K51-P490, E193-K494, F333-L340, L245-P290, Y270-K494, F331-G365 | 3-methyl-crotonyl CoA carboxylase homolog [C. elegans] g3876562 | BLAST-GenBank<br>HMMER-PFAM<br>BLIMPS-BLOCKS<br>PROFILESCAN<br>BLIMPS-PRINTS<br>BLAST-PRODOM<br>BLAST-DOMO<br>MOTIFS |
| 8 | 644 | S30 S253 S370 T457 T496 T593 T594 S43 T65 S207 S237 S269 S626 Y247 Y521 | N282 | AMP-binding domain signature: E79-V538, T101-V540, S240-L293, H255-L275, F262-H277, I260-K271, V570-R635<br>Ligase/synthetase signature: T356-R640 | predicted long chain fatty acid CoA ligase [C. elegans] g4262587 | BLAST-GenBank<br>HMMER-PFAM<br>BLIMPS-BLOCKS<br>PROFILESCAN<br>BLIMPS-PRINTS<br>BLAST-PRODOM<br>BLAST-DOMO<br>MOTIFS |
| 9 | 504 | S122 S134 S428 S111 T349 | N9 N39 N209 N426 | Signal peptide: M1-A67<br>Aminotransferase class-II pyridoxal-PO4 attachment site: A104-R113 | | SPSCAN<br>BLIMPS-BLOCKS<br>MOTIFS |

Table 2 (cont.)

| Polypeptide SEQ ID NO: | Amino Acid Residues | Potential Phosphorylation Sites | Potential Glycosylation Sites | Signature Sequences, Motifs, and Domains | Homologous Sequences | Analytical Methods and Databases |
|---|---|---|---|---|---|---|
| 10 | 489 | S51 T150 T220 S465 S477 T90 S200 T250 T350 S433 S467 Y83 | | RNA methyl transferase SpoU family: P344-G354<br>CTP synthetase domain: K2-N455, M1-Y457, M335-Y457 | CTP synthetase homolog (CTPsH) [Mus musculus] g1654185 | BLAST-GenBank BLIMPS-PFAM BLAST-PRODOM BLAST-DOMO MOTIFS |
| 11 | 258 | S105 S115 S139 | | Signal peptide: M1-G43<br>ATP synthase signature: Y98-L129<br>S-adenosyl methionine synthetase signature: A209-V257 | | SPSCAN HMMER-PFAM PROFILESCAN MOTIFS |
| 12 | 555 | S22 T72 T110 S132 S165 S190 S258 S342 S363 S446 S539 S31 T179 S212 S312 T318 S339 S380 S392 T393 T407 S428 T519 Y24 | | Aminoacyl tRNA synthetase signature: V73-A82, K305-N315 (p<1.1e-3)<br>Cysteinyl-tRNA synthetase signature: R66-A77, I103-V112, E230-C248, D261-E282, Y67-E282, H76-A494 | putative cysteinyl-tRNA synthetase [S. pombe] g1044932 | BLAST-GenBank BLIMPS-BLOCKS BLIMPS-PRINTS BLAST-PRODOM BLAST-DOMO MOTIFS |
| 13 | 463 | S54 S79 S124 S235 T253 T295 T411 T15 S366 Y84 | N52 | Succinyl-CoA ligase family: I97-F111, V181-E193, L314-A343, L53-D367, D290-K442, L53-I463 | ATP-specific succinyl-CoA synthetase beta subunit [Homo sapiens] g3766196 | BLAST-GenBank BLIMPS-BLOCKS BLAST-PRODOM BLAST-DOMO MOTIFS |

## Table 2 (cont.)

| Polypeptide SEQ ID NO: | Amino Acid Residues | Potential Phosphorylation Sites | Potential Glycosylation Sites | Signature Sequences, Motifs, and Domains | Homologous Sequences | Analytical Methods and Databases |
|---|---|---|---|---|---|---|
| 14 | 399 | T151 S179 S19 S72 T95 T151 S157 S191 S192 T217 T218 S260 T318 S337 S353 | | Signal peptide: M1-G42<br>Class-I aminoacyl-tRNA synthetase signature: P54-L64 | | SPSCAN<br>MOTIFS |
| 15 | 339 | S187 T232 T266 S9 S31 T221 T231 S274 S276 T278 S324 S31 S36 T78 S223 S317 Y265 | | Phenylalanyl-tRNA synthetase family: V81-E261 | similar to phenylalanyl-tRNA synthetase [C. elegans] g3876233 | BLAST-GenBank<br>BLAST-PRODOM<br>MOTIFS |

# Table 3

| Nucleotide SEQ ID NO: | Selected Fragments | Tissue Expression (Fraction of Total) | Disease or Condition (Fraction of Total) | Vector |
|---|---|---|---|---|
| 16 | 153-197<br>600-644<br>1198-2042 | Nervous (0.240)<br>Reproductive (0.231)<br>Hematopoietic/Immune (0.124) | Cancer (0.488)<br>Inflammation/Trauma (0.306)<br>Cell proliferation (0.198) | pINCY |
| 17 | 163-207<br>1090-1134 | Gastrointestinal (0.333)<br>Reproductive (0.205)<br>Hematopoietic/Immune (0.141) | Cancer (0.462)<br>Inflammation/Trauma (0.372)<br>Cell proliferation (0.103) | pINCY |
| 18 | 601-645 | Reproductive (0.216)<br>Gastrointestinal (0.205)<br>Nervous (0.148) | Cancer (0.477)<br>Inflammation/Trauma (0.330)<br>Cell proliferation (0.227) | PBLUESCRIPT |
| 19 | 1-531<br>1634-2122 | Reproductive (0.224)<br>Nervous (0.165)<br>Gastrointestinal (0.153) | Cancer (0.365)<br>Inflammation (0.282)<br>Cell proliferation (0.176) | pINCY |
| 20 | 345-418<br>1272-1762<br>2039-2357 | Cardiovascular (0.194)<br>Reproductive (0.181)<br>Nervous (0.153) | Cancer (0.514)<br>Cell proliferation (0.194)<br>Inflammation (0.181) | pINCY |
| 21 | 1-109 | Gastrointestinal (0.175)<br>Nervous (0.175)<br>Reproductive (0.175) | Cancer (0.427)<br>Inflammation (0.252)<br>Cell proliferation (0.243) | pINCY |
| 22 | 2045-2317 | Nervous (0.232)<br>Reproductive (0.214)<br>Cardiovascular (0.179) | Cancer (0.411)<br>Inflammation (0.268)<br>Trauma (0.161) | pINCY |
| 23 | 1-508, 840-1183<br>1656-1741<br>1968-2254 | Reproductive (0.266)<br>Nervous (0.188)<br>Endocrine (0.125) | Cancer (0.578)<br>Inflammation (0.234)<br>Cell proliferation (0.125) | pINCY |
| 24 | | Reproductive (0.315)<br>Gastrointestinal (0.150)<br>Hematopoietic/Immune (0.150) | Cancer (0.433)<br>Inflammation (0.283)<br>Cell proliferation (0.126) | pINCY |
| 25 | 1-151 | Reproductive (0.333)<br>Nervous (0.250)<br>Hematopoietic (0.167) | Cancer (0.583)<br>Inflammation (0.333)<br>Cell proliferation (0.167) | pINCY |

## Table 3 (cont.)

| Nucleotide SEQ ID NO: | Selected Fragments | Tissue Expression (Fraction of Total) | Disease or Condition (Fraction of Total) | Vector |
|---|---|---|---|---|
| 26 | 1-214<br>567-970 | Nervous (0.286)<br>Reproductive (0.286)<br>Cardiovascular (0.143)<br>Endocrine (0.143) | Cancer (0.524)<br>Inflammation (0.190)<br>Trauma (0.190) | pINCY |
| 27 | 1-206<br>1129-1206 | Reproductive (0.243)<br>Hematopoietic/Immune (0.143)<br>Cardiovascular (0.129)<br>Gastrointestinal (0.129)<br>Nervous (0.129) | Cancer (0.457)<br>Cell proliferation (0.229)<br>Inflammation (0.186) | pINCY |
| 28 | | Nervous (0.238)<br>Gastrointestinal (0.190)<br>Reproductive (0.190) | Cancer (0.429)<br>Inflammation (0.274)<br>Cell proliferation (0.131) | pINCY |
| 29 | 1-234<br>1229-1477 | Reproductive (0.331)<br>Nervous (0.204)<br>Gastrointestinal (0.116) | Cancer (0.508)<br>Cell proliferation (0.188)<br>Inflammation (0.182) | pINCY |
| 30 | 1-871<br>1026-1138<br>1356-1660 | Reproductive (0.378)<br>Nervous (0.162)<br>Gastrointestinal (0.135) | Cancer (0.649)<br>Cell proliferation (0.189)<br>Inflammation (0.189) | pINCY |

Table 4

| Nucleotide SEQ ID NO: | Library | Library Description |
|---|---|---|
| 16 | SINTNOT13 | Library was constructed using RNA isolated from ileum tissue obtained from a 25-year-old Asian female during a partial colectomy and temporary ileostomy. Pathology indicated moderately active chronic ulcerative colitis, involving colonic mucosa from the distal margin to the ascending colon. Family history included hyperlipidemia, malignant cervical neoplasm, viral hepatitis A, and depressive disorder. |
| 17 | UTRSNOT08 | Library was constructed using RNA isolated from uterine tissue removed from a 35-year-old Caucasian female during a vaginal hysterectomy with dilation and curettage. Pathology indicated that the endometrium was secretory phase with a benign endometrial polyp 1 cm in diameter. The cervix showed mild chronic cervicitis. Family history included atherosclerotic coronary artery disease and type II diabetes. |
| 18 | ENDANOT01 | Library was constructed using RNA isolated from aortic endothelial cell tissue from an explanted heart removed from a male during a heart transplant. |
| 19 | PANCTUT01 | This library was constructed using RNA isolated from pancreatic tumor tissue removed from a 65-year-old Caucasian female. Pathology indicated invasive adenocarcinoma. Patient history included type II diabetes, osteoarthritis, cardiovascular disease, benign neoplasm in the large bowel, and a cataract. Family history included cardiovascular disease, type II diabetes, and stomach cancer. |
| 20 | BRAINOT09 | This library was constructed using RNA isolated from brain tissue removed from a Caucasian male fetus, who died at 23 weeks' gestation. |
| 21 | KIDNTUT13 | This library was constructed using RNA isolated from kidney tumor tissue removed from a 51-year-old Caucasian female. Pathology indicated renal cell carcinoma. Patient history included depressive disorder, hypoglycemia, and uterine endometriosis. Family history included calculus of the kidney, colon cancer, and type II diabetes. |
| 22 | ADRENOT08 | This library was constructed using RNA isolated from adrenal tissue removed from a 20-year-old Caucasian male, who died from head trauma. |

## Table 4 (cont.)

| Nucleotide SEQ ID NO: | Library | Library Description |
|---|---|---|
| 23 | DRGLNOT01 | This library was constructed using RNA isolated from dorsal root ganglion tissue removed from the low thoracic/high lumbar region of a 32-year-old Caucasian male who died from acute pulmonary edema and bronchopneumonia, bilateral pleural and pericardial effusions, and malignant lymphoma (natural killer cell type). Patient history included probable cytomegalovirus infection, hepatic congestion and steatosis, splenomegaly, hemorrhagic cystitis, thyroid hemorrhage, and Bell's palsy. Treatment included radiation therapy. |
| 24 | COTRNOT01 | This library was constructed using RNA isolated from diseased transverse colon tissue obtained from a 26-year-old Caucasian male. Pathology indicated minimally active pancolitis with areas of focal severe colitis with perforation, consistent with Crohn's disease. |
| 25 | 293TF3T01 | This library was constructed using RNA isolated from a serum-starved transformed embryonal cell line (293-EBNA) derived from kidney epithelial tissue. The cells were transformed with adenovirus 5 DNA. |
| 26 | BRSTNOT27 | This library was constructed using RNA isolated from breast tissue removed from a 57-year-old Caucasian female. Pathology for the associated tumor tissue indicated residual micro-scopic infiltrating adenocarcinoma and extensive intraductal carcinoma. Immunoperoxidase stains for estrogen and progesterone receptors were positive. Patient history included benign hypertension, hyperlipidemia, cardiac dysrhythmia, a benign colon neoplasm, a solitary breast cyst, and a breast neoplasm. Family history included benign hypertension, acute leukemia, primary liver cancer, and lung cancer. |
| 27 | PROSTMT02 | This library was constructed using RNA isolated from diseased prostate tissue removed from a 66-year-old Caucasian male. Pathology indicated adenofibromatous hyperplasia. Pathology from the associated tumor indicated adenocarcinoma. The patient presented with elevated prostate specific antigen (PSA) and induration. Family history included acute myocardial infarction, atherosclerotic coronary artery disease, type II diabetes, hyperlipidemia, and Jakob-Creutzfeldt disease. |

Table 4 (cont.)

| Nucleotide SEQ ID NO: | Library | Library Description |
|---|---|---|
| 28 | PLACFER01 | This library was constructed from RNA isolated from placental tissue from a Caucasian fetus, who died at 16 weeks' gestation from fetal demise and hydrocephalus. Patient history included umbilical cord wrapped around the head and shoulders. Serology was positive for anti-CMV. Family history included multiple pregnancies and live births, and an abortion. |
| 29 | FIBAUNT02 | This library was constructed using RNA isolated from untreated aortic adventitial fibroblasts removed from a 65-year-old Caucasian female. |
| 30 | FTUBTUT02 | This library was constructed using RNA isolated from fallopian tube tumor tissue removed from an 85-year-old Caucasian female. Pathology indicated poorly differentiated mixed endometrioid and serous adenocarcinoma. Pathology for the associated uterus tumor indicated focal and metastatic endometrioid adenocarcinoma and moderately differentiated invasive adenocarcinoma. Patient history included medullary carcinoma of the thyroid and myocardial infarction. |

# Table 5

| Program | Description | Reference | Parameter Threshold |
|---|---|---|---|
| ABI FACTURA | A program that removes vector sequences and masks ambiguous bases in nucleic acid sequences. | PE Biosystems, Foster City, CA. | |
| ABI/PARACEL FDF | A Fast Data Finder useful in comparing and annotating amino acid or nucleic acid sequences. | PE Biosystems, Foster City, CA; Paracel Inc., Pasadena, CA. | Mismatch <50% |
| ABI AutoAssembler | A program that assembles nucleic acid sequences. | PE Biosystems, Foster City, CA. | |
| BLAST | A Basic Local Alignment Search Tool useful in sequence similarity search for amino acid and nucleic acid sequences. BLAST includes five functions: blastp, blastn, blastx, tblastn, and tblastx. | Altschul, S.F. et al. (1990) J. Mol. Biol. 215:403-410; Altschul, S.F. et al. (1997) Nucleic Acids Res. 25:3389-3402. | *ESTs*: Probability value= 1.0E-8 or less<br>*Full Length sequences*: Probabilit value= 1.0E-10 or less |
| FASTA | A Pearson and Lipman algorithm that searches for similarity between a query sequence and a group of sequences of the same type. FASTA comprises as least five functions: fasta, tfasta, fastx, tfastx, and ssearch. | Pearson, W.R. and D.J. Lipman (1988) Proc. Natl. Acad Sci. USA 85:2444-2448; Pearson, W.R. (1990) Methods Enzymol. 183:63-98; and Smith, T.F. and M.S. Waterman (1981) Adv. Appl. Math. 2:482-489. | *ESTs*: fasta E value=1.06E-6<br>*Assembled ESTs*: fasta Identity= 95% or greater and<br>Match length=200 bases or greate fastx E value=1.0E-8 or less<br>*Full Length sequences:*<br>fastx score=100 or greater |
| BLIMPS | A BLocks IMProved Searcher that matches a sequence against those in BLOCKS, PRINTS, DOMO, PRODOM, and PFAM databases to search for gene families, sequence homology, and structural fingerprint regions. | Henikoff, S. and J.G. Henikoff (1991) Nucleic Acids Res. 19:6565-6572; Henikoff, J.G. and S. Henikoff (1996) Methods Enzymol. 266:88-105; and Attwood, T.K. et al. (1997) J. Chem. Inf. Comput. Sci. 37:417-424. | Score=1000 or greater;<br>Ratio of Score/Strength = 0.75 or larger; and, if applicable,<br>Probability value= 1.0E-3 or less |
| HMMER | An algorithm for searching a query sequence against hidden Markov model (HMM)-based databases of protein family consensus sequences, such as PFAM. | Krogh, A. et al. (1994) J. Mol. Biol. 235:1501-1531; Sonnhammer, E.L.L. et al. (1988) Nucleic Acids Res. 26:320-322. | Score=10-50 bits for PFAM hits, depending on individual protein families |

## Table 5 (cont.)

| Program | Description | Reference | Parameter Threshold |
|---|---|---|---|
| ProfileScan | An algorithm that searches for structural and sequence motifs in protein sequences that match sequence patterns defined in Prosite. | Gribskov, M. et al. (1988) CABIOS 4:61-66; Gribskov, M. et al. (1989) Methods Enzymol. 183:146-159; Bairoch, A. et al. (1997) Nucleic Acids Res. 25:217-221. | Normalized quality score ≥ GCG-specified "HIGH" value for that particular Prosite motif. Generally, score=1.4-2.1. |
| Phred | A base-calling algorithm that examines automated sequencer traces with high sensitivity and probability. | Ewing, B. et al. (1998) Genome Res. 8:175-185; Ewing, B. and P. Green (1998) Genome Res. 8:186-194. | |
| Phrap | A Phils Revised Assembly Program including SWAT and CrossMatch, programs based on efficient implementation of the Smith-Waterman algorithm, useful in searching sequence homology and assembling DNA sequences. | Smith, T.F. and M.S. Waterman (1981) Adv. Appl. Math. 2:482-489; Smith, T.F. and M.S. Waterman (1981) J. Mol. Biol. 147:195-197; and Green, P., University of Washington, Seattle, WA. | Score= 120 or greater; Match length= 56 or greater |
| Consed | A graphical tool for viewing and editing Phrap assemblies. | Gordon, D. et al. (1998) Genome Res. 8:195-202. | |
| SPScan | A weight matrix analysis program that scans protein sequences for the presence of secretory signal peptides. | Nielson, H. et al. (1997) Protein Engineering 10:1-6; Claverie, J.M. and S. Audic (1997) CABIOS 12:431-439. | Score=3.5 or greater |
| Motifs | A program that searches amino acid sequences for patterns that matched those defined in Prosite. | Bairoch, A. et al. (1997) Nucleic Acids Res. 25:217-221; Wisconsin Package Program Manual, version 9, page M51-59, Genetics Computer Group, Madison, WI. | |

## Claims

**1.** An isolated polypeptide comprising an amino acid sequence selected from the group consisting of:

a) an amino acid sequence selected from the group consisting of SEQ ID NO: 2, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14 and SEQ ID NO: 1;
b) a naturally occurring amino acid sequence having at least 90% sequence identity to an amino acid sequence selected from the group consisting of SEQ ID NO: 2, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14 and SEQ ID NO: 1;
c) a biologically active fragment of an amino acid sequence selected from the group consisting of SEQ ID NO: 2, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14 and SEQ ID NO: 1;
d) an immunogenic fragment of an amino acid sequence selected from the group consisting of SEQ ID NO: 2, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14 and SEQ ID NO: 1.

**2.** An isolated polypeptide of claim 1 selected from the group consisting of SEQ ID NO: 2, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14 and SEQ ID NO: 1.

**3.** An isolated polynucleotide encoding a polypeptide of claim 1 or 2.

**4.** An isolated polynucleotide encoding a polypeptide of claim 2 selected from the group consisting of SEQ ID NO: 17, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29 and SEQ ID NO: 16.

**5.** A recombinant polynucleotide comprising a promoter sequence operably linked to a polynucleotide of claim 3.

**6.** A cell transformed with a recombinant polynucleotide of claim 5.

**7.** A transgenic organism comprising a recombinant polynucleotide of claim 5.

**8.** A method for producing a polypeptide of claim 1, the method comprising:

a) culturing a cell under conditions suitable for expression of the polypeptide, wherein said cell is transformed with a recombinant polynucleotide, and said recombinant polynucleotide comprises a promoter sequence operably linked to a polynucleotide encoding the polypeptide of claim 1, and
b) recovering the polypeptide so expressed.

**9.** An isolated antibody which specifically binds to a polypeptide of claim 1.

**10.** An isolated polynucleotide comprising a polynucleotide sequence selected from the group consisting of:

a) a polynucleotide sequence selected from the group consisting of SEQ ID NO: 17, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29 and SEQ ID NO: 16;
b) a naturally occurring polynucleotide sequence having at least 90% sequence identity to a polynucleotide sequence selected from the group consisting of SEQ ID NO: 17, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29 and SEQ ID NO: 16;
c) a polynucleotide sequence complementary to a);
d) a polynucleotide sequence complementary to b);
e) an RNA equivalent of a)-d); and
f) an isolated polynucleotide comprising at least 60 contiguous nucleotides of a polynucleotide of a) to e).

**11.** A method for detecting a target polynucleotide in a sample, said target polynucleotide having a sequence of a polynucleotide of claim 10, the method comprising:

a) hybridizing the sample with a probe comprising at least 20 contiguous nucleotides comprising a sequence complementary to said target polynucleotide in the sample, and which probe specifically hybridizes to said target polynucleotide , under conditions whereby a hybridization complex is formed between said probe and said target polynucleotide or fragments thereof, and
b) detecting the presence or absence of said hybridization complex, and, optionally, if present, the amount thereof.

**12.** A method of claim 11, wherein the probe comprises at least 60 contiguous nucleotides.

**13.** A method for detecting a target polynucleotide in a sample, said target polynucleotide having a sequence of a polynucleotide of claim 10, the method comprising:

a) amplifying said target polynucleotide or fragment thereof using polymerase chain reaction amplification, and
b) detecting the presence or absence of said amplified target polynucleotide or fragment thereof, and, optionally, if present, the amount thereof.

**14.** A method for screening a compound for effectiveness as an agonist of a polypeptide of claim 1, the method comprising:

a) exposing a sample comprising a polypeptide of claim 1 to a compound; and
b) detecting agonist activity in the sample.

**15.** A composition comprising an effective amount of a polypeptide of claim 1 or an agonist compound identified by a method of claim 14 and a pharmaceutically acceptable excipient.

**16.** A method for treating a disease or condition associated with decreased expression of functional SYNT, comprising administering to a patient in need of such treatment a pharmaceutical composition of claim 15.

**17.** A method for screening a compound for effectiveness as an antagonist of a polypeptide of claim 1, the method comprising:

a) exposing a sample comprising a polypeptide of claim 1 to a compound; and
b) detecting antagonist activity in the sample.

**18.** A composition comprising an antagonist compound identified by a method of claim 17 and a pharmaceutically acceptable excipient.

**19.** A method for treating a disease or condition associated with overexpression of functional SYNT, comprising administering to a patient in need of such treatment a composition of claim 18.

**20.** A method of screening for a compound that specifically binds to the polypeptide of claim 1, said method comprising the steps of:

a) combining the polypeptide of claim 1 with at least one test compound under suitable conditions; and
b) detecting binding of the polypeptide of claim 1 to the test compound, thereby identifying a compound that specifically binds to the polypeptide of claim 1.

**21.** A method of screening for a compound that modulates the activity of the polypeptide of claim 1, said method comprising:

a) combining the polypeptide of claim 1 with at last one test compound under conditions permissive for the activity of the polypeptide of claim 1;
b) assessing the activity of the polypeptide of claim 1 in the presence of the test compound, and
c) comparing the activity of the polypeptide of claim 1 in the presence of the test compound with the activity of the polypeptide of claim 1 in the absence of the test compound, wherein a change in the activity of the polypeptide of claim 1 in the presence of the test compound is indicative of a compound that modulates the activity of the polypeptide of claim 1.

**22.** A method for screening a compound for effectiveness in altering expression of a target polynucleotide, wherein said target polynucleotide comprises a sequence of claim 4, the method comprising:

a) exposing a sample comprising the target polynucleotide to a compound, and
b) detecting altered expression of the target polynucleotide.

**23.** A method for assessing toxicity of a test compound, said method comprising:

a) treating a biological sample containing nucleic acids with the test compound;
b) hybridizing the nucleic acids of the treated biological sample with a probe comprising at least 20 contiguous nucleotides of a polynucleotide of claim 10 under conditions whereby a specific hybridization complex is formed between said probe and a target polynucleotide in the biological sample, said target polynucleotide comprising a polynucleotide sequence of a polynucleotide of claim 10 or fragment thereof;
c) quantifying the amount of hybridization complex; and
d) comparing the amount of hybridization complex in the treated biological sample with the amount of hybridization complex in an untreated biological sample, wherein a difference in the amount of hybridization complex in the treated biological sample is indicative of toxicity of the test compound.